# EUROPEAN PATENT APPLICATION

(11) **EP 2 345 418 A1**
(43) Date of publication of application: **20.07.2011**
(21) Application number: 11161639.7
(22) Date of filing: 14.05.2008
(51) Int. Cl.: A61K 38/40, A23C 13/12, A23C 15/00, A23C 17/00, A23C 21/00, A61K 31/592, A61K 31/593, A61K 36/48, A61P 25/00, A61P 37/00

(54) **Milk fat for the treatment of mucositis**

(30) Priority: 14.05.2007 NZ 55516307
(62) Divisional of application: 08766954.5
(71) Applicant: Fonterra Co-Operative Group Limited, Auckland (NZ)
(72) Inventor: Kanwar, Jagat, Rakesh, Palmerston North (NZ); Krissansen, Geoffrey, Wayne, Palmerston North (NZ); Sun, Xueying, Palmerston North (NZ); Palmano, Kay, Patricia, Palmerston North (NZ); MacGibbon, Alastair, Kenneth, Hugh, Palmerston North (NZ)
(74) Representative: Walker, Ross Thomson

(57) **Abstract**

The present invention relates to administration of milk fat or a milk fat analogue, optionally with at least one additional therapeutic factor, preferably lactoferrin or metal ion lactoferrin, preferably iron lactoferrin, preferably bovine lactoferrin, preferably iron bovine lactoferrin, or a metal ion functional variant or functional fragment thereof, to treat mucositis.

SEQUENCE LISTING

<110>  Fonterra Co-operative Group Limited

<120>  METHODS OF IMMUNE OR HEMATOLOGICAL ENHANCEMENT, INHIBITING TUMOUR
        FORMATION OR GROWTH, AND TREATING OR PREVENTING CANCER, CANCER
       SYMPTOMS, OR THE SYMPTOMS OF CANCER TREATMENTS

<130>  E24123EP/FB22497-RTW/kzm

<140>  08766954.5
<141>

<150>  PCT/NZ2008/000105
<151>  2008-05-14

<160>  4

<170>  PatentIn version 3.5

<210>  1
<211>  31
<212>  PRT
<213>  Synthetic

<400>  1

Phe Phe Ser Ala Ser Cys Val Pro Gly Ala Asp Lys Gly Gln Phe Pro
1               5                   10                  15

Asn Leu Cys Arg Leu Cys Ala Gly Thr Gly Glu Asn Lys Cys Ala
            20                  25                  30

<210>  2
<211>  23
<212>  PRT
<213>  Synthetic

<400>  2

Thr Lys Cys Phe Gln Trp Gln Arg Asn Met Arg Lys Val Arg Gly Pro
1               5                   10                  15

Pro Val Ser Cys Ile Lys Arg
            20

<210>  3
<211>  11
<212>  PRT
<213>  Bos taurus

<400>  3

Arg Arg Trp Gln Trp Arg Met Lys Lys Leu Gly
1               5                   10

<210>  4
<211>  17
<212>  PRT
<213>  Bos taurus

<400>  4

Trp Lys Leu Leu Ser Lys Ala Gln Glu Lys Phe Gly Lys Asn Lys Ser
1               5                   10                  15

Arg

## Description

### FIELD OF THE INVENTION

The present invention relates to methods of immune or hematological enhancement, inhibiting tumour formation or growth, and treating or preventing cancer, cancer symptoms, or the symptoms of cancer treatments by administration of milk fat or a milk fat analogue, optionally with at least one additional therapeutic agent such as an anti-tumour agent, preferably selected from lactoferrin (including iron-lactoferrin). The methods and medicinal uses of the invention may be carried out by employing dietary (as foods or food supplements), nutraceutical or pharmaceutical compositions. Compositions useful in the methods of the invention are also provided.

### BACKGROUND OF THE INVENTION

Milk is a rich biological fluid that provides nutrition at a time of rapid growth and development in the neonate. Because of this, it contains many growth regulators, in addition to the substrates necessary for infant development.

The health benefits of ingesting milk in terms of the risk o68f developing cancer have been a topic of investigation and wide debate. It has been reported that the consumption of either high-fat dairy foods (Larsson, et al., 2005) or low-fat milk (Ma, et al., 2001 and Goodman, et al., 2002) may reduce the risk of certain cancers, including colorectal and ovarian cancer. Conversely, other studies report that high intakes of low fat milk increase the risk of certain cancers (Larsson, et al., 2006). A review of the literature reported that the evidence for association of milk products with ovarian cancer risk is limited and inconsistent (Schulz*, et al.,* 2004). Both whole milk and low-fat milk have been associated with a reduced risk of breast cancer (Shin, *et al.,* 2002 and Bradlow and Sepkovic, 2002), whereas they were reported to be risk factors in other studies (Knekt, *et al.,* 1996 and Gaard, *et al.,*1995)*.* Low fat milk promotes the development of carcinogen-induced mammary tumours in rats (Qin, et *al.,* 2004), and low fat milk has been associated with an increased risk of prostate cancer (Tseng, *et al.,* 2005 and Veierod, *et al.,* 1997). One large prospective study reported that adult milk consumption tended to be negatively related to breast cancer incidence (Hjartaker, *et al.,* 2001), whereas recent reviews concluded that there was no consistent pattern of increased or decreased breast cancer risk with high consumption of either low or high fat dairy products (Moorman and Terry, 2004 and Parodi, 2005). In conclusion, the epidemiological evidence regarding the cancer risks and benefits of ingesting milk is inconsistent.

Milk fat contains a number of components, including conjugated linoleic acid (CLA), sphingomyelin, butyric acid, vaccenic acid, branched chain fatty acids, ether lipids, β-carotene, and vitamins A and D, that have been investigated to assess their anti-cancer potential (reviewed in Parodi, 1999 and Parodi, 1997). For example, the cis 9, trans 11 (c9, t11)-CLA isomer, and its precursor vaccenic acid reportedly inhibit the growth of tumour cell lines (Miller, et al., 2003 and O'Shea, et al., 2000). Dietary CLA reportedly reduces the incidence of colon tumours in 1,2-dimethylhydrazine-treated rats by increasing apoptosis (Kim and Park, 2003). Other studies report dietary CLA inhibits the initiation of mouse skin carcinogenesis by 7,12-dimethyl-benz[a]anthracene (DMBA) (Ha, et al., 1987), and of mouse fore-stomach neoplasia induced by benzo[a]pyrene (Chen, et al., 2003). It has been reported that the feeding of vaccenic acid and c9, t11 CLA as butter fat inhibited the development of mammary carcinomas in rats (Corl, et al., 2003, Banni, et al., 2001). CLA has been reported to inhibit angiogenesis which may contribute to its efficacy as a chemopreventive agent (Masso-Welch, et al., 2002).

In contrast, other studies report that dietary CLA, either alone or in combination with a soy protein isolate, failed to inhibit the in vivo growth and development of prostate tumour cells in rats (*Cohen, et al.,* 2003), and the azoxymethane-induced development of aberrant crypt foci in colons of male Sprague-Dawley rats (Ealey, *et al.,* 2001). Further, the weight of rat hepatomas was reported to be significantly higher in rats fed a CLA diet (*Yamasaki, et al.,* 2001). An epidemiology study reported that the intake of CLA-containing food groups was not related to breast cancer incidence (*Voorrips, et al.,* 2002). As with the epidemiological studies of milk fat consumption in humans, the results of the different studies of CLA and anti-tumour activity are inconsistent.

It has previously been reported that tumours do not respond well to chemotherapy in all cases. For example, chemotherapy efficacy varies for cancer sufferers depending on the cancer type, the nature and doses of the drugs used for treatment, the mechanisms by which the drugs work, and the therapeutic regimes.

It is known in the field that cancers differ in their sensitivity to chemotherapy, from the usually and often sensitive (e.g. lymphomas, acute lymphoblastic leukemia (ALL), chronic lymphocytic leukemia (CLL), Hodgkin's disease, intermediate and high grade non-Hodgkin's lymphoma, for example, diffuse large cell lymphoma, Burkitt's lymphoma, lymphoblastic lymphoma, choriocarcinoma, embryonal tumours, myelomatosis, oat cell carcinoma of bronchus, testicular carcinoma, Ewing's sarcoma, Wilms' tumor, skin cancer) where complete clinical cures can be achieved to the largely resistant (bladder cancer, esophageal cancer, non-small cell lung cancer, hepatocellular carcinoma, renal carcinoma, pancreatic carcinoma, head and neck cancer, cervical carcinoma, liver carcinoma, lung carcinomas that are not oat cell). It has previously been reported that EL-4 tumours larger than 0.3 cm in diameter become completely non-responsive to immunotherapy and anti-angiogenic therapy (Kanwar, et al., 1999 and Sun, et al., 2001), and chemotherapy (Kanwar et al.-WO 2006/054908).

Moreover, the treatment of cancer, whether by radiotherapy, surgery, chemotherapy or other methods, frequently causes or exacerbates associated symptoms or disorders. For example, cancer patients undergoing therapy are frequently cachectic, while chemotherapy can cause mucositis of the small intestine associated with cellular apoptosis in the crypts which precedes villous atrophy (Keefe, et al., 2000).

It would therefore be desirable to provide an improved method of inhibiting tumour formation or growth or reducing the symptoms of or severity of disorders associated with cancer or the treatment of cancer using milk fat, optionally with one or more additional therapeutic agents or to at least provide the public with a useful choice.

### SUMMARY OF THE INVENTION

Accordingly, one aspect of the invention relates to a method of inhibiting tumour formation, inhibiting tumour growth, inhibiting tumour metastasis or treating or preventing cancer in a subject, the method comprising separate, simultaneous or sequential administration of an effective amount of milk fat or a milk fat analogue and one or more therapeutic agents, such as one or more anti-tumour agents, to a subject in need thereof, preferably the one or more anti-tumour agents are selected from anti-tumour food factors, chemotherapeutic agents, immunotherapeutic agents, hematopoietic agents, anticachectic agents, or antimucositic agents, more preferably the one or more therapeutic agents is a lactoferrin (Lf).

Another aspect of the invention relates to a method of stimulating the immune system of a subject, the method comprising administration of an-effective amount of milk fat or a milk fat analogue to a subject in need thereof. In one embodiment, the method of stimulating the immune system comprises separate, simultaneous or sequential administration to the subject of milk fat and one or more anti-tumour agents, preferably the one or more anti-tumour agents is selected from anti-tumour food factors, chemotherapeutic agents, immunotherapeutic agents, hematopoietic agents, anticachectic agents, or antimucositic agents, more preferably the one or more therapeutic agents is a lactoferrin.

In one embodiment, the administration increases the production of Th1 and Th2 cytokine within a tumor of the subj ect. In one embodiment, the administration increases the production of Th1 and Th2 cytokines within the intestine of the subject. In one embodiment, the administration increases the level of Th1 and Th2 cytokines in the systemic circulation of the subject. In one embodiment, the administration increases an anti-tumour immune response in the subject.

Another aspect of the invention relates to a method of inducing apoptosis in a subject, the method comprising administration of an effective amount of milk fat or a milk fat analogue to a subject in need thereof. In one embodiment the method of inducing apoptosis in a subject in need thereof comprises separate, simultaneous or sequential administration to the subject of milk fat and at least one anti-tumour agent, preferably the at least one anti-tumour agent is selected from anti-tumour food factors, chemotherapeutic agents, immunotherapeutic agents, hematopoietic agents, anticachectic agents, or antimucositic agents, more preferably the at least one additional therapeutic agent is lactoferrin. In one embodiment the apoptosis is of tumour cells.

Another aspect of the invention relates to a method of inhibiting angiogenesis in a subject, the method comprising administration of an effective amount of milk fat or a milk fat analogue to a subject in need thereof. In one embodiment the method of inhibiting angiogenesis in a subject in need thereof comprises separate, simultaneous or sequential administration to the subject of milk fat and at least one anti-tumour agent, preferably the at least one anti-tumour agent is selected from anti-tumour food factors, chemotherapeutic agents, immunotherapeutic agents, hematopoietic agents, anticachectic agents, or antimucositic agents, more preferably the at least one additional therapeutic agent is lactoferrin. In one embodiment the angiogenesis is tumour angiogenesis.

Another aspect of the invention relates to a method of treating or preventing anemia caused by low hemoglobin or red blood cell levels, cachexia, mucositis, or leukopaenia, or of maintaining or improving one or more of the white blood cell count, the red blood cell count, and the myeloid cell count of a subject, the method comprising administration of an effective amount of milk fat or a milk fat analogue to a subject in need thereof.

Another aspect of the invention relates to a method of maintaining or improving one or more of the white blood cell count, the red blood cell count, or the myeloid cell count of a subject comprising administration to the subject of milk fat or a milk fat analogue, preferably with the separate, simultaneous or sequential administration to the subject of at least one anti-tumour agent, preferably the at least one anti-tumour agent is selected from anti-tumour food factor, chemotherapeutic agents, immunotherapeutic agents, hematopoietic agents, anticachectic agents, or antimucositic agents, more preferably the at least one additional therapeutic agent is lactoferrin.

Another aspect of the invention relates to a method of treating or preventing anemia in a subject comprising administration to the subject of milk fat or a milk fat analogue, preferably with the separate, simultaneous or sequential administration to the subject of at least one anti-tumour agent, preferably the at least one anti-tumour agent is selected from anti-tumour food factors, chemotherapeutic agents, immunotherapeutic agents, hematopoietic agents, anticachectic agents, or antimucositic agents, more preferably the at least one additional therapeutic agent is lactoferrin.

Another aspect of the invention relates to a method of treating or preventing cachexia in a subject comprising administration to the subject of milk fat or a milk fat analogue, preferably with the separate, simultaneous or sequential administration to the subject of at least one anti-tumour agent, preferably the at least one anti-tumour agent is selected from anti-tumour food factors, chemotherapeutic agents, immunotherapeutic agents, hematopoietic agents, anticachectic agents, or antimucositic agents, more preferably the at least one additional therapeutic agent is lactoferrin.

Another aspect of the invention relates to a method of treating or preventing mucositis in a subject comprising administration to the subject of milk fat or a milk fat analogue, preferably with the separate, simultaneous or sequential administration to the subject of at least one anti-tumour agent, preferably the at least one anti-tumour agent is selected from anti-tumour food factors, chemotherapeutic agents, immunotherapeutic agents, hematopoietic agents, anticachectic agents, or antimucositic agents, more preferably the at least one additional therapeutic agent is lactoferrin.

Another aspect of the invention relates to a method of inhibiting tumour formation, inhibiting tumour growth, inhibiting tumour metastasis or treating or preventing cancer in a subject comprising the administration to the subject of a milk fat fraction such as a phospholipid fraction, hard milk fat fraction, soft milk fat fraction, sphingolipid fraction, milk fat globular membrane fraction, phospholipid fraction, or complex lipid fraction, or a combination of any two or more thereof, optionally with at least one additional therapeutic agent, preferably the at least one additional therapeutic agent is selected from anti-tumour food factors, immunotherapeutic agents, hematopoietic agents, anticachectic agents, or antimucositic agents, more preferably the at least one additional therapeutic agent is lactoferrin (Lf).

Another aspect of the invention relates to a method of increasing the responsiveness of a subject to a cancer therapy comprising administration to the subject of milk fat or a milk fat analogue, preferably with the separate, simultaneous or sequential administration to the subject of at least one anti-tumour agent, preferably the at least one anti-tumour agent is selected from anti-tumour food factors, chemotherapeutic agents, immunotherapeutic agents, hematopoietic agents, anticachectic agents, or antimucositic agents, more preferably the at least one additional therapeutic agent is lactoferrin.

Another aspect of the invention relates to a method of increasing the sensitivity of a tumour in a subject to a cancer therapy comprising administration to the subject of milk fat or a milk fat analogue, preferably with the separate, simultaneous or sequential administration to the subject of at least one anti-tumour agent, preferably the at least one anti-tumour agent is selected from anti-tumour food factors, chemotherapeutic agents, immunotherapeutic agents, hematopoietic agents, anticachectic agents, or antimucositic agents, more preferably the at least one additional therapeutic agent is lactoferrin.

Another aspect of the invention relates to a method of speeding the recovery of a subject undergoing cancer therapy comprising administration to the subject of milk fat or a milk fat analogue, preferably with the separate, simultaneous or sequential administration to the subject of at least one anti-tumour agent, preferably the at least one anti-tumour agent is selected from anti-tumour food factors, chemotherapeutic agents, immunotherapeutic agents, hematopoietic agents, anticachectic agents, or antimucositic agents, more preferably the at least one additional therapeutic agent is lactoferrin.

Another aspect of the invention relates to use of milk fat in the manufacture of a composition for a purpose as herein described, preferably the composition comprises or is administered separately, simultaneously or sequentially with at least one additional therapeutic agent, preferably the at least one additional therapeutic agent is an anti-tumour agent, preferably the anti-tumour agent is selected from anti-tumour food factors, immunotherapeutic agents, hematopoietic agents, anticachectic agents, or antimucositic agents, more preferably the at least one additional therapeutic agent is lactoferrin.

Another aspect of the invention relates to use of milk fat, optionally with at least one additional therapeutic agent, in the manufacture of a composition for a purpose as herein described.

Another aspect of the invention relates to use of milk fat and at least one additional therapeutic agent in the manufacture of a composition for a purpose as herein described, wherein the composition is formulated to provide separate, simultaneous or sequential administration of the milk fat and the at least one additional therapeutic agent.

Another aspect of the invention relates to use of milk fat and at least one additional therapeutic agent in the manufacture of a composition for a purpose as herein described, wherein the milk fat is administered separately, simultaneously or sequentially with the additional therapeutic agent.

Another aspect of the invention relates to use of milk fat and at least one additional therapeutic agent in the manufacture of a composition for a purpose as herein described, wherein the milk fat is formulated for administration separately, simultaneously or sequentially with the additional therapeutic agent.

Another aspect of the invention relates to a composition comprising, consisting essentially of or consisting of milk fat and one or more, two or more or three or more additional therapeutic agents.

Another aspect of the invention relates to a product comprising, consisting essentially of or consisting of milk fat and one or more, two or more or three or more additional therapeutic agents as a combined preparation for simultaneous, separate or sequential use for a purpose as described herein.

The following embodiments may relate to any of the above aspects.

In preferred embodiments, the at least one therapeutic agent is an anti-tumour agent. In preferred embodiments, the anti-tumour agent is lactoferrin.

In one embodiment the lactoferrin is selected from the group comprising a lactoferrin polypeptide, a functional lactoferrin variant, a functional lactoferrin fragment, metal ion lactoferrin, a metal ion lactoferrin functional variant, and a metal ion lactoferrin functional fragment, or a mixture of any two or more thereof. In one embodiment the lactoferrin is apo-lactoferrin. In another embodiment the lactoferrin is naturally iron-saturated. In another embodiment the lactoferrin is substantially fully iron saturated.

Purposes described herein include a purpose selected from inhibiting tumour formation, inhibiting tumour growth, inhibiting tumour metastasis or treating or preventing cancer in a subject in need thereof, stimulating the immune system of a subject in need thereof, increasing the production of Th1 and Th2 cytokines within a tumor of a subject in need thereof, increasing the production of Th1 and Th2 cytokines within the intestine of a subject in need thereof, increasing the level of Th1 and Th2 cytokines in the systemic circulation of a subject in need thereof, increasing an anti-tumour immune response in a subject in need thereof, inducing apoptosis in a subject in need thereof, inducing apoptosis of tumour cells in a subject in need thereof, inhibiting angiogenesis in a subject in need thereof, inhibiting tumour angiogenesis in a subject in need thereof, maintaining or improving one or more of the white blood cell count, the red blood cell count, or the myeloid cell count in a subject in need thereof, treating or preventing anemia in a subject in need thereof, treating or preventing cachexia in a subject in need thereof, treating or preventing mucositis in a subject in need thereof, increasing the responsiveness of a subject to a cancer therapy, increasing the sensitivity of a tumour in a subject to a cancer therapy, and speeding the recovery of a subject undergoing cancer therapy.

In one embodiment, the at least one therapeutic agent or anti-tumour agent is selected from the group consisting of anti-tumour food factors, immunotherapeutic agents, hematopoietic agents, anticachectic agents, or antimucositic'agents.

In one embodiment the anti-tumour food factor is selected from vitamin D and vitamin D analogues (including but not limited to those referenced below), soy protein, one or more soybean components (including those those referenced below), polyphenols, lycopene, wheat bran, flavonoids, inositol, resveratrol, propolis, mushroom extract, anthocyanins, almonds, ginseng, casein hydrolysate, and combinations thereof.

In one embodiment the anti-tumour food factor is selected from the group comprising anti-tumour foods and anti-tumour food components. Preferably one or more, two or more or three or more anti-tumour food factors are administered.

In one embodiment the anti-tumour food may be a functional food or derivative thereof that has anti-cancerous properties including those described herein.

In one embodiment the anti-tumour food component may be selected from those described herein.

In one embodiment a method of the invention comprises administration of a composition consisting essentially of or consisting of milk fat and at least one additional therapeutic agent, preferably the at least one additional therapeutic agent is selected from a lactoferrin, a functional lactoferrin variant, a functional lactoferrin fragment, metal ion lactoferrin, a metal ion lactoferrin functional variant, a metal ion lactoferrin functional fragment, or a mixture thereof. Preferably the composition consists essentially of or consists of one or more, two or more or three or more anti-tumour food factors.

In one embodiment of a use of the invention, a composition is manufactured for inhibiting tumour, formation in a subject, inhibiting tumour growth in a subject, inhibiting tumour metastasis in a subject, treating or preventing cancer in a subject, stimulating the immune system in a subject, increasing the production of Th1 and Th2 cytokines within a tumor in a subject, increasing the production of Th1 and Th2 cytokines within the intestine of a subject, increasing the level of Th1 and Th2 cytokines in the systemic circulation of a subject, increasing an anti-tumour immune response in a subject, inducing apoptosis in a subject, inducing apoptosis of tumour cells in a subject, inhibiting angiogenesis in a subject, inhibiting tumour angiogenesis in a subject, maintaining or improving one or more of the white blood cell count, the red blood cell count, or the myeloid cell count of a subject, treating or preventing anemia in a subject in need thereof, treating or preventing cachexia in a subject in need thereof, treating or preventing mucositis in a subject in need thereof, treating or preventing leukocytopenia in a subject in need thereof, increasing the responsiveness of a subject to a cancer therapy, increasing the responsiveness of a tumour in a subject to a cancer therapy or speeding the recovery of a subject undergoing cancer therapy.

In one embodiment the administration is oral, topical or parenteral administration.

In one embodiment the subject is suffering from or is susceptible to cancer; has undergone therapy, but is in relapse or is susceptible to relapse; has a tumour refractory to therapy with a chemotherapeutic, radiotherapeutic, anti-angiogenic or immunotherapeutic agent; or has previously undergone surgery, unsuccessful surgery or unsuccessful therapy with a chemotherapeutic, radiotherapeutic, anti-angiogenic or immunotherapeutic agent.

In one embodiment, the milk fat is selected from dairy lipids, dairy lipid fractions, dairy lipid hydrolysates, and dairy lipid fraction hydrolysates. Preferred milk fats are dairy fats, particularly bovine milk fats.

In other embodiments, the milk fat is any mammalian milk fat including but not limited to sheep, goat, pig, mouse, water buffalo, camel, yak, horse, donkey, llama or human milk fat.

In one embodiment the lactoferrin is any mammalian lactoferrin including but not limited to sheep, goat, pig, mouse, water buffalo, camel, yak, horse, donkey, llama, bovine or human lactoferrin. Preferably the lactoferrin is bovine lactoferrin.

In one embodiment the lactoferrin is apo-lactoferrin. In one embodiment the lactoferrin, functional lactoferrin variant or functional lactoferrin fragment is free of metal ions. In one embodiment the lactoferrin or functional variant or functional fragment thereof is at least about 5, 10, or 20% metal ion saturated on a stoichiometric basis.

In one embodiment the metal ion is an ion selected from the group comprising aluminium, bismuth, copper, chromium, cobalt, gold, iron, manganese, osmium, platinum, ruthenium, and zinc ions, or any combination of any two or more thereof, or other ions that will coordinate specifically in a lactoferrin metal ion binding pocket. Preferably the metal ion is an iron ion.

In one embodiment, the lactoferrin, functional lactoferrin variant or functional lactoferrin fragment is involved in non-specific ion binding. Preferably, the ions that may be non-specifically bound to the lactoferrin, functional lactoferrin variant or functional lactoferrin fragment are selected from aluminium, calcium, bismuth, copper, chromium, cobalt, gold, iron, manganese, osmium, platinum, ruthenium, selenium, and zinc ions, or any combination of any two or more thereof. The ion may be any ion or mixture of ions that will non-specifically bind to the lactoferrin, functional lactoferrin variant or functional lactoferrin fragment, preferably calcium and selenium ions.

In one embodiment the metal ion lactoferrin or a metal ion functional variant or functional fragment thereof is at least about 25, 30, 3 5, 40, 45, 50, 55, 60, 65, 70, 75, 80, 85, 90, 91, 92, 93, 94, 95, 96, 97, 98, 99, 99.5 or 100% metal ion saturated on a stoichiometric basis.

In one embodiment the metal ion lactoferrin or a metal ion functional variant or functional fragment thereof is at least about 105, 110, 115, 120,125,130, 135, 140, 145, 150, 155, 166, 165, 170, 175, 180, 185, 190, 195 or 200% metal ion saturated on a stoichiometric basis.

In one embodiment, a composition useful herein comprises about 2 grams to about 210 grams of milk fat or a milk fat analogue and from about 0.1 grams to about 210 grams of one or more anti-tumour agents. In one embodiment the composition comprises about 2, 4, 6, 8, 10,15, 20, 25, 30, 35, 40, 45, 50, 55, 60, 65, 70, 75, 80, 85, 90, 95, 100,105, 110, 115, 120, 125, 130, 135, 140, 145, 150, 155, 160, 165, 170, 175, 180, 185, 190, 195, 200, 205 or 210 grams of milk fat or a milk fat analogue and useful ranges may be selected between any of these values. In one embodiment the composition comprises about 2, 4, 6, 8, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205 or 210 grams of one or more anti-tumour agents and useful ranges may be selected between any of these values. Preferably the one or more anti-tumour agents are selected from the group comprising lactoferrin, apo-lactoferrin, a lactoferrin polypeptide, a functional lactoferrin variant, a functional lactoferrin fragment, metal ion lactoferrin, naturally iron-saturated lactoferrin, substantially fully iron-saturated lactoferrin, a metal ion lactoferrin functional variant, a metal ion lactoferrin functional fragment, or a mixture thereof. In various embodiment the composition comprises
(a) about 2 grams to about 210 grams of milk fat or a milk fat analogue and from about 0.7 grams to about 70 grams of the one or more anti-tumour agents;
(b) about 35 grams to about 210 grams of milk fat or a milk fat analogue and from about 0.3.5 grams to about 210 grams of the one or more anti-tumour agents;
(c) about 10 grams to about 200 grams of milk fat or a milk fat analogue and from about 2.5 grams to about 70 grams of the one or more anti-tumour agents;
(d) about 10 grams to about 200 grams of milk fat or a milk fat analogue and from about 0.25 grams to about 5 grams of the one or more anti-tumour agents;
(e) about 15 grams to about 30 grams of milk fat or a milk fat analogue and from about 1 grams to about 6 grams of the one or more anti-tumour agents; or
(f) about 3 grams to about 8 grams of milk fat or a milk fat analogue and from about 0.1 grams to about 1 grams of the one or more anti-tumour agents.

In one embodiment the method comprises administration with milk fat of a mixture of metal ion lactoferrin and at least one metal ion functional variant or functional fragment thereof.

In one embodiment the milk fat and the additional therapeutic agent, preferably lactoferrin, provide a synergistic therapeutic effect that is greater than the effect of either one alone or greater than the additive effects of either one alone. For example, there is a greater effect on inhibition of tumour formation or growth, tumour regression, cytolytic effects, immune enhancement, generation of Th1 and Th2 cytokines, maintenance or improvement in white blood cell count, red blood cell count, or myeloid cell count, treatment or prevention of anemia, cachexia, mucositis, or the responsiveness of a subject or a tumour to the treatment method. In one embodiment, the lactoferrin and the anti-tumour food factor allow the administration of a co-administered or sequentially administered cancer therapy to be reduced or increased in dose or in length of administration, as appropriate.

In one embodiment a method of the invention further comprises separate, simultaneous or sequential administration of at least one cancer therapy.

In one embodiment the cancer therapy is an anti-tumour agent or anti-tumour therapy.

In one embodiment the milk fat, optionally with at least one additional therapeutic agent, and at least one anti-tumour agent or anti-tumour therapy are administered separately, simultaneously or sequentially.

In one embodiment the anti-tumour therapy is selected from therapies such as, but not limited to, surgery, chemotherapies, radiation therapies, hormonal therapies, biological therapies/immunotherapies, cellular therapies, anti-angiogenic therapies, cytotoxic therapies, vaccines, nucleic acid-based vaccines (e.g. nucleic acids expressing a cancer antigen such as DNA vaccines including p185 vaccines), viral-based therapies (e.g. adeno-associated virus, lentivirus), gene therapies, small molecule inhibitor therapies, nucleotide-based therapies (e.g. RNAi, antisense, ribozymes etc), antibody-based therapies, oxygen and ozone treatments, embolization, and/or chemoembolization therapies. In one embodiment the anti-tumour agent comprises one or more angiogenesis inhibitors.

In one embodiment the anti-tumour agent is a chemotherapeutic agent or an immunotherapeutic agent. In one embodiment the at least one anti-tumour agent is a chemotherapeutic agent. Preferably the chemotherapeutic agent is selected from tubulin disruptors,"DNA intercalators, and mixtures thereof. In one embodiment tubulin disruptors include but are hot limited to those listed in published international patent application WO 2006/054908 that is incorporated by reference herein. In one embodiment DNA intercalators include but are not limited to those listed in published international patent application WO 2006/054908 that is incorporated by reference herein. In one embodiment the chemotherapeutic agent is paclitaxel, doxorubicin, epirubicin, fluorouracil, cyclophosphamide or methotrexate.

In one embodiment the anti-tumour agent is an immunotherapeutic agent. Preferably the immunotherapeutic agent is an expression plasmid encoding the T cell co-stimulator B7-1, a T cell co-stimulator, or a functionally related molecule, for example a soluble B7-Ig chimera. In one embodiment the anti-tumour agent comprises immune cell therapy. Preferably the therapy is dendritic cell therapy.

In one embodiment the chemotherapeutic agent is paclitaxel, doxorubicin, epirubicin, fluorouracil, cyclophosphamide or methotrexate.

In one embodiment the anti-tumour agent is an immunotherapeutic agent. Preferably the immunotherapeutic agent is an expression plasmid encoding the T cell co-stimulator B7-1, a T cell co-stimulator, or a functionally related molecule, for example a soluble B7-Ig chimera.

In one embodiment the anti-tumour agent comprises immune cell therapy. Preferably the therapy is dendritic cell therapy.

In one embodiment the anti-tumour agent comprises one or more angiogenesis inhibitors.

In one embodiment the at least one anti-tumour agent is administered orally or parenterally, preferably by intravenous, intraperitoneal or intratumoural injection.

In one embodiment the milk fat, optionally with at least one additional therapeutic agent,is administered daily for at least about 1, 2, 3, 4, 5, 6, 7, 8, 9 or 10 weeks before administration of the anti-tumour agent or anti-tumour therapy.

In one embodiment the milk fat, optionally with at least one additional therapeutic agent, is administered for at least about *1, 2, 3, 4, 5, 6, 7, 8, 9, 10, 11, 12, 13, 14, 15, 16, 17,* 18, 19, 20 or 21 days or for at least about 1, 2, 3, 4, 5, 6, 7 or 8 weeks or for at least about 1, 2, 3, 4, 5 or 6 months before administration of the anti-tumour agent or the anti-tumour therapy.

In one embodiment the milk fat, optionally with at least one additional therapeutic agent, is administered for at least about 1, 2, 3,4, 5, 6,7, 8, 9,10,11,12,13,14,15,16,17, 18, 19, 20 or 21 days or for at least about 1, 2, 3, 4, 5, 6, 7 or 8 weeks or for at least about 1, 2, 3, 4, 5 or 6 months after administration of the anti-tumour agent or the anti-tumour therapy has begun.

In one embodiment the milk fat, optionally with at least one additional therapeutic agent, is administered at least once daily including continuously over a day orally or by parenteral drip or a combination of administrative routes, with or without a cancer therapy.

In one embodiment the tumour or the cancer is a solid tumour, a leukemia, lymphoma, multiple myeloma, a hematopoietic tumor of lymphoid lineage, a hematopoietic tumor of myeloid lineage, a colon carcinoma, a breast cancer, a melanoma, a skin cancer or a lung cancer.

In one embodiment the tumour or the cancer is a leukemia such as but not limited to, acute leukemia, acute lymphocytic leukemia, acute granulocytic leukemia, acute myelocytic leukemia such as myeloblastic, promyelocytic, myelomonocytic, monocytic, erythroleukemia leukemia and myelodysplastic syndrome, chronic leukemia such as but not limited to, chronic myelocytic leukemia, chronic granulocytic leukemia, chronic lymphocytic leukemia, and hairy cell leukemia.

In one embodiment the tumour or the cancer is a lymphoma such as but not limited to Hodgkin's disease and non-Hodgkin's disease.

In one embodiment the tumour or the cancer comprises a hematopoietic tumor of myeloid lineage such as but not limited to acute and chronic myelogenous leukemia, smoldering multiple myeloma, nonsecretory myeloma and osteosclerotic myeloma.

In one embodiment the tumour or the cancer comprises a hematopoietic tumor of lymphoid lineage, including leukemia, acute and chronic lymphocytic leukemia, acute and chronic lymphoblastic leukemia, B-cell lymphoma, T-cell lymphoma, Burkitts lymphoma.

In one embodiment the tumour or the cancer comprises a hematopoietic tumor of B lymphoid lineage.

In one embodiment the tumour or the cancer comprises a hematopoietic tumor of T lymphoid lineage.

In one embodiment the tumour is a large tumour. In one embodiment the tumour is or the cancer comprises
(a) a tumour that is at least about 0.3, 0.4 or 0.5 cm in diameter, or
(b) a tumour that is refractory to therapy with one at least one immunotherapeutic, anti-angiogenic or chemotherapeutic agent.

In one embodiment one or more of the white blood cell count, the red blood cell count, or the myeloid cell count of the subject is maintained or improved.

In one embodiment the tumour is reduced in size or substantially eradicated.

In one embodiment, when administered the lactoferrin is administered in a dosage form comprising digestible protein, preferably casein or other protein such as other edible proteins.

In one embodiment the composition is a food, drink, food additive, drink additive, dietary supplement, nutritional product, medical food, nutraceutical, medicament or pharmaceutical. Preferably the composition is formulated for oral or topical administration.. Preferably the composition is formulated for oral or parenteral administration. In one embodiment the composition comprises milk fat and a milk protein fraction.

In one embodiment the composition comprises a milk composition selected from fresh or recombined whole milk, recombined or fresh skim milk, reconstituted whole or skim milk powder, skim milk concentrate, skim milk powder, skim milk retentate, concentrated milk, buttermilk, ultrafiltered milk retentate, milk protein concentrate (MPC), milk protein isolate (MPI), calcium depleted milk protein concentrate (MPC), low fat milk, low fat milk protein concentrate (MPC), colostrum, a colostrum fraction, colostrum protein concentrate (CPC), colostrum whey, an immunoglobulin fraction from colostrum, whey, whey protein isolate (WPI), whey protein concentrate (WPC), sweet whey, lactic acid whey, mineral acid whey, or reconstituted whey powder.

In one embodiment the milk fat is formulated for coadministration with the at least one additional therapeutic agent. In one embodiment the milk fat is formulated for sequential administration with the at least one additional factor.

In one embodiment when a composition of the invention or a composition employed in a method of the invention comprises lactoferrin, the composition provides a population of lactoferrin polypeptides or functional variants or fragments thereof wherein at least about 25, 30, 35, 40, 45, 50, 55, 60, 65, 70, 75, 80, 85, 90, 91, 92, 93, 94, 95, 96, 97, 98, 99, 99.5 or 100% of the available metal ion-binding pockets in the population are bound to a metal ion, preferably an iron ion.

In one embodiment when a composition of the invention or a composition employed in a method of the invention comprises lactoferrin, the composition provides a population of lactoferrin polypeptides or functional variants or fragments thereof wherein about 100% of the available metal ion-binding pockets in the population are bound to a metal ion, preferably an iron ion, and additional metal ions are bound to the lactoferrin molecules in non-specific binding sites so that the lactoferrin is at least about 105, 110, 115, 120, 125, 130, 135, 140, 145, 150, 155, 160, 165, 170,175, 180,185, 190, 195 or 200% metal ion saturated on a stoichiometric basis.

In one embodiment, the milk fat or milk fat analogue comprises
(a) between about 23%(w/w) and about 32%(w/w) palmitic acid;
(b) between about 15%(w/w) and about 22%(w/w) oleic acid;
(c) between about 10%(w/w) and about 15%(w/w)stearic acid;
(d) between about 9%(w/w) and about 12%(w/w) myristic acid;
(e) between about 3%(w/w) and about 5%(w/w) butyric acid;
(f) any two of a), b), c), d), or e) above;
(g) any three of a), b), c), d), or e) above;
(h) any four of a), b), c), d), or e) above; or
(i) each of a), b), c), d), and e) above.

It is intended that reference to a range of numbers disclosed herein (for example, 1 to 10) also incorporates reference to all rational numbers within that range (for example, 1, 1. 1, 2, 3, 3.9, 4, 5, 6, 6.5, 7, 8, 9 and 10) and also any range of rational numbers within that range (for example, 2 to 8, 1.5 to 5.5 and 3.1 to 4.7) and, therefore, all sub-ranges of all ranges expressly disclosed herein are hereby expressly disclosed. These are only examples of what is specifically intended and all possible combinations of numerical values between the lowest value and the highest value enumerated are to be considered to be expressly stated in this application in a similar manner.

In this specification where reference has been made to patent specifications, other external documents, or other sources of information, this is generally for the purpose of providing a context for discussing the features of the invention. Unless specifically stated otherwise, reference to such external documents is not to be construed as an admission that such documents, or such sources of information, in any jurisdiction, are prior art, or form part of the common general knowledge in the art.

The invention may also be said broadly to consist in the parts, elements and features referred to or indicated in the specification of the application, individually or collectively, in any or all combinations of two or more of said parts, elements or features, and where specific integers are mentioned herein that have known equivalents in the art to which the invention relates, such known equivalents are deemed to be incorporated herein as if individually set forth.

### BRIEF DESCRIPTION OF THE DRAWINGS

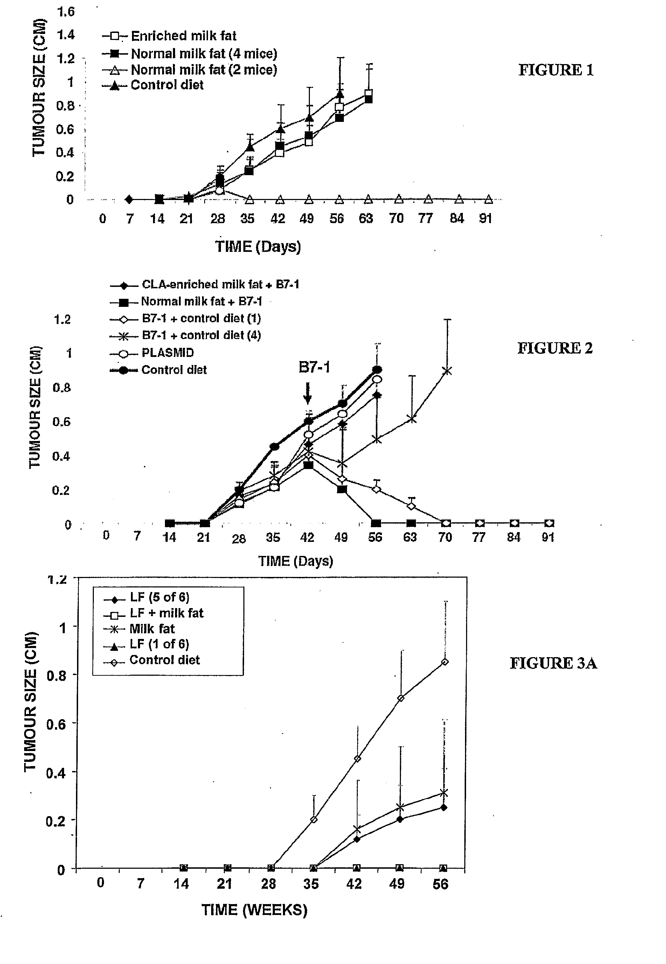

Figure 1 is a graph showing that milk fat inhibits the growth of lymphomas and inhibits tumorigenesis. Mice were fed the control AIN93G diet or the same diet where a proportion of the fat was substituted with either milk fat, or enriched milk fat. After 2 weeks on the diets, 2 x 105 EL-4 cells were injected into the flanks of mice. Tumour size as measured by two perpendicular diameters (in centimetres) was monitored until day 91, or until tumours reached 1 cm in diameter. Each point represents the mean tumour size with 95% confidence intervals for either 6 mice, or the number of mice indicated.

Figure 2 is a graph showing that milk fat augments B7-1 immunogene therapy to eradicate tumours. Mice were fed the control AIN93G diet or the same diet where a proportion of the fat was substituted with either milk fat, or enriched milk fat. After 2 weeks on the diets, 2 x 10⁵ EL-4 cells were injected into the flanks of mice. Tumour size as measure by two perpendicular diameters (in centimetres) was monitored. The tumours were injected with DNA-liposome complexes containing 60 µg of a B7-1 expression plasmid when tumours reached ~0.4 cm in diameter. The timing of administration of the plasmid is indicated by the arrow. Tumour size as measured by two perpendicular diameters (in centimetres) was monitored until day 91, or until tumours reached 1 cm in diameter. Each point represents the mean tumour size with 95% confidence intervals for either 6 mice, or the number of mice indicated.

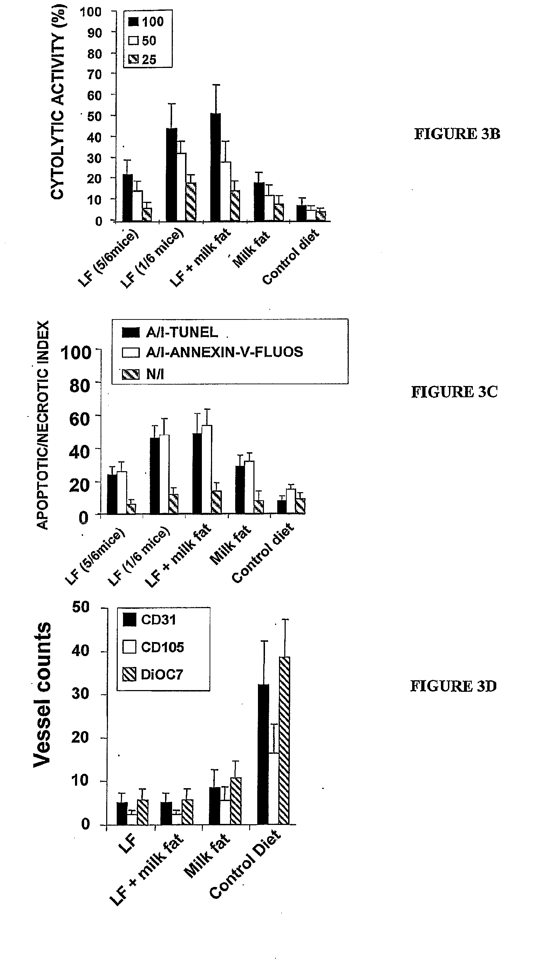

Figure 3 is four graphs showing that milk fat synergizes with iron-saturated lactoferrin (Lf+) to completely prevent tumorigenesis. (A) Effects on tumorigenesis. Mice were fed the control AIN93G diet or the same diet where a proportion of the fat and protein was substituted with either Lf+, milk fat, or a combination of Lf+ and milk fat. Day 0 refers to the day the mice were placed on their diets. After 2 weeks on the diets, 2 x 105 EL-4 cells were injected into the flanks of mice. Tumour size as measured by two perpendicular diameters (in centimetres) was monitored until day 56. Each point represents the mean tumour size with 95% confidence intervals for either 6 mice, or the number of mice indicated. (B) Effects on anti-tumour cytolytic activity. Splenocytes were harvested from mice in (A) at day 56 and tested for their cytolytic activity against EL-4 target cells. The percent cytotoxicity is plotted against various effector-to-target cell ratios (E:T ratios). Each point represents the mean percent cytotoxicity obtained from 6 mice or the number of mice indicated. Error bars represent 95% confidence intervals. (C) Effects on tumour cell apoptosis. Sections were prepared from tumours as in (A) at day 56, and were stained by the terminal deoxynucleotidyltransferase-mediated deoxyuridine triphosphate-digoxigenin nick end labeling (TUNEL) method, and also by the annexin-V-fluos method. The number of apoptotic cells detected by TUNEL or annexin-V-fluos staining of tumour sections was determined for 10 randomly selected fields viewed at x40 magnification. The apoptotic index (A/I) is the number of apoptotic (TUNEL or annexin-V-fluos positive) cells x (100/total number of cells). Bars indicate 95% confidence intervals. (D) Effects on tumour angiogenesis. Sections were prepared from mice in (A) at day 56, and stained with either the anti-CD31 mAb MEC13.3 or an anti-CD105 mAb to visualize blood vessels, or alternatively DiO7 was injected into the tail vein one minute prior to collecting tissues in order to visualize blood flow. Stained blood vessels were counted from six mice in six blindly chosen random fields.

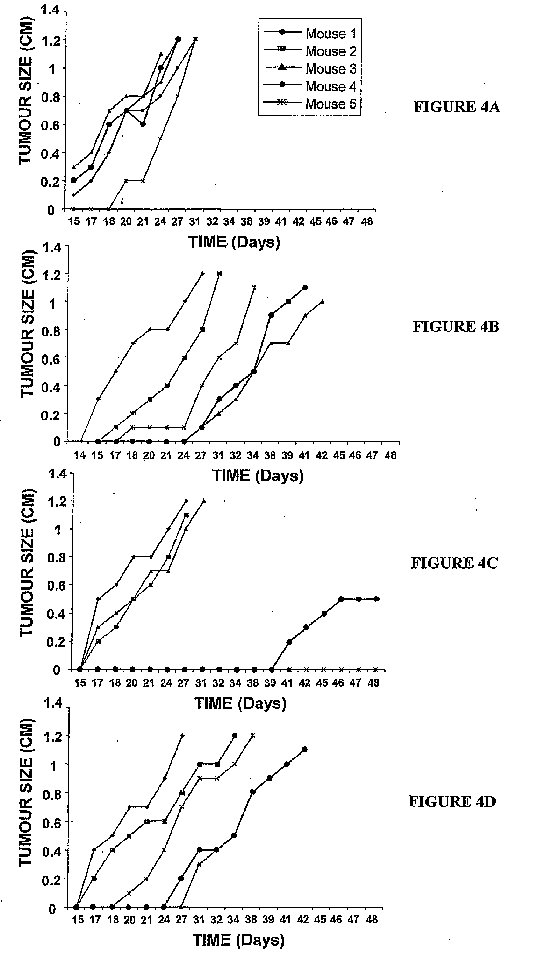

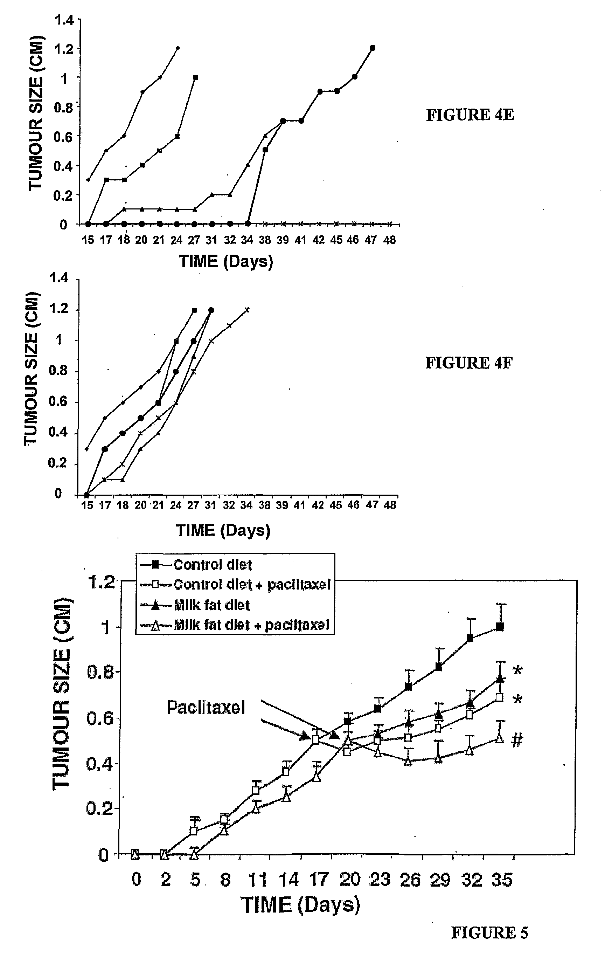

Figure 4 is six graphs showing that alpha lipid powder (Phospholac 600^{™}) and sphingomyelin inhibit the growth of tumours. Mice were fed the control AIN93G diet (A) or the same diet where a proportion of the fat or protein was substituted with either Lf+ (B), Phospholac 600TM (C), sphingomyelin (D), a combination of Lf+ and Phospholac 600TM (E), or a combination of Lf+ and sphingomyelin (F). Tumour size as measured by two perpendicular diameters (in centimetres) is plotted against time for each individual mouse. The time scale on the x-axis is not linear.

Figure 5 is a graph showing that milk fat suppresses the growth of primary breast cancer tumours. Balb/c mice were placed on a milk fat diet or the corresponding control diet. Two weeks later, tumours were established by s.c. injection of 2 x 104 4T1 tumour cells into the right flank of mice. Paclitaxel was administered i.p. when the tumours reached 0.5 cm in diameter. The mice were monitored for tumour growth, and tumour size was measured every three days. *Significant (P < 0.05) difference versus mice fed the control diet. #Significant (P < 0.05) difference versus mice fed the control diet and treated with paclitaxel.

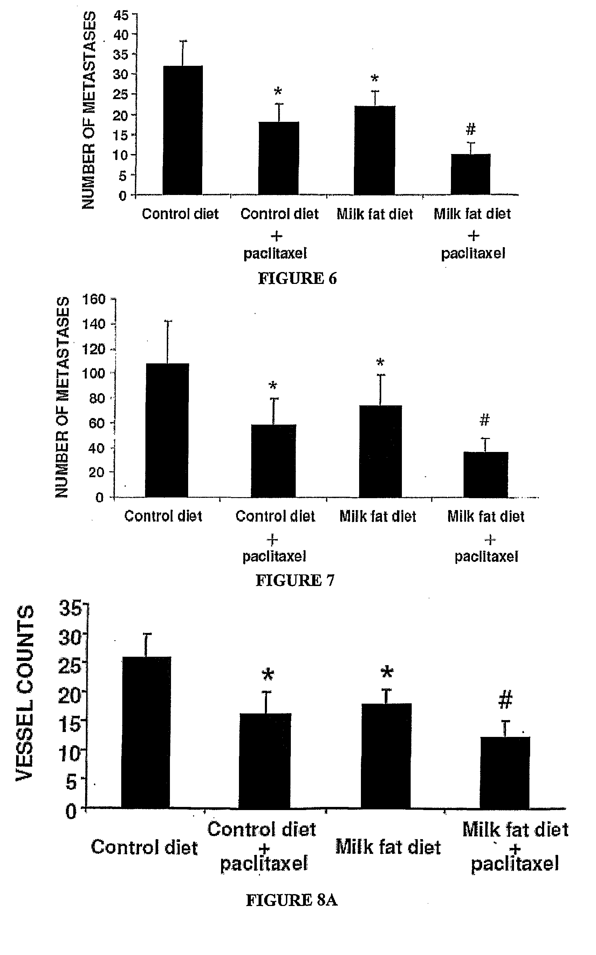

Figure 6 is a graph showing that milk fat suppresses the growth of breast cancer tumours that metastasize to the lung, and augments the effects of paclitaxel. Mice in Figure 5 were euthanased at day 35 and their lungs removed. The numbers of metastatic tumours on the surface of the lungs from mice fed either the control diet, the control diet and treated with paclitaxel, the milk fat diet, or the milk fat diet and treated with paclitaxel were counted, and are expressed as mean number ± SEM. *Significant (P < 0.05) difference versus mice fed the control diet. ^{#}Significant (P < 0.05) difference versus mice fed the control diet and treated with paclitaxel.

Figure 7 is a graph showing that milk fat suppresses the growth of breast cancer tumours that metastasize to the liver, and augments the effects of paclitaxel. Mice in Figure 5 were euthanased at day 35 and their livers removed, sectioned, and stained with hematoxylin/eosin. The numbers of metastatic tumours inside livers from mice fed either the control diet, the control diet and treated with paclitaxel, the milk fat diet, or the milk fat diet and treated with paclitaxel were counted, and are expressed as mean number ± SEM. *Significant (P < 0.05) difference versus mice fed the control diet. #Significant (P < 0.05) difference versus mice fed the control diet and treated with paclitaxel.

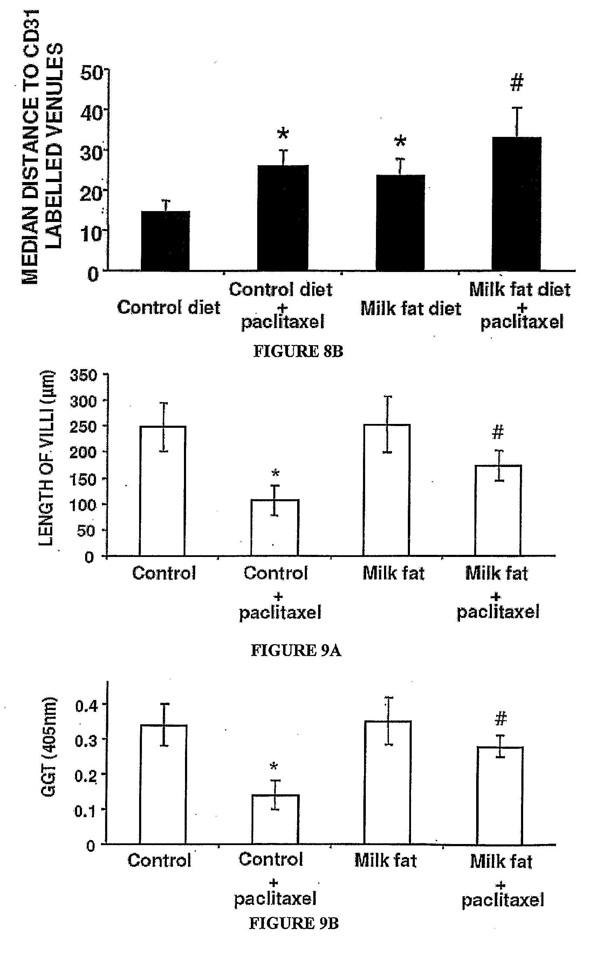

Figure 8 is two graphs showing that milk fat inhibits tumour angiogenesis. 4T1 tumours from mice in Figure 5 were excised, sectioned, and stained with an anti-CD31 mAb to identify vascular endothelial cells. Blood vessels stained with the anti-CD31 mAb were counted in blindly chosen random fields to record mean vessel density (A), or the median distance to the nearest CD31 mAb-labeled blood vessel from an array point (B). Error bars represent ± SEM. *Significant difference (P < 0.05) versus mice fed the control diet. # Significant difference (P < 0.05) versus mice fed the control diet and treated with paclitaxel.

Figure 9 is two graphs showing that milk fat protects against chemotherapy-induced gut damage. Mice in Figure 5 were euthanased at day 35 and their jejuni excised, sectioned, and stained with hematoxylin/eosin. (A) Mean (± SEM) length of the jejunal villi. (B) Mean (± SEM) activity of γ-GGT. *Significant difference (P < 0.05) versus mice fed the control diet. # Significant difference (P < 0.05) versus mice fed the control diet and treated with paclitaxel.

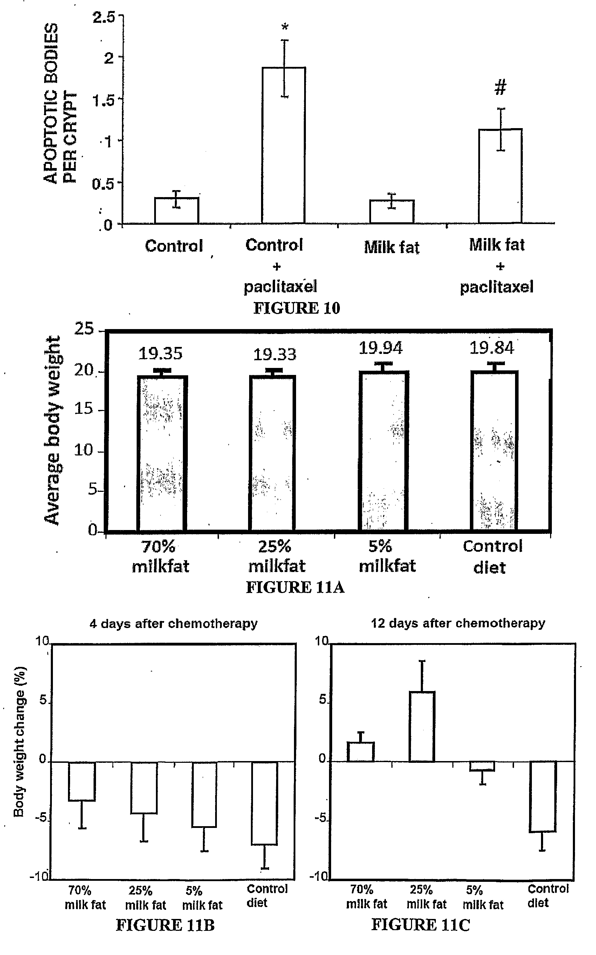

Figure 10 is a graph showing that milk fat prevents chemotherapy-induced apoptosis of gut cells. Jejuni as in Figure 9 were stained by the TUNEL method. Apoptotic bodies were counted in 10 randomly selected crypts and expressed as apoptotic bodies per crypt (mean ± SEM). *Significant difference (P < 0.05) versus mice fed the control diet. # Significant difference (P < 0.05) versus mice fed the control diet and treated with paclitaxel.

Figure 11 is three graphs showing that milk fat inhibits the loss of body weight due to chemotherapy, as described in Example 14. (A) Milk fat in the diet does not significantly increase body weight in healthy mice. Mice were fed the control AIN93G diet or the same diet where a proportion of the fat was substituted with either 70%, 25%, or 5% milk fat for 4 weeks prior to chemotherapy. At the end of the 4 week period the average body weights of the mice were recorded (actual figures provided at the tops of the bars). (B, C) Milk fat in the diet prevents body weight loss due to chemotherapy, and hastens weight gain. After 4 weeks on the above diets the mice in (A) were injected with 300 mg/Kg of cyclophosphamide. The percent change in body weight was recorded 4 (B) and 12 (C) days later and compared to initial body weight. *** P<0.001 and ** P<0.01 compared to control diet.

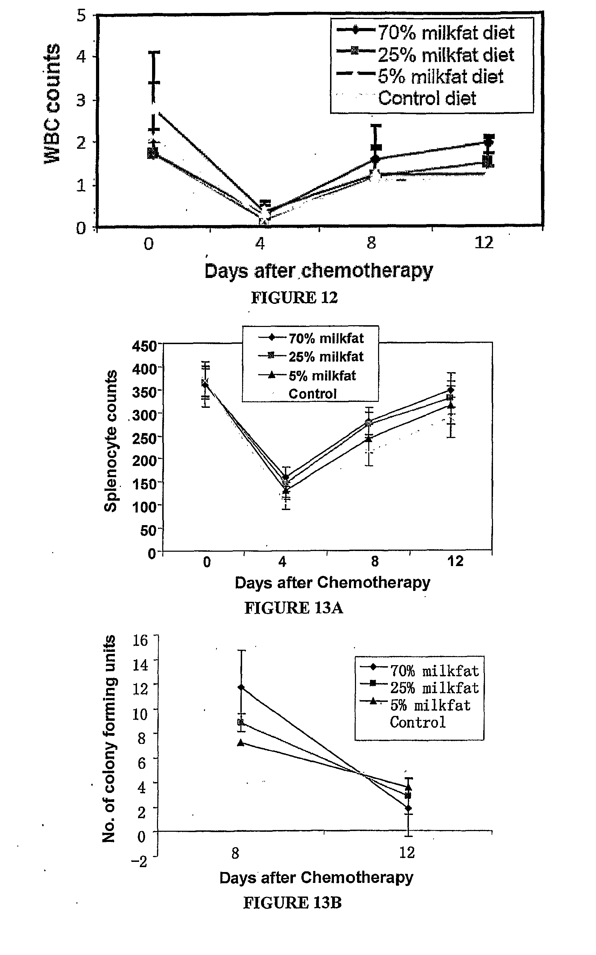

Figure 12 is a graph showing that milk fat at high doses inhibits the loss of peripheral WBC due to chemotherapy, as described in Example 15. The peripheral WBC count was recorded for mice in Figure 11 on the day of injection of cyclophosphamide, and 4, 8, and 12 days later.

Figure 13 is two graphs showing that milk fat inhibits the loss of spleen cellularity due to chemotherapy, and hastens renewal of spleen cellularity, as described in Example 15. (A) Milk fat prevents loss of spleen cellularity. Milk fat had no effect on spleen cellularity when fed to mice for 4 weeks prior to chemotherapy. Significant inhibition of the loss in spleen cellularity was achieved at day 4 after chemotherapy by all three milk fat diets, by the 70% and 25% milk fat diets at day 8, and by the 70% milk fat diet at day 12; compared to the control diet. (B) Milk fat stimulates the formation of splenic colony forming units. Significant stimulation of the formation of splenic colony forming units was achieved at day 8 after chemotherapy by all three milk fat diets, compared to the control diet, whereas by day 12 the situation had reversed as progenitor cells formed by the milk fat diets were no longer required. * Significant difference (P<0.05) versus mice fed the control diet.

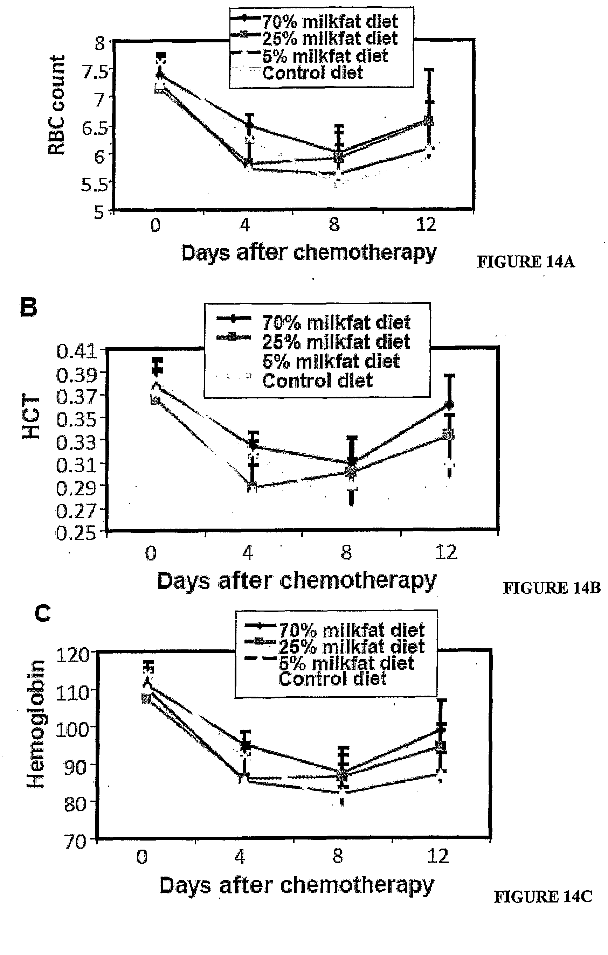

Figure 14 is three graphs showing that the milk fat diets attenuate aspects of anemia, as described in Example 16. The RBC count, the HCT level, and hemoglobin levels in cardiac samples were recorded for mice in Figure 11 on the day of injection of cyclophosphamide, and 4, 8, and 12 days later. (A) Milk fat diets increase the RBC count, but the results are not significant. (B) Milk fat diets increase the HCT level (RBC volume). The two highest doses of milk fat significantly increased HCT levels at days 8 and 12. (C) Milk fat diets increase haemoglobin levels. The two highest doses of milk fat significantly increased hemoglobin levels at day 12, and the 25% milk fat diet also increased haemoglobin levels at day 8.

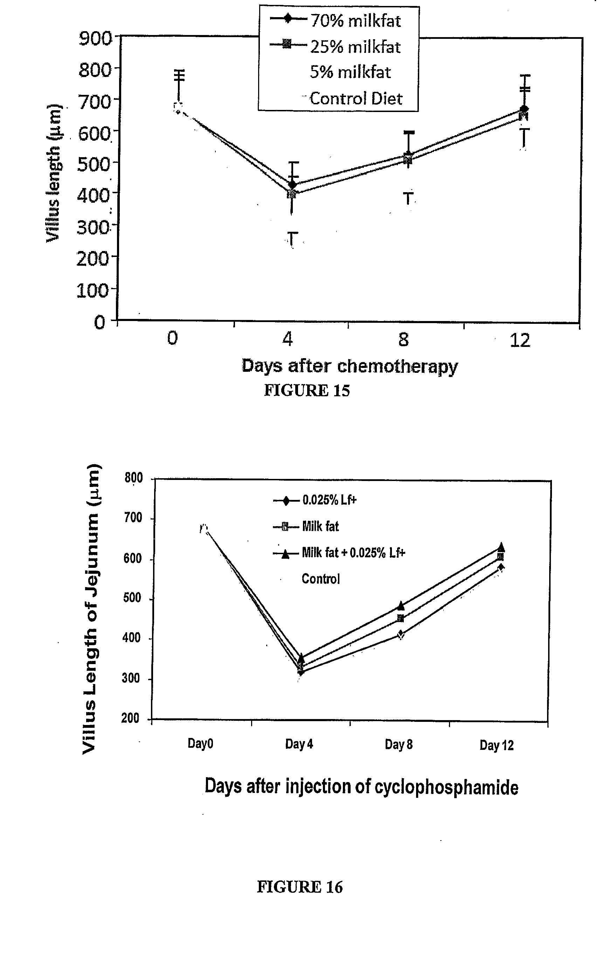

Figure 15 is a graph showing that the milk fat diets prevent damage to the small intestine, as described in Example 17. The lengths of the villi of the jejunum were recorded for mice in Figure 11 on the day of injection of cyclophosphamide, and 4, 8, and 12 days later.

Figure 16 is a graph showing the recovery of the intestinal villi following cyclophosphamide-mediated damage to the small intestine. Mice were fed one of four diets (control diet, and diets substitured with 0.025% Lf+, milk fat, and a combination of the latter), and the lengths of the villi of the jejunum were recorded on the day of injection of cyclophosphamide, and 4, 8, and 12 days later. On day 8, the average villi length for the combination of milk fat and 0.025% Lf+ was significantly greater than for the milk fat only or the 0.025% Lf+ only, providing evidence of a synergistic effect.

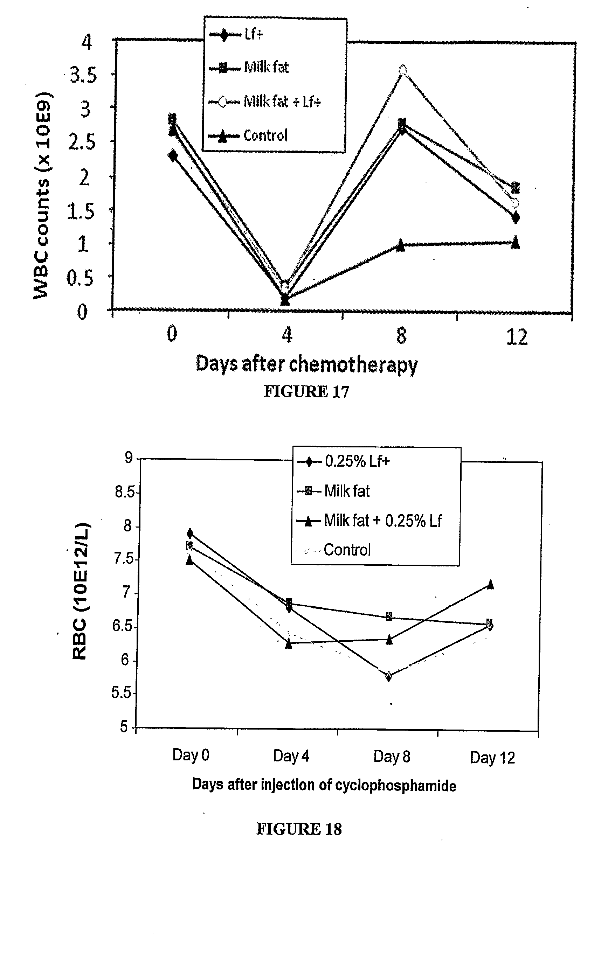

Figure 17 is a graph showing the WBC counts recovered faster following cyclophosphamide chemotherapy for mice fed diets containing milk fat, 0.025% Lf+, or a combination of the latter than for mice fed the control diet. WBC counts were recorded in cardiac blood samples from mice in Figure 16 on the day of injection of cyclophosphamide, and 4, 8, and 12 days later. The increase in WBC count between day 4 and day 8 for the combination of milk fat and 0.025% Lf+ was significantly greater than for the milk fat only or the 0.025% Lf+ only, providing evidence of a synergistic effect.

Figure 18 is a graph showing that the RBC counts recover faster following cyclophosphamide chemotherapy for mice fed diets containing milk fat, 0.25% Lf+, or a combination of the latter than for mice fed the control diet. RBC counts were recorded in cardiac blood samples from mice in Figure 16 on the day of injection of cyclophosphamide, and 4, 8, and 12 days later. On day 12, the RBC count for the combination of milk fat and 0.25% Lf+ was significantly greater than for the milk fat only or the 0.25% Lf+ only, providing evidence of a synergistic effect.

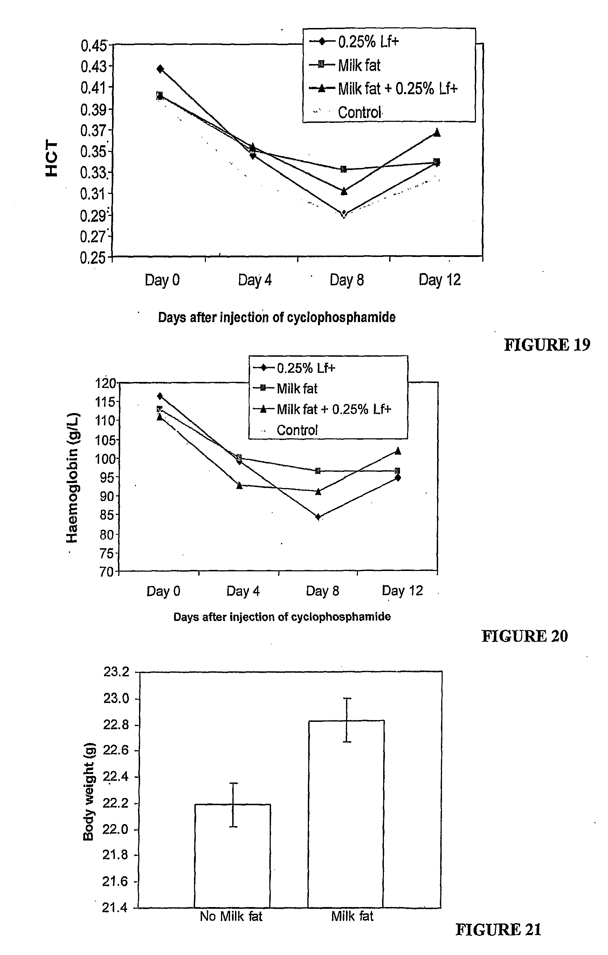

Figure 19 is a graph showing that HCT recovers faster following cyclophosphamide chemotherapy for mice fed diets containing milk fat, 0.25% Lf+, or a combination of the latter than for mice fed the control diet. HCT (RBC volume) was recorded in cardiac blood samples from mice in Figure 16 on the day of injection of cyclophosphamide, and 4, 8, and 12 days later. On day 12, the HCT for the combination of milk fat and 0.25% Lf+ was significantly greater than for the milk fat only or the 0.025% Lf+ only, providing evidence of a synergistic effect.

Figure 20 is a graph showing that hemoglobin recovers faster following cyclophosphamide chemotherapy for mice fed diets containing milk fat, 0.25% Lf+, or a combination of the latter than for mice fed the control diet. Hemoglobin was recorded in cardiac blood samples from mice in Figure 16 on the day of injection of cyclophosphamide, and 4, 8, and 12 days later. On day 12, the hemoglobin for the combination of milk fat and 0.25% Lf+ was greater than for the milk fat only or the 0.25% Lf+ only. The comparison with 0.25% Lf only was significant.

Figure 21 is a graph showing that milk fat increases body weight following cyclophosphamide chemotherapy for mice fed diets containing milk fat with or without Lf+. Body weights were recorded in mice in Figure 16 on the day of injection of cyclophosphamide, and 4, 8, and 12 days later. When records from all days were pooled, the weights for any mice supplemeted with milk fat were significantly greater than for the groups not supplemented with milk fat.

### DETAILED DESCRIPTION OF THE INVENTION

### 1. Definitions

The terms "anhydrous milk fat" and "AMF" are used interchangeably herein and refer to the milk fat fraction produced by phase inversion of cream, or from melted butter. Milk fat may be any mammalian milk fat including but not limited to bovine, sheep, goat, pig, mouse, water buffalo, camel, yak, horse, donkey, llama or human milk fat, with bovine milk fat being a preferred source. Methods commonly used for the preparation of AMF are disclosed in Bylund (Ed., 1995), incorporated herein in its entirety. Preferred AMF is typically about 60%, about 70%, about 80%, about 90%, about 95%, greater than about 95%, about 96%, about 97%, about 98%, about 99%, about 99.5%, or 100% fat, with AMF of about 99% fat, 99.5% fat or greater being more preferred. AMF is frequently further fractionated into "hard"(H) and "soft"(S) fractions, the latter can be further fractionated into "soft hard"(SH) and "soft soft"(SS) fractions, the latter can again be further fractionated into "soft soft hard"(SSH) and "soft soft soft"(SSS) fractions. As will be appreciated, each fraction differs in fatty acid composition. Non-limiting exemplary fatty acid compositions for AMF and derivative fractions are shown in Tables 1 to 5 below.

**Table 1. Exemplary AMF composition**

| **Fatty acid component** | **Mean (%w/w)** | **Min (% w/w)** | **Max (% w/w)** |
|---|---|---|---|
| c4:0 (butyric acid) | 3.6 | 3.3 | 4.1 |
| c6:0 (caproic acid) | 2.2 | 1.9 | 2.4 |
| c8:0 (caprylic acid) | 1.2 | 1.1 | 1.4 |
| c10:0 (capric acid) | 2.6 | 2.2 | 2.8 |
| c10:1 (2-decenoate) | 0.3 | 0.3 | 0.3 |
| c12:0 (lauric acid) | 2.9 | 2.5 | 3.2 |
| c12:1 (11-dodecenoic acid) | 0.1 | 0.1 | 0.1 |
| c 13:0 br (tridecanoic acid br) | 0.1 | 0.1 | 0.1 |
| c13:0 (tridecanoic acid) | 0.1 | 0.1 | 0.1 |
| c 14:0 br (myristic acid br) | 0.2 | 0.1 | 0.2 |
| c14:0 (myristic acid) | 10.4 | 9.5 | 10.8 |
| c14:1 (myristoleic acid) | 0.9 | 0.6 | 1.0 |
| c15:0 iso | 0:4 | 0.3 | 0.5 |
| c15:0 ante-iso | 0.6 | 0.5 | 0.7 |
| c15:0 (pentadecanoic acid) | 1.4 | 1.1 | 1.5 |
| c16:0 br | 0.3 | 0.2 | 0.3 |
| c16:0 (palmitic acid) | 28.7 | 25.4 | 30.4 |
| c16:1 (palmitoleic acid) | 1.9 | 1.6 | 2.0 |
| c17:0 iso | 0.7 | 0.6 | 0.7 |
| c17:0 ante-iso | 0.5 | 0.5 | 0.5 |
| c17:0 (margaric acid) | 0.7 | 0.6 | 0.8 |
| c17:1 | 0.3 | 0.3 | 0.4 |
| c18:0 (stearic acid) | 11.5 | 10.8 | 13.6 |
| c18:1 (oleic acid) | 23.4 | 21.8 | 26.4 |
| c18:2 (linoleic acid) | 1.4 | 1.3 | 1.7 |
| c18:2 conj | 1.3 | 1.0 | 1.8 |
| c18:3 | 0.8 | 0.7 | 0.9 |
| c20:0 (arachidic acid) | 0.2 | 0.1 | 0.2 |
| c20:1 | 0.3 | 0.2 | 0.3 |

**Table 2. Exemplary Fraction H composition**

| **Fatty acid component** | **Mean (% w/w)** | **Min (% w/w)** | **Max (% w/w)** |
|---|---|---|---|
| c4:0 (butyric acid) | 2.0 | 1.8 | 2.1 |
| c6:0 (caproic acid) | 1.3 | 1.2 | 1.4 |
| c8:0 (caprylic acid) | 0.8 | 0.8 | 0.9 |
| c10:0 (capric acid) | 2.2 | 1.9 | 2.4 |
| c10:1 (2-decanoate) | 0.2 | 0.1 | 0.2 |
| c12:0 (lauric acid) | 3.0 | 2.6 | 3.4 |
| c12:1 (11-dodecenoic acid) | 0.0 | 0.0 | 0.1 |
| c13:0 br (tridecanoic acid br) | 0.1 | 0.1 | 0.1 |
| c13:0 (tridecanoic acid) | 0.1 | 0.1 | 0.1 |
| c14:0 br (myristic acid br) | 0.1 | 0.1 | 0.2 |
| c14:0 (myristic acid) | 11.8 | 10.7 | 12.6 |
| c14:1 (myristoleic acid) | 0.6 | 0.3 | 0.7 |
| c15:0 iso | 0.4 | 0.3 | 0.5 |
| c15:0 ante-iso | 0.5 | 0.4 | 0.6 |
| c 15:0 (pentadecanoic acid) | 1.6 | 1.2 | 1.7 |
| c16:0 br | 0.3 | 0.3 | 0.3 |
| c16:0 (palmitic acid) | 34.8 | 31.5 | 36.6 |
| c16:1 (palmitoleic acid) | 1.3 | 1.1 | 1.6 |
| c17:0 iso | 0.8 | 0.7 | 0.8 |
| c17:0 ante-iso | 0.5 | 0.5 | 0.6 |
| c17:0 (margaric acid) | 0.9 | 0.8 | 0.9 |
| c17:1 | 0.2 | 0.2 | 0.3 |
| c18:0 (stearic acid) | 15.2 | 13.9 | 19.7 |
| c18:1 (oleic acid) | 17.0 | 15.5 | 19.8 |
| c18:2 (linoleic acid) | 1.3 | 1.1 | 1.5 |
| c18:2 conj | 0.8 | 0.6 | 1.1 |
| c18:3 | 0.5 | 0.4 | 0.6 |
| c20:0 (arachidic acid) | 0.2 | 0.2 | 0.3 |
| c20:1 | 0.2 | 0.1 | 0.2 |

**Table 3. Exemplary Fraction SH composition**

| **Fatty acid component** | **Mean (% w/w)** | **Min (% w/w)** | **Max (% w/w)** |
|---|---|---|---|
| c4:0 (butyric acid) | 4.0 | 3.7 | 4.3 |
| c6:0 (caproic acid) | 2.4 | 2.1 | 2.6 |
| c8:0 (caprylic acid) | 1.2 | 1.1 | 1.4 |
| c10:0 (capric acid) | 2.4 | 2.2 | 2.7 |
| c10:1 (2-decenoate) | 0.3 | 0.2 | 0.3 |
| c12:0 (lauric acid) | 2.5 | 2.3 | 2.7 |
| c12:1 (11-dodecenoic acid) | 0.1 | 0.0 | 0.1 |
| c13:0 br (tridecanoic acid br) | 0.1 | 0.1 | 0.1 |
| c13:0 (tridecanoic acid) | 0.1 | 0.1 | 0.1 |
| c14:0 br (myristic acid br) | 0.1 | 0.1 | 0.2 |
| c14:0 (myristic acid) | 9.8 | 9.0 | 10.3 |
| c14:1 (myristoleic acid) | 0.8 | 0.5 | 0.9 |
| c15:0 iso | 0.4 | 0.3 | 0.4 |
| c15:0 ante-iso | 0.5 | 0.4 | 0.6 |
| c 15:0 (pentadecanoic acid) | 1.4 | 1.1 | 1.5 |
| c16:0 br | 0.2 | 0.2 | 0.3 |
| c16:0 (palmitic acid) | 32.8 | 29.8 | 34.0 |
| c16:1 (palmitoleic acid) | 1.5 | 1.3 | 1.8 |
| c17:0 iso | 0.6 | 0.6 | 0.7 |
| c17:0 ante-iso | 0.4 | 0.4 | 0.5 |
| c17:0 (margaric acid) | 0.8 | 0.8 | 0.9 |
| c17:1 ' | 0.3 | 0.2 | 0.3 |
| c18:0 (stearic acid) | 13.2 | 12.5 | 16.1 |
| c18:1 (oleic acid) | 19.5 | 17.4 | 22.2 |
| c18:2 (linoleic acid) | 1.3 | 1.2 | 1.5 |
| c18:2 conj | 1.2 | 1.0 | 1.6 |
| c18:3 | 0.7 | 0.6 | 0.7 |
| c20:0 (arachidic acid) | 0.2 | 0.2 | 0.3 |
| c20:1 | 0.2 | 0.2 | 0.3 |

**Table 4. Exemplary Fraction SSH composition**

| **Fatty acid component** | **Mean (% w/w)** | **Min (% w/w)** | **Max (%w/w)** |
|---|---|---|---|
| c4:0 (butyric acid) | 4.0 | 3.9 | 4.3 |
| c6:0 (caproic acid) | 2.4 | 2.2 | 2.6 |
| c8:0 (caprylic acid) | 1.4 | 1.2 | 1.6 |
| c10:0 (capric acid) | 2.8 | 2.4 | 3.4 |
| c10:1 (2-decenoate) | 0.3 | 0.3 | 0.3 |
| c12:0 (lauric acid) | 3.2 | 2.7 | 3.8 |
| c12:1 (11-dodecenoic acid) | 0.1 | 0.1 | 0.1 |
| c13:0 br (tridecanoic acid br) | 0.1 | 0.1 | 0.1 |
| c13:0 (tridecanoic acid) | 0.1 | 0.1 | 0.1 |
| c14:0 br (myristic acid br) | 0.2 | 0.1 | 0.2 |
| c14:0 (myristic acid) | 11.5 | 10.6 | 12.2 |
| c14:1 (myristoleic acid) | 0.9 | 0.7 | 1.0 |
| c15:0 iso | 0.4 | 0.4 | 0.5 |
| c15:0 ante-iso | 0.6 | 0.6 | 0.7 |
| c15:0 (pentadecanoic acid) | 1.4 | 1.2 | 1.5 |
| c16:0 br | 0.3 | 0.2 | 0.3 |
| c16:0 (palmitic acid) | 28.6 | 25.7 | 30.0 |
| c16:1 (palmitoleic acid) | 1.8 | 1.6 | 2.0 |
| c17:0 iso | 0.7 | 0.6 | 0.7 |
| c17:0 ante-iso | 0.5 | 0.5 | 0.5 |
| c17:0 (margaric acid) | 0.7 | 0.6 | 0.8 |
| c17:1 | 0.3 | 0.3 | 0.4 |
| c18:0 (stearic acid) | 10.6 | 10.2 | 11.3 |
| c18:1 (oleic acid) | 22.2 | 20.3 | 24.8 |
| c18:2 (linoleic acid) | 1.4 | 1.3 | 1.5 |
| c18:2 conj | 1.3 | 1.1 | 117 |
| c18:3 | 0.8 | 0.8 | 1.0 |
| c20:0 (arachidic acid) | 0.2 | 0.1 | 0.2 |
| c20:1 | 0.2 | 0.0 | 0.3 |

**Table 5. Exemplary Fraction SSS composition**

| **Fatty acid component** | **Mean (%w/w)** | **Min (% w/w)** | **Max (% w/w)** |
|---|---|---|---|
| c4:0 (butyric acid) | 4.4 | 4.0 | 4.7 |
| c6:0 (caproic acid) | 2.7 | 2.4 | 2.8 |
| c8:0 (caprylic acid) | 1.6 | 1.4 | 1.8 |
| c10:0 (capric acid) | 3.4 | 2.8 | 3.7 |
| c10:1 (2-decenoate) | 0.4 | 0.3 | 0.4 |
| c12:0 (lauric acid) | 3.7 | 3.2 | 4.1 |
| c12:1 (11-dodecenoic acid) | 0.1 | 0.1 | 0.1 |
| c13:0 br (tridecanoic acid br) | 0.2 | 0.1 | 0.2 |
| c13:0 (tridecanoic acid) | 0.1 | 0.1 | 0.1 |
| c14:0 br (myristic acid br) | 0.2 | 0.2 | 0.2 |
| c14:0 (myristic acid) | 10.2 | 9.5 | 11.0 |
| c14:1 (myristoleic acid) | 1.2 | 0.8 | 1.3 |
| c15:0 iso | 0.5 | 0.4 | 0.5 |
| c15:0 ante-iso | 0.8 | 0.7 | 0.9 |
| c15:0 (pentadecanoic acid) | 1.1 | 0.9 | 1.2 |
| c16:0 br | 0.3 | 0.2 | 0.3 |
| c16:0 (palmitic acid) | 20.0 | 18.4 | 21.1 |
| c16:1 (palmitoleic acid) | 2.6 | 2.2 | 3.0 |
| c17:0 iso | 0.6 | 0.5 | 0.6 |
| c17:0 ante-iso | 0.5 | 0.5 | 0.5 |
| c17:0 (margaric acid) | 0.4 | 0.4 | 0.5 |
| c17:1 | 0.5 | 0.5 | 0.6 |
| c18:0 (stearic acid) | 6.7 | 5.8 | 7.8 |
| c18:1 (oleic acid) | 30.8 | 28.2 | 33.4 |
| c18:2 (linoleic acid) | 1.9 | 1.7 | 2.1 |
| c18:2 conj | 1.7 | 1.3 | 2.3 |
| c18:3 | 1.3 | 1.1 | 1.4 |
| c20:0 (arachidic acid) | 0.1 | 0.1 | 0.1 |
| c20:1 | 0.3 | 0.1 | 0.4 |

The terms "anti-tumour food factor", "anti-tumour food" and "anti-tumour flood component" refer to foods and food components that are capable of inhibiting tumour formation or growth and preferably capable of augmenting the ability of milk fat and/or lactoferrin to inhibit tumour formation or growth.

The term "anti-tumour factors" refers at least to apoptosis inducing factors and may include anti-tumour cytolytic antibodies and tumoricidal cytokines such as TNF-α.

The term "anti-tumour immune response" refers to the ability of milk fat or lactoferrin to stimulate the generation of antigen-specific cytolytic activity (the activity of immune cells, particularly cytotoxic T-lymphocytes) and/or NK cell activity, improve the cellular immune response to antigens (through the activity of at least cytotoxic T-lymphocytes), improve immune protection (by at least restoring the activity of cytotoxic T-lymphocytes and/or NK cells and enhancing cytokine production), restore immune protection (by at least restoring or stimulating the activity of cytotoxic T-lymphocytes and/or NK cell activity and enhancing cytokine production), generate pro-inflammatory and immunoregulatory mediators (Th1 and Th2 cytokines), and/or generate anti-tumour cytolytic antibodies and tumoricidal cytokines such as TNF-α.

The term "anticachectic agent" and its grammatical variant anticachetic agent means an agent capable of reversing, retarding or halting cachexia or having activity to relieve one or more of the symptoms of cachexia including progressive loss of body weight (inclusive of weight loss due to lipolysis and weight loss due to myolysis), anemia, edema, and anorexia in a subject. Anticachectic agents include cyclooxygenase inhibitors (e.g. indomethacin) corticosteroids and glucocorticoids, such as prednisolone, methylprednisolone, and dexamethasone, progestational agents, such as megestrol acetate, medroxyprogesterone acetate, cannabinoids, such as tetrahydrocannabinols and dronabinol, serotonin antagonists, such as cyproheptadine, prokinetic agents, such as metoclopramide and cisapride, anabolic steroids, such as nandrolone decanoate and fluoxymesterone, inhibitors of phosphoenolpyruvate carboxykinase, such as hydrazine sulfate, methylxanthine analogs, such as pentoxifylline and lisofylline, thalidomide, cytokines and anticytokines, such as Anti-IL-6 antibody, IL-12, branched-chain amino acids, lipid metabolism improving agents, such as eicosapentanoic acid, inhibitors of prostaglandin synthesis, such as indomethacin and ibuprofen, hormones, such as melatonin, β2-adrenoceptor agonists such as clenbuterol, metoclopramides, growth hormone, IGF-1, and antibodies to the cachexia-inducing factors TNF-alpha, LIF, IL-6, and oncostatin M.

The term "antimucositic agent" means an agent capable of ameliorating gut damage such as aloeration or reversing, retarding or halting mucositis or having activity to relieve the symptoms of mucositis.

The term "comprising" as used in this specification means "consisting at least in part of". When interpreting statements in this specification that include that term, the features, prefaced by that term in each statement, all need to be present but other features can also be present. Related terms such as "comprise" and "comprised" are to be interpreted in the same manner.

An "effective amount" is the amount required to confer therapeutic effect. The interrelationship of dosages for animals and humans (based on milligrams per meter squared of body surface) is described by Freireich, et al. (1966). Body surface area can be approximately determined from height and weight of the subject. See, e.g., Scientific Tables, Geigy Pharmaceuticals, Ardley, New York, 1970, 537. Effective doses also vary, as recognized by those skilled in the art, dependent on route of administration, excipient usage, and the like.

The terms "enhance the immune system" and "stimulate the immune system" (and different tenses of these terms) refer to the ability of milk fat to stimulate the generation of antigen-specific cytolytic activity (the activity of immune cells, particularly cytotoxic T-lymphocytes) and/or NK cell activity, improve the cellular immune response to antigens (through the activity of at least cytotoxic T-lymphocytes), improve immune protection (by at least restoring the activity of cytotoxic T-lymphocytes and/or NK cells and enhancing cytokine production), restore immune protection (by at least restoring or stimulating the activity of cytotoxic T-lymphocytes and/or NK cell activity and enhancing cytokine production) or generate pro-inflammatory and immunoregulatory mediators (Th1 and Th2 cytokines).

The term "functional lactoferrin fragment" is intended to mean a naturally occurring or non-naturally occurring portion of a lactoferrin polypeptide that has activity when assayed according the examples below, and includes metal ion functional fragments. Useful lactoferrin fragments include truncated lactoferrin polypeptides, metal ion-binding hydrolysates of lactoferrin, fragments that comprise the N-lobe metal ion binding pocket, fragments that comprise the C-lobe metal ion binding pocket, and metal ion-binding fragments generated (by artificial or natural processes) and identified by known techniques as discussed below. Published international patent applications WO 2006/054908 and WO 2007/043900 report preparation and use of lactoferrin fragments and are incorporated herein by reference.

The term "functional lactoferrin variant" is intended to mean a variant of a lactoferrin polypeptide that has activity when assayed according the examples below, and includes metal ion functional variants.

The term "glycosylated" when used in relation to a lactoferrin polypeptide, functional variant or functional fragment is intended to mean that the lactoferrin is fully or partially glycosylated with naturally occurring or non-naturally occurring human or bovine glycosyl groups. Glycosylated and aglycosyl forms of lactoferrin are known (see Pierce, et al. (1991); Metz-Boutigue, et al. (1984); van Veen, et al. (2004)).

The term "hematopoietic agent" means an agent capable of regulating and preferably stimulating hematopoiesis and/or lymphopoiesis, and includes agents which improve the quality of the blood, whether by increasing the number of blood cells such as erythrocytes, lymphocytes or myeloid cells, or by increasing the level of hemoglobin. Preferred hematopoietic agents are useful in the treatment of anemia. Exemplary hematopoietic agents include hematopoietic, lymphopoietic, and myeloid growth factors and recombinant equivalents, such as erythropoietin including epoetin alpha, thrombopoietin, IL-1-12, IL-20granulocyte/macrophage colony-stimulating factor (GM-CSF) including sargramostim (LEUKINE), monocyte/macrophage colony-stimulating factore (M-CSF or CSF-1), macrophage colony-stimulating factore (M-CSF) granulocyte colony-stimulating factor (G-CSF) including filgrastim (NEUPOGEN), stem cell factor (SCF), FTL-3 ligand (FL), iron and iron salts such as ferrous sulphate, ferrous fumarate, ferrous gluconate etc, ferric edetate, iron dextran, sodium ferric gluconate complex (FERRLECIT), pyridoxine, riboflavin, vitamins including B12, and folic acid. See also Goodman & Gilman's The Pharmacological Basis of Therapeutics, 10th Edition, Harman JG and Limbird LE eds., McGraw-Hill, New York, Chapter 54. Hematopoietic Agents: Growth Factors, Minerals, and Vitamins, pp1487-1517.

The term "increasing the responsiveness of a subject" is intended to mean that a subject exhibits a greater reduction in the rate of tumour growth, in tumour size, or in clinical symptoms of disease than a subject who is not subjected to a method of the invention. In one embodiment, the treated subject also benefits from one or more of restored vitamin status, reduced time on chemotherapy, reduced chemotherapy dose, increased immune stamina, increased nutritional health, reduced cachexia, reduced mucositis, reduced anemia, reduced hematological suppression, or increased hemapoesis.

The term "increasing the sensitivity of a tumour" is intended to mean that a tumour exhibits a greater reduction in the rate of tumour growth, in tumour size, or is eradicated whereas a tumour that is not subjected to a method of the invention will not exhibit these effects.

The term "immunotherapeutic agent" is intended to mean an agent that stimulates anti-tumour immunological activity, also referred to herein as anti-tumour immunity and anti-tumour immune rosponse(s). Agents that stimulate anti-tumour immunological activity are preferably those that directly or indirectly stimulate T-cells and/or NK cells to kill tumour cells. An exemplary in vitro assay for assessing whether a selected agent stimulates anti-tumour immunological activity is the CTL assay described below.

The term "inhibiting tumour formation" is intended to mean that tumours do not form, or that tumours form but do not establish or grow, or that tumours form but remain small, benign and do not become cancerous or metastasize, or that tumours grow more slowly. Tumour formation may be monitored through CT scans and tumor markers where available.

The term "inhibiting tumour growth" is intended to mean that tumours do not form in a subject treated according to the invention, or that one or more tumours that may be present in a subject treated according to the invention do not grow in size or become cancerous or metastasize, or that one or more tumours present in a subject treated according to the invention reduce in size (preferably by at least about 20, 30, 40, 50, 60, 70, 80, 90 or 100% by volume) or that one or more tumours present in a subject treated according to the invention are eradicated. Tumour size may be monitored through CT scans and tumor markers where available.

The terms "iron-lactoferrin" and "iron-saturated lactoferrin" as used herein are intended to refer to a population of lactoferrin polypeptides providing a population of iron-binding pockets where at least about 25, 30, 35, 40, 45, 50, 55, 60, 65, 70, 75, 80, 85, 90, 91, 92, 93, 94, 95, 96, 97, 98, 99, 99.5, 99.9 or 100% of the metal ion-binding pockets present in the population have an iron ion bound.

The term "lactoferrin polypeptide" refers to a non-glycosylated or glycosylated wild-type lactoferrin amino acid sequence or homologous lactoferrin sequences from other species such as those described below. A lactoferrin polypeptide has two metal-ion binding pockets and so can bind metal ions in a stoichiometric ratio of 2 metal ions per lactoferrin molecule. One metal ion-binding pocket is present in the N-terminal lobe (N-Iobe) of lactoferrin and the other pocket is present in the C-terminal lobe (C-lobe) (Moore et al, 1997). Verified sequences of bovine and human lactotransferrins (lactoferrin precursors), lactoferrins and peptides therein can be found in Swiss-Prot (http://au.expasy.org/cgi-bin/sprot-search-ful). Indicative lactoferrin polypeptides include the bovine lacto,transferrin precursor accession number P24627, bovine lactoferrin, the human lactotransferrin precursor accession number P02788 and human lactoferrin. Published international patent applications WO 2006/054908 and WO 2007/043900 report preparation and use of lactoferrin polypeptides and amino acid sequences thereof, and each application is incorporated herein by reference. Lactoferrin polypeptides may bind "natural" levels of metal ions, typically iron ions. For example, bovine lactoferrin is naturally about 10% to 20% (preferably 15%) iron saturated. Apo-lactoferrin and lactoferrin of at least 1 % metal ion saturation is useful herein.

The term "large tumour" is intended to mean a tumour that is refractory to therapy with one at least one immunotherapeutic, anti-angiogenic or chemotherapeutic agent, preferably refractory to therapy with at least one at least one immunotherapeutic or chemotherapeutic agent. In one embodiment a large tumour is a tumour that is at least about 0.3, 0.4, 0.5, 0.6, 0.7 or 0.8 cm in diameter. In one embodiment a large tumour is a tumour that is about 0.3 to about 0.8, about 0.4 to about 0.8, about 0.5 to about 0.8, about 0.6 to about 0.8 or about 0.7 to about 0.8 cm in diameter. In one embodiment a large tumour is a tumour that is refractory to therapy by immunotherapy or anti-angiogenic therapy or chemotherapy.

The term "metal ion-binding" is intended to refer to binding of a metal ion in an iron binding pocket of a lactoferrin polypeptide or in an iron binding pocket of a fragment of a lactoferrin polypeptide that is still able to form the iron binding pocket.

The terms "metal ion lactofen-in" and "metal ion-saturated lactoferrin" are intended to refer to a population of lactoferrin polypeptides that provide a population of metal ion-binding pockets where at least about 25% of the metal ion-binding pockets present in the population have a metal ion bound. It should be understood that the population may contain polypeptides of different species; for example, some molecules binding no ion and others each binding one or two ions. In cases where different metal ions are used, some molecules may bind a metal ion selected from, for example, the group comprising aluminium, bismuth, copper, chromium, cobalt, gold, iron, manganese, osmium, platinum, ruthenium, zinc ions, or other ions that will coordinate specifically in a lactoferrin metal ion binding pocket, and others may bind a different ion. In some cases, the population may comprise polypeptides involved in non-specific ion binding, where one or more ions, preferably metal ions, are non-specifically bound, i.e., not bound in the metal-ion binding pocket, to the polypeptide. Non-limiting examples of ions that may be non-specifically bound to lactoferrin polypeptides are calcium and selenium.

Equally, the terms "metal ion lactoferrin fragment" and "metal ion-saturated lactoferrin fragment" are intended to refer to a population of lactoferrin polypeptide fragments that provide a population of metal ion-binding pockets where at least about 25% of the metal ion-binding pockets present in the population have a metal ion bound.

The present invention may employ a mixture of lactoferrin polypeptides and lactoferrin fragments. In such an embodiment, the population of metal ion-binding pockets is made up of two pockets for every lactoferrin polypeptide and one or two pockets for every lactoferrin fragment, depending on the nature of the fragments.

The degree of saturation may be determined by spectrophotometric analysis (Brock & Arzabe, 1976; Bates et al, 1967; Bates et al, 1973). It should be understood that there may be metal ion-exchange between lactoferrin polypeptides. In one embodiment, iron saturated lactoferrin may be prepared by the method of Law et al., (1977). In another embodiment, iron saturated lactoferrin may be prepared by the method of Kawakami et al (1993). Metal-ion saturated lactoferrin may be prepared by binding metal ions to the metal ion binding sites in lactoferrin, including the metal ion binding pockets such as the Fe binding pockets and other non-specific binding sites on the lactoferrin molecule or lactoferrin fragment.

In one embodiment at least about 25, 30, 35, 40, 45, 50, 55, 60, 65, 70, 75, 80, 85, 90, 91, 92, 93, 94, 95, 96, 97, 98, 99, 99.5, 99.9 or 100% of the metal ion-binding pockets present in the population of lactoferrin molecules have a metal ion bound and useful ranges may be selected between any of the foregoing values (for example, about 25 to about 100%, about 30 to about 100%, about 35 to about 100%, about 40 to about 100%, about 45 to about 100%, about 50 to about 100%, about 55 to about 100%, about 60 to about 100%, about 65 to about 100%, about 70 to about 100%, about 75 to about 100%, about 80 to about 100%, about 85 to about 100%, about 90 to about 100%, about 95 to about 100% and about 99 to about 100%). In one embodiment the metal ion lactoferrin is super-saturated lactoferrin.

The term "metal ion lactoferrin functional fragment" is intended to mean a naturally occurring or non-naturally occurring portion of a lactoferrin polypeptide that has one or two metal ion binding pockets and that has activity when assayed according the examples below. Useful lactoferrin fragments include truncated lactoferrin polypeptides, metal ion-binding hydrolysates of lactoferrin, fragments that comprise the N-lobe metal ion binding pocket, fragments that comprise the C-lobe metal ion binding pocket, and metal ion-binding fragments generated (by artificial or natural processes) and identified by known techniques as discussed below.

The term "milk fat" includes mammalian lipids and lipid fractions, lipid hydrolysates, and lipid fraction hydrolysates. Preferred milk fats are dairy fats, particularly bovine milk fats. Preferred milk fat has one or more of palmitic acid, oleic acid, stearic acid, or myristic acid as the most abundant fatty acid(s) present, preferably palmitic, oleic, stearic and myristic acids are the most abundant fatty acids present. In particularly preferred embodiments, the milk fat has a) substantially the same percentage by weight of palmitic acid as does normal bovine milk fat (between about 23%(w/w) and about 32%(w/w), typically about 28%(w/w) - see Table 1.2, PF Fox and PLH McSweeney eds, Advanced Dairy Chemistry Volume 2 - Lipids, 3rd Ed, Springer NY, NY (2006) ISBN-10:0-387-26364-0); b) substantially the same percentage by weight of oleic acid as does normal bovine milk fat (between about 15%(w/w) and about 22%(w/w), typically about 17%(w/w) - see Fox and McSweeny *ibid*); c) substantially the same percentage by weight of stearic acid as does normal bovine milk fat (between about 10%(w/w) and about 15%(w/w), typically about 12%(w/w) - see Fox and McSweeny *ibid*); d) substantially the same percentage by weight of myristic acid as does normal bovine milk fat (between about 9%(w/w) and about 12%(w/w), typically about 11 %(wlw) - see Fox and McSweeny ibid); e) substantially the same percentage by weight of butyric acid as does normal bovine milk fat (between about 3%(w/w) and about 5%(w/w), typically about 4%(w/w) - see Fox and McSweeny *ibid);* f) any two of a), b), c), d), or e) above; g) any three of a), b), c), d), or e) above; h) any four of a), b), c), d), or e) above; i) each of a), b), c), d), and e) above. Anhydrous milk fat (AMF) is preferred, particularly AMF having substantially the same percentage by weight palmitic, oleic and stearic acid composition as normal bovine milk fat, more preferably substantially the same fatty acid compositions as normal bovine milk fat (see Fox and McSweeny *ibid*).

The term "milk fat analogue" includes any combination of plant, animal or marine oils that is blended to provide one or more of palmitic acid, oleic acid, stearic acid, or myristic acid as the most abundant fatty acid(s) present, preferably palmitic, oleic, stearic and myristic acids are the most abundant fatty acids present, so that the milk fat analogue has a) substantially the same percentage by weight of palmitic acid as does normal bovine milk fat (between about 23%(w/w) and about 32%(w/w), typically about 28%(w/w) - see Fox and McSweeny *ibid*); b) substantially the same percentage by weight of oleic acid as does normal bovine milk fat (between about 15%(w/w) and about 22%(w/w), typically about 17%(w/w) - see Fox and McSweeny *ibid*); c) substantially the same percentage by weight of stearic acid as does normal bovine milk fat (between about 10%(w/w) and about 15%(w/w), typically about 12%(w/w) - see Fox and McSweeny *ibid*); d) substantially the same percentage by weight of myristic acid as does normal bovine milk fat (between about 9%(w/w) and about 12%(w/w), typically about 11 %(w/w) - see Fox and McSweeny *ibid*); e) substantially the same percentage by weight of butyric acid as does normal bovine milk fat (between about 3%(w/w) and about 5%(w/w), typically about 4%(w/w) - see Fox and McSweeny *ibid*); f) any two of a), b), c), d), or e) above; g) any three of a), b), c), d), or e) above; h) any four of a), b), c), d), or e) above; i) each of a), b), c), d), and e) above. Suitable oils may include edible or cooking oils including palm, olive, soybean, canola, corn, sunflower, safflower, peanut, grape seed, sesame, nut, almond, cashew, hazelnut, macadamia, pecan, pistachio, and walnut, and other edibles include acai, amaranth, apricot, argan, artichoke, avocado, babassu, ben, blackcurrant seed, borage seed, borneo tallow nut, bottle gourd, buffalo gourd, carob pod (algaroba), cohune, coriander seed, evening primrose, false flax, hemp, kapok seed, lallemantia, meadowfoam seed, mustard, okra seed (hibiscus seed), perilla seed, pequi, pine nut, poppyseed, prune kernel, pumpkin seed, quinoa, ramtil, rice bran, tea (camellia), thistle, watermelon seed, and wheat germ oils, marine oils including shellfish, fish, anchovy, bailcal, bloater, cacha, carp, eel, eulachon, herring, hoki, hilsa, jack fish, katla, kipper, mackerel, orange roughy, pangas, pilchard, black cod, salmon, sardine, shark, sprat, trout, tuna, whitebait, and swordfish oils, and combinations of any two or more thereof.

The term "oral administration" includes oral, buccal, enteral and intra-gastric administration.

The term "parenteral administration" includes but is not limited to topical (including administration to any dermal, epidermal or mucosal surface), subcutaneous, intravenous, intraperitoneal, intramuscular and intratumoural (including any direct administration to a tumour) administration.

The term "pharmaceutically acceptable carrier" is intended to refer to a carrier including but not limited to an excipient, diluent or auxiliary that can be administered to a subject as a component of a composition of the invention. Preferred carriers do not reduce the activity of the composition and are not toxic when administered in doses sufficient to deliver an effective amount of milk fat, a milk fat derivative or a component thereof, including, for example, cis-9, trans-11 CLA and TVA, or, when administered, of a lactoferrin polypeptide or functional variant or functional fragment thereof. The formulations can be administered orally, nasally or parenterally.

The term "subject" is intended to refer to an animal, preferably a mammal, more preferably a maminalian companion animal or human. Preferred companion animals include cats, dogs and horses.

The term "super-saturated lactoferrin" refers to a population of lactoferrin polypeptides or functional fragments providing a population of metal ion-binding pockets where sufficient metal ions are available to fill 100% of the binding pockets and additional metal ions are present and bound by non-specific binding sites on the lactoferrin polypeptide or lactoferrin fragment. In other words, a stoichiometric excess of metal ions is provided. Preferably no free metal ions are present in a composition of the invention comprising super-saturated lactoferrin, although metal ion exchange between binding pockets, between non-specific binding sites and between binding pockets and non-specific binding sites may occur. Preferably super-saturated lactoferrin does not form insoluble aggregates. In one embodiment the super-saturated lactoferrin is at least about 105, 110, 115, 120, 125, 130, 135, 140, 145, 150, 155, 160,165, 170, 175, 180, 185, 190, 195 or 200% metal ion saturated, preferably iron saturated. Useful saturation ranges include about 25 to about 200%, about 30 to about 200%, about 35 to about 200%, about 40 to about 200%, about 45 to about 200%, about 50 to about 200%, about 55 to about 200%, about 60 to about 200%, about 65 to about 200%, about 70 to about 200%, about 75 to about 200%, about 80 to about 200%, about 85 to about 200%, about 90 to about 200%, about 95 to about 200% and about 100 to about 200% metal ion saturation.

The term "treat" and its derivatives should be interpreted in their broadest possible context. The term should not be taken to imply that a subject is treated until total recovery. Accordingly, "treat" broadly includes maintaining a subject's disease progression or symptoms at a substantially static level, increasing a subject's rate of recovery, amelioration and/or prevention of the onset of the symptoms or severity of a particular condition, or extending a patient's quality of life. The term "treat" also broadly includes the maintenance of good health for sensitive individuals and building stamina for disease prevention.

The term "variant" refers to a naturally occurring (an allelic variant, for example) or non-naturally occurring (an artificially generated mutant, for example) lactoferrin polypeptide or lactoferrin fragment that varies from the predominant wild-type amino acid sequence of a lactoferrin polypeptide of a given species (such as those listed below) or fragment thereof by the addition, deletion or substitution of one or more amino acids.

Generally, polypeptide sequence variant possesses qualitative biological activity in common when assayed according to the examples below. Further, these polypeptide sequence variants may share at least about 50%, 55%, 60%, 65%, 70%, 75%, 80%, 85%, 90%, 95%, 96%, 97%, 98% or 99% sequence identity. Also included within the meaning of the term "variant" are homologues of lactoferrin polypeptides. A homologue is typically a polypeptide from a different species but sharing substantially the same biological function or activity as the corresponding polypeptide disclosed herein.

Preferred variant polypeptides preferably have at least about 70, 75, 80, 85, 90, 95 or 99% identity, preferably at least about 90, 95 or 99% identity to a lactoferrin sequence described herein, including those sequences described in published international patent applications WO 2006/054908 and WO 2007/043900 that are incorporated herein by reference. Variant fragments preferably have at least about 70, 75, 80, 85, 90, 95 or 99% identity, preferably at least about 90, 95 or 99% identity to a fragment described herein, including those sequences described in published international patent applications WO 2006/054908 and WO 2007/043900. Identity can be determined by comparing a candidate amino acid sequence to a sequence described herein, such as a lactoferrin polypeptide or fragment thereof using the BLAST suite of programs (version 2.2.12; 28 August 2005) that is publicly available from NCBI (ftp://ftp.ncbi.nih.gov/blast/).

Conservative substitutions of one or several amino acids of a lactoferrin polypeptide sequence without significantly altering its biological activity are also useful. A skilled artisan will be aware of methods for making phenotypically silent amino acid substitutions (see for example Bowie et al., (1990)).

The term "vitamin D" refers to one or more vitamin D compounds selected from the group comprising vitamin D1 [lumisterol], vitamin D2 [calciferol or ergocalciferol], vitamin D3 [cholecalciferol], vitamin D4 [22-dihydroerogocalciferol] and vitamin D5 [sitocalciferol], and any mixture of any two or more thereof. The term "vitamin D analogue" refers to any compound that will bind and activate a vitamin D receptor (VDR). The VDR is a ligand-activated intracellular receptor that acts as a transcription factor and binds vitamin D response elements (VDREs) in the promoter/enhancer regions of genes including but not limited to genes that exert antiproliferative effects on tumour cells by causing arrest in the G0/G1 phase of the cell cycle, down-regulating growth promoting factors such as IGF-1, up-regulating negative growth regulators such as transforming growth factor beta, causing tumour apoptosis, inhibiting tumour angiogenesis and inhibiting metastasis. Assays for assessing VDR binding are known; for example, immunoassays that measure the expression of genes regulated by vitamin D. Therefore, candidate vitamin D analogues may be readily assessed without undue experimentation for use according to the present invention.

### 2. Dairy lipids and lipid fractions

Dairy lipids are discussed comprehensively by Fox and McSweeney (2006), hereby incorporated by reference. Fraction of dairy lipids is discussed in the Dairy Processing Handbook, 1995 and by Illingworth, 2002, and Rombaut et al, 2006, all hereby incorporated by reference.

Examples of dairy lipid fractions useful according to the invention include cream, butter, anhydrous milk fat (AMF) (typically produced by phase inversion of cream or butter), butter milk, butter serum, hard milk fat fractions, soft milk fat fractions, sphingomyelin fractions, milk fat globular membrane fractions, phospholipid fractions, and complex lipid fractions, and combinations thereof, and hydrolysates thereof.

Multistage fractionation of milk fat may be carried out by differential crystallisation. Milk fat fractions are heated to a set temperature and the crystallised or solid ("stearin") and liquid ("olein") fractions are separated. Multi-step fractionation refers to re-fractionation in a subsequent step of a product of a previous fractionation step.

Other fractionation methods include phase inversion, interesterification, glycerolysis, solvent fractionation, supercritical fractionation, near supercritical fractionation, distillation, centrifugal fractionation, suspension crystallisation, dry crystallisation, fractionation with a modifier (e.g. soaps or emulsifiers), and combinations of these methods.

Lipids present in the compositions of the invention may be fully or partially nodified, whether naturally, chemically, enzymatically, or by any other methods known in the art, including, for example, glycosylated, sialylated, esterified, phosphorylated or hydrolysed.

Lipid hydrolysates may be prepared using known techniques, including but not limited to acid hydrolysis, base hydrolysis, enzymatic hydrolysis using a lipase, for example as described in Fox and McSweeney ((2006), Chapter 15 by HC Deeth and CH Fitz-Gerald), and microbial fermentation. One method of base hydrolysis includes adding 1% KOH (in ethanol) and heating for 10 minutes. Hydrolysed material may be neutralised with acetic acid or hydrochloric acid.

Milk fat globule membrane material may be isolated according to the acidification method of Kanno & Dong-Hyun, 1990, and further fractionated into complex lipid and lipoprotein fractions by the addition of methanol, as described by Kanno et al, 1975. A phospholipid fraction may be isolated by extracting the lipid mixture with acetone according to the procedure of Purthi et al, 1970. Lipid residue may be further enriched in complex lipids by the selective extraction of simple lipids with pentane.

In one embodiment, the milk fat comprises one or more fatty acids selected from butyric acid (C4:0), caproic acid (C6:0), caprylic acid (C8:0), capric acid (C10:0), lauric acid (C12:0), myristic acid (C14:0), palmitic acid (C16:0), palmitoleic acid (C16:1), stearic acid (C18:0), oleic acid (C18:1 cis-9; cis-9-octadecenoic acid), elaidic acid (C18:1 trans-9; trans-9-octadecenoic acid), vaccenic acid (C18:1 trans-11; trans-11-octadecenoic acid), cis-vaccenic acid (C18:1 cis-11; cis-11-octadecenoic acid), arachidic acid (C20:0), and behenic acid (C22:0), optionally substituted with one or more groups selected from hydroxyl, methyl, ethyl and propyl groups, and salts, esters and amides thereof, and combinations thereof. The optional substituent(s) may be present at any position along the carbon chain. Preferred milk fat comprises the fatty acids butyric acid, caproic acid, caprylic acid, capric acid, lauric acid, myristic acid, palmitic acid, palmitoleic acid, stearic acid, oleic acid, and elaidic acid, salts, esters and amides thereof, and combinations thereof. These fatty acids are the major components of bovine milk fat.

In one embodiment the composition comprises, consists essentially of or consists of at least about 0.1, 0.2, 0.5, 1, 5, 10, 15, 20, 25, 30, 35, 40, 45, 50, 55, 60, 65, 70, 75, 80, 85, 90, 95, 99, 99.5, 99.8 or 99.9% by weight of fresh, recombined or powdered whole milk or milk derivative, preferably milk fat, and useful ranges may be selected between any of these foregoing values (for example, from about 0.1 to about 50%, from about 0.2 to about 50%, from about 0.5 to about 50%, from about 1 to about 50%, from about 5 to about 50%, from about 10 to about 50%, from about 15 to about 50%, from about 20 to about 50%, from about 25 to about 50%, from about 30 to about 50%, from about 35 to about 50%, from about 40 to about 50%, from about 45 to about 50%, from about 0.1 to about 60%, from about 0.2 to about 60%, from about 0.5 to about 60%, from about 1 to about 60%, from about 5 to about 60%, from about 10 to about 60%, from about 15 to about 60%, from about 20 to about 60%, from about 25 to about 60%, from about 30 to about 60%, from about 35 to about 60%, from about 40 to about 60%, from about 45 to about 60%, from about 0.1 to about 70%, from about 0.2 to about 70%, from about 0.5 to about 70%, from about 1 to about 70%, from about 5 to about 70%, from about 10 to about 70%, from about 15 to about 70%, from about 20 to about 70%, from about 25 to about 70%, from about 30 to about 70%, from about 35 to about 70%, from about 40 to about 70%, from about 45 to about 70%, from about 0.1 to about 80%, from about 0.2 to about 80%, from about 0.5 to about 80%, from about 1 to about 80%, from about 5 to about 80%, from about 10 to about 80%, from about 15 to about 80%, from about 20 to about 80%. from about 25 to about 80%, from about 30 to about 80%, from about 35 to about 80%, from about 40 to about 80%, from about 45 to about 80%, from about 0.1 to about 90%, from about 0.2 to about 90%, from about 0.5 to about 90%, from about 1 to about 90%, from about 5 to about 90%, from about 10 to about 90%, from about 15 to about 90%; from abut 20 to about 90%, from about 25 to about 90%, from about 30 to about 90%, from about 35 to about 90%, from about 40 to about 90%, from about 45 to about 90%, from about 0.1 to about 99%, from about 0.2 to about 99%, from about 0.5 to about 99%, from about 1 to about 99%, from about 5 to about 99%, from about 10 to about 99%, from about 15 to about 99%, from about 20 to about 99%, from about 25 to about 99%, from about 30 to about 99%, from about 35 to about 99%, from about 40 to about 99%, and from about 45 to about 99%).

In one embodiment the composition comprises at least about 0.001, 0.01, 0.05, 0.1, 0.15,0.2,0.3,0.4,0.5,1,2,3,4, 5,6,7, 8,9,10,11,12,13,14,15,16,17,18 or 19 grams of fresh, recombined or powdered whole milk for milk derivative, preferably milk fat, of Formula (I) or (II) and useful ranges may be selected between any of these foregoing values (for example, from about 0.01 to about 1 grams, about 0.01 to about 10 grams, about 0.01 to about 19 grams, from about 0.1 to about 1 grams, about 0.1 to about 10 grams, about 0.1 to about 19 grams, from about 1 to about 5 grams, about 1 to about 10 grams, about 1 to about 19 grams, about 5 to about 10 grams, and about 5 to about 19 grams).

In one embodiment the composition comprises, consists essentially of or consists of about 0.1, 0.2, 0.5, 1, 5, 10, 15, 20, 25, 30, 35, 40, 45, 50, 55, 60, 65, 70, 75, 80, 85, 90, 95, 99, 99.5, 99.8 or 99.9% by weight of a lactoferrin polypeptide, a functional lactoferrin variant, a functional lactoferrin fragment, metal ion lactoferrin, a metal ion lactoferrin functional variant, or a metal ion lactoferrin functional fragment, or a mixture of any two or more thereof, and useful ranges may be selected between any of these foregoing values (for example, from about 0.1 to about 50%, from about 0.2 to about 50%, from about 0.5 to about 50%, from about 1 to about 50%, from about 5 to about 50%, from about 10 to about 50%, from about 15 to about 50%, from about 20 to about 50%, from about 25 to about 50%, from about 30 to about 50%, from about 35 to about 50%, from about 40 to about 50%, from about 45 to about 50%, from about 0.1 to about 60%, from about 0.2 to about 60%, from about 0.5 to about 60%, from about 1 to about 60%, from about 5 to about 60%, from about 10 to about 60%, from about 15 to about 60%, from about 20 to about 60%, from about 25 to about 60%, from about 30 to about 60%, from about 35 to about 60%, from about 40 to about 60%, from about 45 to about 60%, from about 0.1 to about 70%, from about 0.2 to about 70%, from about 0.5 to about 70%, from about 1 to about 70%, from about 5 to about 70%, from about 10 to about 70%, from about 15 to about 70%, from about 20 to about 70%, from about 25 to about 70%, from about 30 to about 70%, from about 35 to about 70%, from about 40 to about 70%, from about 45 to about 70%, from about 0.1 to about 80%, from about 0.2 to about 80%, from about 0.5 to about 80%, from about 1 to about 80%, from about 5 to about 80%, from about 10 to about 80%, from about 15 to about 80%, from about 20 to about 80%, from about 25 to about.80%, from about 30 to about 80%, from about 35 to about 80%, from about 40 to about 80%, from about 45 to about 80%, from about 0.1 to about 90%, from about 0.2 to about 90%, from about 0.5 to about 90%, from about 1 to about 90%, from about 5 to about 90%, from about 10 to about 90%, from about 15 to about 90%, from about 20 to about 90%, from about 25 to about 90%, from about 30 to about 90%, from about 35 to about 90%, from about 40 to about 90%, from about 45 to about 90%, from about 0.1 to about 99%, from about 0.2 to about, 99%, from about 0.5 to about 99%, from about 1 to about 99%, from about 5 to about 99%, from about 10 to about 99%, from about 15 to about 99%, from about 20 to about 99%, from about 25 to about 99%, from about 30 to about 99%, from about 35 to about 99%, from about 40 to about 99%, and from about 45 to about 99%).

### 3. Compositions useful according to invention

A composition useful herein may be formulated as a food, drink, food additive, drink additive, dietary supplement, nutritional product, medical food, nutraceutical, medicament or pharmaceutical. Appropriate formulations may be prepared by an art skilled worker with regard to that skill and the teaching of this specification.

In one embodiment the present invention relates to use of milk fat or a milk fat analogue, optionally with at least one anti-tumour agent, preferably lactoferrin, in the manufacture of a food, drink, food additive, drink additive, dietary supplement, nutritional product, medical food, nutraceutical, medicament or pharmaceutical. Preferably the composition is formulated for oral or topical administration. Preferably the composition is formulated for oral or parenteral administration. Preferably the composition is for inhibiting tumour growth, inhibiting tumour metastasis, inducing apoptosis, inducing apoptosis of tumour cells, treating or preventing cancer, increasing the responsiveness of a subject or the sensitivity of a tumour to a therapy, maintaining or improving one or more of the white blood cell count, the red blood cell count, or the myeloid cell count of a subject, increasing the production of Th1 and Th2 cytokines within the intestine or a tumour of a subject, treating or preventing anemia, cachexia, mucositis, in a subject in need thereof, or other uses, as described above. Preferably the milk fat or milk fat analogue and the at least one additional therapeutic agent, such as one or more anti-tumour agents, such as lactoferrin, is as described herein. Preferably the anti-tumour factor is one described herein.

In one embodiment the composition is' in the form of a tablet, a caplet, a pill, a hard or soft capsule or a lozenge.

In one embodiment the composition is in the form of a cachet, a dispensable powder, granules, a suspension, an elixir, a liquid, a drink, or any other form that can be added to food or drink, including for example water or fruit juice. In one embodiment the composition is an enteral product, a solid enteral product or a liquid enteral product.

In one embodiment the composition further comprises one or more constituents (such as antioxidants) which prevent or reduce degradation of the composition during storage or after administration.

In one embodiment, compositions useful herein include any edible consumer product which is able to carry fats, fatty acids or lipid. When the composition comprises as the at least one additional therapeutic agent a proteinaceous factor, such as lactoferrin, the edible consumer product is one able to carry protein. Examples of suitable edible consumer products include baked goods, powders, liquids, confectionary products, reconstituted fruit products, snack bars, food bards muesli bars, spreads, sauces, dips, dairy products including ice creams, yoghurts and cheeses, drinks including dairy and non-dairy based drinks. (such as milk drinks including milk shakes, and yogurt drinks), milk powders, sports supplements including dairy and non-dairy based sports supplements, food additives such as protein sprinkles and dietary supplement products including daily supplement tablets. Within this embodiment, a composition useful herein may also be an infant formula, in powder or liquid form. Suitable nutraceutical compositions useful herein may be provided in similar forms.

Compositions useful herein may further include other factors such as calcium, zinc, magnesium, selenium, vitamin C, vitamin D, vitamin E, vitamin K2, complex carbohydrates, edible or cooking oils including palm, olive, soybean, canola, corn, sunflower, safflower, peanut, grape seed, sesame, nut, almond, cashew, hazelnut, macadamia, pecan, pistachio, and walnut, and other edibles include acai, amaranth, apricot, argan, artichoke, avocado, babassu, ben, blackcurrant seed, borage seed, borneo tallow nut, bottle gourd, buffalo gourd, carob pod (algaroba), cohune, coriander seed, evening primrose, false flax, hemp, kapok seed, lallemantia, meadowfoam seed, mustard, okra seed (hibiscus seed), perilla seed, pequi, pine nut, poppyseed, prune kernel, pumpkin seed, quinoa, ramtil, rice bran, tea (camellia), thistle, watermelon seed, or wheat germ oil, or a combination thereof.

The compositions useful herein may be formulated to allow for administration to a subject by any chosen route, including but not limited to oral or parenteral (including topical, subcutaneous, intramuscular and intravenous) administration.

In general, for oral administration a dietary (a food, food additive or food supplement for example), nutraceutical or pharmaceutical composition useful herein may be formulated by a skilled worker according to known formulation techniques.

Thus, a pharmaceutical composition useful according to the invention may be formulated with an appropriate pharmaceutically acceptable carrier (including excipients, diluents, auxiliaries, and combinations thereof) selected with regard to the intended route of administration and standard pharmaceutical practice. See for example, Remington's Pharmaceutical Sciences, 16th edition, Osol, A. Ed., Mack Publishing Co., 1980.

While the preferred route of administration is oral, it should be understood that any mode of administration may be suitable for any composition of the invention, including administration by multiple routes, including different routes for different agents. Therefore, inhalation (nasal or buccal inhalation) and vaginal and rectal administration of any composition of the invention is also contemplated. Intramedullar, epidural, intra-articular, and intra-pleural administration of any composition of the invention is also contemplated. Administration of milk fat or a milk fat analogue, optionally with at least one additional factor, by a first administration route accompanied by separate, simultaneous or sequential administration of the other agent by a second administration route is also contemplated; for example, oral administration of milk fat accompanied by topical administration of the at least one additional therapeutic agent.

A dosage form useful herein may be administered orally as a powder, liquid, tablet or capsule. Suitable dosage forms may contain additional agents as required, including emulsifying, antioxidant, flavouring or colouring agents, or have an enteric coating. Suitable enteric coatings are known. Enteric coatings surrounding the active ingredients and prevent the release of the active ingredients in the stomach but allow release after the dosage form has left the stomach. Dosage forms useful herein may be adapted for immediate, delayed, modified, sustained, pulsed or controlled release of the active components. Suitable formulations may contain additional agents as required, including emulsifying, antioxidant, flavouring or colouring agents.

Capsules can contain any standard pharmaceutically acceptable materials such as gelatin or cellulose. Tablets can be formulated in accordance with conventional procedures by compressing mixtures of the active ingredients with a solid carrier and a lubricant. Examples of solid carriers include starch and sugar bentonite. Active ingredients can also be administered in a form of a hard shell tablet or a capsule containing a binder, e.g., lactose or mannitol, a conventional filler, and a tabletting agent. Pharmaceutical compositions can also be administered via the parenteral route. Examples of parenteral dosage forms include aqueous solutions, isotonic saline or 5% glucose of the active agent, or other well-known pharmaceutically acceptable excipient. Cyclodextrins, or other solubilising agents well-known to those familiar with the art, can be utilized as pharmaceutical excipients for delivery of the therapeutic agent.

Injectable dosage forms may be formulated as liquid solutions or suspensions. Solid forms suitable for solution in, or suspension in, liquid prior to injection may also be prepared. The dosage form may also be emulsified. Milk fat or a milk fat analogue, and when present the at least one additional factor may be mixed with carriers such as, for example, water, saline, dextrose, glycerol, ethanol, or the like and combinations thereof.

Sustained-release preparations may be prepared incorporating milk fat, optionally with at least one additional factor. Suitable examples of sustained-release preparations include semi-permeable matrices of solid hydrophobic polymers containing milk fat, and when present the at least one additional therapeutic agent, such as lactoferrin or a functional variant or functional fragment thereof. The matrices may be in the form of shaped articles, e.g., films, or microcapsules. Examples of sustained-release matrices include polyesters, hydrogels (for example, poly(2-hydroxyethyl- methacrylate), or poly(vinylalcohol)), polylactides (see US 3,773,919), copolymers of L-glutamic acid and ethyl-L-glutamate, non-degradable ethylene-vinyl acetate, and degradable lactic acid-glycolic acid copolymers such as the LUPRON DEPOT™ (injectable microspheres composed of lactic acid-glycolic acid copolymer and leuprolide acetate).

Topical formulations comprising milk fat or a milk fat analogue, and when present the at least one additional therapeutic agent, may be prepared as lotions, creams, ointments, pastes or salves using known carriers for such applications.

The present invention also relates to a parenteral unit dosage form comprising milk fat, optionally with at least one additional therapeutic agent, and at least one anti-tumour agent. Preferably the at least one anti-tumour agent is selected from paclitaxel, doxorubicin, epirubicin, fluorouracil, cyclophosphamide, methotrexate, an expression plasmid encoding the T cell co-stimulator B7-1 and dendritic cell therapy. Alternatively the agent is selected from any of those described herein. Preferably the milk fat or milk fat analogue is as described above.

The efficacy of a composition useful according to the invention can be evaluated both *in vitro* and *in vivo.* See, e.g., the examples below. Briefly, in one embodiment the composition can be tested for its ability, to for example, inhibit tumour formation or tumour growth *in vitro*. For *in vivo* studies, the composition can be fed to or injected into an animal (e.g., a mouse) and its effects on tumour size or morphology are then assessed. Based on the results, an appropriate dosage range and administration route can be determined.

The compositions useful herein may be used alone or in combination with one or more other therapeutic agents. The therapeutic agent may be a food, drink, food additive, drink additive, food component, drink component, dietary supplement, nutritional product, medical food, nutraceutical, medicament or pharmaceutical. The therapeutic agent is preferably effective to attenuate one or more of the symptoms of a condition associated with cancer, or associated with or associated with a condition causing anemia (including macrocytic and microcytic anemia), hematologic suppression, mucositis, or cachexia. Preferred therapeutic agents include anti-tumour food factors, immunotherapeutic agents, hematopoietic agents, anticachectic agents, and antimucositic agents, and lactoferrin is a particularly preferred therapeutic agent.

In one embodiment the milk fat or a milk fat analogue, optionally with at least one additional therapeutic agent are formulated for administration separately, simultaneously or sequentially with at least one anti-tumour agent or anti-tumour therapy described herein.

in one embodiment the milk fat or a milk fat analogue, optionally with at least one additional therapeutic agent are formulated for coadministration with the at least one anti-tumour agent or anti-tumour therapy described herein.

In one embodiment the milk fat or a milk fat analogue, optionally with at least one additional therapeutic agent are formulated for sequential administration with the at least one anti-tumour agent or anti-tumour therapy described herein.

In one embodiment the milk fat or a milk fat analogue is included as or is delivered as an adjuvant for the anti-tumour agent or anti-tumour therapy in that the milk fat or milk fat analogue enhances or potentiates the effects of the anti-tumour agent or anti-tumour therapy. At least one additional therapeutic agent may be delivered separately.

When used in combination with another therapeutic agent, the administration of a composition useful herein and the other therapeutic agent may be simultaneous or sequential. Simultaneous administration includes the administration of a single dosage form that comprises all components or the administration of separate dosage forms at substantially the same time. Sequential administration includes administration according to different schedules, preferably so that there is an overlap in the periods during which the composition useful herein and other therapeutic agent are provided.

Suitable agents with which the compositions of the invention can be co-administered include chemotherapeutic agents, immunotherapeutic agents, anticachectic agents, antimucositic agents, hematopoietic agents, and other suitable agents known in the art. Such agents are preferably administered parenterally, preferably by intravenous, subcutaneous, intramuscular, intraperitoneal, intramedullar, epidural, intradermal, transdermal (topical), transmucosal, intra-articular, and intrapleural, as well as oral, inhalation, vaginal and rectal administration.

Additionally, it is contemplated that a composition in accordance with the invention may be formulated with additional active ingredients which may be of benefit to a subject in particular instances. For example, therapeutic agents that target the same or different facets of the disease process may be used.

Suitable agents with which the compositions useful herein can be co-administered include alpha v beta 3 integrin receptor antagonists, antiestrogens or SERMs (Selective Estrogen Receptor Modulators) (including but not limited to tamoxifen, raloxifene, lasofoxifene, toremifene, azorxifene, clomiphene, droloxifene, idoxifene, levormeloxifene, zuclomiphene, enclomiphene, nafoxidene, and salts thereof), antiresorptive agents, bisphosphonates (including but not limited to alendronate, clodronate, etidronate, ibandronate, incadronate, minodronate, neridronate, olpadronate, pamidronate, piridronate, risedronate, tiludronate, zoledronate, and pharmaceutically acceptable salts thereof), calcium receptor antagonists; calcium supplements, cathepsin K inhibitors, Dual Action Bond Agents (DABAs) (including but not limited to strontium ranelate), estrogen and estrogen derivatives (including but not limited to 17 beta-estradiol, estrone, conjugated estrogen, equine estrogen, and 17 beta-ethynyl estradiol), flavonoids, folic acid, osteoanabolic agents, osteoprotegerin, progestin and progestin derivatives (including but not limited to norethindrone and medroxyprogesterone acetate), vacuolar ATPase inhibitors, antagonists of VEGF, thiazolidinediones, calcitonin, protein kinase inhibitors, parathyroid hormone (PTH), PTH analogs, recombinant parathyroid hormone, growth hormone secretagogues, growth hormone releasing hormone, insulin-like growth factor, bone morphogenetic protein (BMP), inhibitors of BMP antagonism, prostaglandin derivatives, fibroblast growth factors, vitamin B6, vitamin D, vitamin D derivatives (including but not limited to 1,25-dihydroxycholecalciferol), vitamin K, vitamin K derivatives, soy isoflavones, calcium salts, fluoride salts, statins (including but not limited to lovastatin, simvastatin, pravastatin, fluvastatin, atorvastatin, cerivastatin, rosuvastatin, and pitavastatin), and combinations thereof, and other suitable agents known in the art.

In one embodiment, a composition useful herein includes or is administered simultaneously or sequentially with other milk components such as whey protein, whey protein fractions (including acidic or basic whey protein fractions or a combination thereof), glycomacropeptide, lactoferrin, vitamin D or calcium, or combinations thereof. Useful milk component-containing compositions include compositions such as a food, drink, food additive, drink additive, dietary supplement, nutritional product, medical food or nutraceutical. Milk fractions enriched for these components may also be employed.

It should be understood that the additional therapeutic agents listed above (both food based and pharmaceutical agents) may also be employed in a method according to the invention where they are administered separately, simultaneously or sequentially with a composition useful herein.

As will be appreciated, the dose of the composition administered, the period of administration, and the general administration regime may differ between subjects depending on such variables as the severity of symptoms of a subject, the type of disorder to be treated, the mode of administration chosen, and the age, sex and/or general health of a subject. However, by way of general example, the inventors contemplate administration of from about 1 mg to about 1000 mg per kg body weight of a composition useful herein is administered per day, preferably about 50 to about 500 mg per kg per day. In one embodiment, the inventors contemplate administration of from about 0.05 mg to about 250 mg per kg body weight of a pharmaceutical composition useful herein.

It should be appreciated that administration may include a single daily dose or administration of a number of discrete divided doses as may be appropriate. It should be understood that a person of ordinary skill in the art will be able without undue experimentation, having regard to that skill and this disclosure, to determine an effective dosage regime (including daily dose and timing of administration) for a given condition.

The present invention also relates to a dietary, nutraceutical or oral pharmaceutical composition comprising, consisting essentially of or consisting of milk fat or a milk fat analogue in combination with lactoferrin, casein, or other protective protein. Preferably the composition consists essentially of about 0.1 to 99 wt % milk fat or a milk fat analogue and about 0.1 to 99 wt % lactoferrin, casein, or other protective protein. More preferably the composition consists essentially of about 0.5 to 10 wt % milk fat and about 10 to 99 wt % lactoferrin, casein, or other protective protein. Most preferably the composition consists essentially of about 1 wt % milk fat and about 20 wt % lactoferrin, casein, or other protective protein. Preferably the milk fat or milk fat analogue is as described above.

In one embodiment a composition of the invention is a milk fraction, preferably a milk fat fraction. In one embodiment the milk fraction comprises at least about 1, 5,10, 15, 20, 25, 30, 35, 40, 45, 50, 55, 60, 65, 70, 75, 80, 85, 90, 95 or 99% by weight milk fat, and useful ranges may be selected from any of these values (for example, from about 1 to about 99% by weight, from about 5 to about 99% by weight, from about 10 to about 99% by weight, from about 15 to about 99% by weight, from about 20 to about 99% by weight, from about 25 to about 99% by weight, from about 30 to about 99% by weight, from about 35 to about 99% by weight, from about 40 to about 99% by weight, from about 45 to about 99% by weight, from about 50 to about 99% by weight, from about 55 to about 99% by weight, from about 60 to about 99% by weight, from about 65 to about 99% by weight, from about 70 to about 99% by weight, from about 75 to about 99% by weight, from about 80 to about 99% by weight, from about 85 to about 99% by weight, from about 90 to about 99% by weight, or from about 95 to about 99% by weight). Preferably, when the composition comprises at least one additional therapeutic agent, the composition may additionally comprise a milk protein fraction.

In one embodiment the milk fat is administered as a milk fat fraction. Preferred milk fat fractions include cream, butter, anhydrous milk fat (AMF) (typically produced by phase inversion of cream or butter), butter milk, butter serum, hard milk fat fractions, soft milk fat fractions, sphingolipid fractions, milk fat globular membrane fractions, phospholipid fractions, and complex lipid fractions, and combinations thereof, and hydrolysates thereof, and fractions of the hydrolysates, and combinations of hydrolysed and/or non-hydrolysed fractions.

Preferred phospholipid fractions can be prepared by separation from anhydrous milk fat (such as that available from Fonterra Co-operative Group Limited, New Zealand) by thin-layer chromatography, and/or liquid chromatography. Desired phospholipid fractions may be prepared using such separation techniques. For example, fractions comprising all three sphingomyelin peaks, comprising phosphatidylcholine and phosphatidylinositol in similar amounts, a small amount of phosphatidylserine, and the first of the three sphingomyelin peak, or comprising a large amount of phosphatidylethanolamine and ceramides and lysophospholipid, are routinely prepared by liquid chromatography. Hydrolysed forms of these fractions can be prepared, for example by adding 1% KOH (in ethanol), and stirring and heating for 10 minutes at pH 9.5-10.0. Generally, hydrolysed samples are preferably neutralised before further use, for example with acetic acid or hydrochloric acid to pH 7.0 and flushed dry in heated mantle under nitrogen.

Commercial phospholipid fractions are also suitable for use in the present invention. The Phospholipid Concentrate PC600™ phospholipid fraction and the Ganglioside G600™ fraction (both available from Fonterra Co-operative Group Limited, New Zealand) are preferred, whether in non-hydrolysed form or in hydrolysed form. Again, these fractions can be hydrolysed as above.

In one embodiment the composition comprises, consists essentially of or consists of about 0.1,0.5,1, 5,10,15,20,25,30,35,40,45 or 50% by weight of fresh, recombined or powdered whole milk or a milk derivative, preferably milk fat, and useful ranges may be selected between any of these foregoing values (for example, from about 0.1 to about 50%, from about 0.2 to about 50%, from about 0.5 to about 50%, from about 1 to about 50%, from about 5 to about 50%, from about 10 to about 50%, from about 15 to about 50%, from about 20 to about 50%, from about 25 to about 50%, from about 30 to about 50%, from about 35 to about 50%, from about 40 to about 50%, and from about 45 to about 50%). The milk derivative is preferably selected from recombined, powdered or fresh milk, reconstituted whole milk powder, milk concentrate, milk retentate, concentrated milk, ultrafiltered milk retentate, milk protein concentrate (MPC), milk protein isolate (MPI), calcium depleted milk protein concentrate (MPC), casein, caseinate, milk fat, cream, butter, anhydrous milk fat (AMF), butter milk, butter serum, hard milk fat fractions, soft milk fat fractions, sphingolipid fractions, milk fat globular membrane fractions, phospholipid fractions, complex lipid fractions, colostrum, a colostrum fraction, colostrum protein concentrate (CPC), colostrum whey, an immunoglobulin fraction from colostrum, whey, whey protein isolate (WPI), whey protein concentrate (WPC), sweet whey, lactic acid whey, mineral acid whey, reconstituted whey powder, a composition derived from any milk or colostrum processing stream, a composition derived from the retentate or permeate obtained by ultrafiltration or microfiltration of any milk or colostrum processing stream, or a composition derived from the breakthrough or adsorbed fraction obtained by chromatographic (including but not limited to ion and gel permeation chromatography) separation of any milk or colostrum processing stream, and combinations thereof, and hydrolysates thereof, and fractions of the hydrolysates, and combinations of hydrolysed and/or non-hydrolysed fractions.

In one embodiment a method of the invention comprises administration of a mixture of milk fat or a milk fat analogue and lactoferrin or at least one functional variant or functional fragment of lactoferrin as described below. Therefore in one embodiment a composition comprises a mixture of milk fat or a milk fat analogue and lactoferrin or at least one functional variant or functional fragment of lactoferrin. In alternative embodiment a composition comprises a mixture of milk fat or a milk fat analogue and at least one functional fragment of lactoferrin.

Compositions of the invention comprising lactoferrin or at least one functional variant or functional fragment thereof may also be administered by parenteral routes including but not limited to subcutaneous, intravenous, intraperitoneal, intramuscular and intratumoural administration. Preferably, lactoferrin is administered parenterally by injection. The milk fat or milk fat analogue may be administered by a separate route, and preferably is administered orally. Those skilled in the art will be able to prepare suitable formulations for parenteral administration without undue experimentation.

In one embodiment the daily dosage range (by any route) is about 0.001 to 250 g of milk fat per day, preferably 0.001 to 100 g, 0.1 to 30 g, 0.1 to 40 g, 0.1 to 50 g, 0.1 to 60 g, 0.1 to 70 g, 0.1 to 80 g, 0.1 to 100 g, 0.1 to 110 g, 0.1 to 120 g, 0.1 to 130 g, 0.1 to 140 g, 0.1 to 150 g, 0.1 to 160 g, 0.1 to 170 g, 0.1 to 180 g, 0.1 to 190 g, 0.1 to 200 g, 0.1 to 210 g, 0.1 to 220 g, 0.1 to 230 g, 0.1 to 240 g, or 0.1 to 250 g per day for a 70 kg adult, preferably 10 mg to 1.5 g/kg/day, preferably 50 mg to 500 mg/kg/day. A higher dose is preferred for short-term treatment and prevention and a lower dose for long-term treatment and prevention.

### 4. Lactoferrin polypeptides

Bovine lactoferrin (bLf) is a single-chain iron-binding glycoprotein of 78 kDa which is present in bovine milk. It is a natural defence protein present in most secretions commonly exposed to normal flora including.milk, colostrum, tears, nasal secretions, saliva, bile, pancreatic juice, intestinal mucus, and genital secretions. It is secreted by neutrophils and present at high levels at sites of bacterial infection. It is a multifunctional protein that may regulate iron absorption in the intestine, promote intestinal cell growth, protect against microbial infection, regulate myelopoiesis, regulate systemic immune responses, and can prevent the development of cancer (reviewed in Ward, et al., 2002; Brock, J H, 2002; Weinburg, E D, 2001; Conneely, O M, 2001; Tomita, et al., 2002 and Tsuda, et al., 2002).

In addition to the useful lactoferrin polypeptides and fragments listed above, examples of lactoferrin amino acid and mRNA sequences that have been reported and are useful in methods of the invention include but are not limited to the amino acid (Accession Number NP_002334) and mRNA (Accession Number NM_002343) sequences of human lactoferrin; the amino acid (Accession Numbers NP_851341 and CAA38572) and mRNA (Accession Numbers X54801 and NM_180998) sequences of bovine lactoferrin; the amino acid (Accession Numbers JC2323, CAA55517 and AAA97958) and mRNA (Accession Number U53857) sequences of goat lactoferrin; the amino acid (Accession Number CAA09407) and mRNA (Accession Number AJ010930) sequences of horse lactoferrin; the amino acid (Accession Numbers NP_999527, AAL40161 and AAP70487) and mRNA (Accession Number NM_214362) sequences of pig lactoferrin; the amino acid (Accession Number NP_032548) and mRNA (Accession Number NM_008522) sequences of mouse lactoferrin; the amino acid (Accession Number CAA06441) and mRNA (Accession Number AJ005203) sequences of water buffalo lactoferrin; and the amino acid (Accession Number CAB53387) and mRNA (Accession Number AJ131674) sequences of camel lactoferrin. These sequences may be used according to the invention in wild type or variant form. Polypeptides encoded by these sequences may be isolated from a natural source, produced as recombinant proteins or produced by organic synthesis, using known techniques.

Methods for generating useful polypeptides and variants are known in the art and discussed below. Useful recombinant lactoferrin polypeptides and fragments and methods of producing them are reported in US patent specifications US 5,571,691, US 5,571,697, US 5,571,896, US 5,766,939, US 5,849,881, US 5,849,885, US 5,861,491, US 5,919,913, US 5,955,316, US 6,066,469, US 6,080,599, US 6,100,054, US 6,111,081, US 6,228,614, US 6,277,817, US 6,333,311, US 6,455,687, US 6,569,831, US 6,635,447, US 2005-0064546 and US 2005-0114911.

Useful variants also include bovine lactoferrin variants bLf-a and bLf-b (Tsuji, et al. (1989); Yoshida, et al. (1991)). Further useful variants include glycosylated and aglycosyl forms of lactoferrin (Pierce, et al. (1991); Metz-Boutigue, et al. (1984); van Veen, et al. (2004)) and glycosylation mutants (having variant points of glycosylation or variant glycosyl side chains).

Useful fragments include the N-lobe and C-lobe fragments (Baker, et al., 2002) and any other lactoferrin polypeptides that retain a lactoferrin binding pocket, such as truncated lactoferrin polypeptides. Other lactoferrin fragments are described in published international patent application WO2007/043900 that is incorporated herein by reference.

Useful truncated lactoferrin polypeptides include polypeptides truncated by about 1 to about 300 amino acids, preferably about 1, 5, 10, 15, 20, 25, 30, 35, 40, 45, 50, 55, 60, 65, 70,75,80, 85,90,95,100,105,110,115,120,125,130,135,140,145,150,155,160,165, 170, 175, 180, 185, 190, 195, 200, 205, 210, 215, 220, 225, 230, 235, 240, 245, 250, 255, 260, 265, 270, 275, 280, 285, 290, 295 or 300 amino acids or more, and including polypeptides truncated at the N-terminus, at the C-terminus or at both the N- terminus and C-terminus, provided that the truncated polypeptide retains at least one of the N-lobe or the C-lobe metal ion-binding pockets. It is reported that residues Asp 60, Tyr 92, Tyr 192, and His 253 of bovine lactoferrin (without the signal sequence) are the amino acid metal ion ligands in the N-lobe. It is reported that residues Asp 395, Tyr 433, Tyr 526, and His 595 of bovine lactoferrin (without the signal sequence) are the amino acid metal ion ligands in the C-lobe. (Karthikeyan, et al., 1999).

Candidate variants or fragments of lactoferrin for use according to the present invention may be generated by techniques including but not limited to techniques for mutating wild type proteins (see Sambrook, et al. (1989) and elsewhere for a discussion of such techniques) such as but not limited to site-directed mutagenesis of wild type lactoferrin and expression of the resulting polynucleotides; techniques for generating expressible polynucleotide fragments such as PCR using a pool of random or selected primers; techniques for full or partial proteolysis or hydrolysis of wild type or variant lactoferrin polypeptides; and techniques for chemical synthesis of polypeptides. Variants or fragments of lactoferrin may be prepared by expression as recombinant molecules from lactoferrin DNA or RNA, or variants or fragments thereof. Nucleic acid sequences encoding variants or fragments of lactoferrin may be inserted into a suitable vector for expression in a cell, including eukaryotic cells such as but not limited to Aspergillus or bacterial cells such as but not limited to E. coli. Lactoferrin variants or fragments may be prepared using know PCR techniques including but not limited to error-prone PCR and DNA shuffling. Error-prone PCR is a process for performing PCR under conditions where the copying fidelity of the DNA polymerase is low, such that a high rate of point mutations is obtained along the entire length of the PCR product (Leung, et al. (1989); Cadwell, et al. (1992)). DNA shuffling refers to forced homologous recombination between DNA molecules of different but highly related DNA sequence in vitro, caused by random fragmentation of the DNA molecule based on sequence homology, followed by fixation of the crossover by primer extension in a PCR reaction (Stemmer (1994)). Suitable lactoferrin nucleic acid sequences for use in such methods include those listed above or may be generated by known methods including, for example, reverse transcription-PCR (RT-PCR) of tissue RNA isolates. Suitable primers for RT-PCR may be designed with reference to the mRNA sequences listed above. Commercial kits are available for RT-PCR (for example, Cells-to-cDNA™ kits from Amnion, USA).

Variants or fragments of lactoferrin may also be generated by known synthetic methods (see Kimmerlin, et al., 2005, for example).

Metal ion-binding variants or fragments of lactoferrin may be obtained by known techniques for isolating metal-binding polypeptides including but not limited to metal affinity chromatography, for example. Candidate variants or fragments of lactoferrin may be contacted with free or immobilised metal ions, such as Fe³⁺ and purified in a suitable fashion. For example, candidate variants or fragments may be contacted at neutral pH with a metal ion immobilised by chelation to a chromatography matrix comprising iminodiacetic acid or tris(carboxymethyl)ethylenediamine ligands. Bound variants or fragments may be eluted from the supporting matrix and collected by reducing the pH and ionic strength of the buffer employed. Metal-bound variants or fragments may be prepared according to the methods described above and below and described in the Examples below.

Functional variants, fragments and hydrolysates of lactoferrin may be obtained by selecting variants, fragments and hydrolysates of lactoferrin and assessing their efficacy in methods of the present invention by employing the methodologies set out in the Examples described below.

In one embodiment the lactoferrin is any mammalian lactoferrin including but not limited to sheep, goat, pig, mouse, water buffalo, camel, yak, horse, donkey, llama, bovine or human lactoferrin. Preferably the lactoferrin is bovine lactoferrin.

In another embodiment the lactoferrin is any recombinant mammalian lactoferrin including but not limited to recombinant sheep, goat, pig, mouse, water buffalo, camel, yak, horse, donkey, llama, bovine or human lactoferrin. Preferably the lactoferrin is recombinant bovine lactoferrin. Recombinant lactoferrin may be produced by expression in cell free expression systems or in transgenic animals, plants, fungi or bacteria, or other useful species.

In yet another embodiment the lactoferrin is isolated from milk, preferably sheep, goat, pig, mouse, water buffalo, camel, yak, horse, donkey, llama, bovine or human milk. Preferably the lactoferrin is isolated from milk by cation exchange chromatography followed by ultrafiltration and diafiltration.

### 5. Isolation of lactoferrin from milk

The following is an exemplary procedure for isolating lactoferrin from bovine milk. Fresh skim milk (7 L, pH 6.5) is passed through a 300 ml column of S Sepharose Fast Flow equilibrated in milli Q water, at a flow rate of 5 ml/min and at 4°C. Unbound protein is washed through with 2.5 bed volumes of water and bound protein eluted stepwise with approximately 2.5 bed volumes each of 0.1 M, 0.35 M, and 1.0 M sodium chloride. Lactoferrin eluting as a discreet pink band in 1 M sodium chloride is collected as a single fraction and dialysed against milli Q water followed by freeze-drying. The freeze-dried powder is dissolved in 25 mM sodium phosphate buffer, pH 6.5 and subjected to rechromatography on S Sepharose Fast Flow with a sodium chloride gradient to 1 M in the above buffer and at a flow rate of 3 ml/min. Fractions containing lactoferrin of sufficient purity as determined by gel electrophoresis and reversed phase HPLC are combined, dialyzed and freeze-dried. Final purification of lactoferrin is accomplished by gel filtration on Sephacryl 300 in 80 mM dipotassium phosphate, pH 8.6, containing 0.15 M potassium chloride. Selected fractions are combined, dialyzed against milli Q water, and freeze-dried. The purity of this preparation is greater than 95% as indicated by HPLC analysis and by the spectral ratio values (280 nm/465 nm) of∼19 or less for the iron-saturated form of lactoferrin.

Other exemplary methods for isolating useful milk fractions comprising lactoferrin are presented in US patent No. 5,932,259 to Kato et al., and US patent No. 5,976,597 to Takada et al.

### 6. Metal ion saturation or depletion of lactoferrin

Iron saturation is achieved by addition of a 2:1 molar excess of 5mM ferric nitrilotriacetate (Foley and Bates (1987)) to a 1% solution of the purified lactoferrin in 50 mM Tris, pH 7.8 containing 10 mM sodium bicarbonate. Excess ferric nitrilotriacetate is removed by dialysis against 100 volumes of milli Q water (twice renewed) for a total of 20 hours at 4° C. The iron-loaded (holo-) lactoferrin may then be freeze-dried. Varying degrees of iron saturation may be obtained by providing less of the metal ion donor, as described in the examples below. Another method of preparing metal ion lactoferrin is reported in published international patent application WO 2006/132553 that is hereby incorporated by reference. A method of maintaining or improving the keeping quality of a metal ion lactoferrin composition is reported in published international patent application WO 2006/096073 that is hereby incorporated by reference.

Iron-depleted (apo-) lactoferrin is prepared by dialysis of a 1% solution of the highly purified lactoferrin sample in water against 30 volumes of 0.1M citric acid, pH 2.3, containing 500 mg/L disodium EDTA, for 30 h at 4° C (Masson and Heremans (1966)). Citrate and EDTA are then removed by dialysis against 30 volumes of milli Q water (once renewed) and the resulting colourless solution may be freeze-dried.

A lactoferrin polypeptide can contain an iron ion (as in a naturally occurring lactoferrin polypeptide) or a non-iron metal ion (e.g., a copper ion, a chromium ion, a cobalt ion, a manganese ion, or a zinc ion). For instance, lactoferrin isolated from bovine milk can be depleted of iron and then loaded with another type of metal ion. For example, copper loading can be achieved according to the same method for iron loading described above. For loading lactoferrin with other metal ions, the method of Ainscough, et al. (1979) can be used.

In one embodiment the metal ion is an ion selected from the group comprising aluminium, bismuth, copper, chromium, cobalt, gold, iron, manganese, osmium, platinum, ruthenium, zinc, or other ions that will coordinate specifically in a lactoferrin metal ion binding pocket. Preferably the metal ion is an iron ion.

In a preparation of a composition for use according to the invention, a lactoferrin polypeptide or metal ion-binding lactoferrin fragment can be of a single species, or of different species. For instance, the polypeptides or fragments can each contain a different number of metal ions or a different species of metal ions; or the lengths of the polypeptides can vary, e.g., some are full-length polypeptides and some are fragments, and the fragments can each represent a particular portion of a full-length polypeptide. Such a preparation can be obtained from a natural source or by mixing different lactoferrin polypeptide species. For example, a mixture of lactoferrin polypeptides of different lengths can be prepared by proteinase digestion (complete or partial) of full-length lactoferrin polypeptides. The degree of digestion can be controlled according to methods well known in the art, e.g., by manipulating the amount of proteinase or the time of incubation, and described below. A full digestion produces a mixture of various fragments of full-length lactoferrin polypeptides; a partial digestion produces a mixture of full-length lactoferrin polypeptides and various fragments.

### 7. Preparation of lactoferrin fragments or lactoferrin hydrolysates

Useful lactoferrin fragments are described in published international patent applications WO 2006/054908 and WO 2007/043900, each incorporated herein in its entirety. Hydrolysates containing candidate functional fragments can be prepared by selecting suitable enzymes with known specificity of cleavage, such as trypsin or chymotrypsin, and controlling/limiting proteolysis by pH, temperature, time of incubation and enzyme to substrate ratio. Refinement of such isolated peptides can be made using specific endopeptidases. As an example, bovine lactoferricin can be produced by cleavage of bovine lactoferrin with pepsin at pH 2.0 for 45 min at 37°C (Facon & Skura, 1996), or at pH 2.5, 37°C for 4h using enzyme at 3% (w/w of substrate) (Tomita et al., 1994). The peptide can then be isolated by reversed phase HPLC (Tomita et al., 1994) or hydrophobic interaction chromatography (Tomita e al., 2002).

Alternatively, lactoferrin peptides can be produced by well established synthetic Fmoc chemistry as described for human kaliocin-1 (NH2-FFSASCVPGADKGQFPNLCRLCAGTGENKCA-COOH) and the lactoferricin derived peptide (NH2-TKCFQWQRNMRKVRGPPVSCIKR-COOH) in Viejo-Diaz et al., (2003); and bovine lactoferricin peptide (NH2-RRWQWRMKKLG-COOH) as described in Nguyen et al., (2005); and lactoferrampin (NH2-WKLLSKAQEKFGKNKSR-COOH) and shorter fragments as described in van der Kraan et al., (2004).

In general, SDS-PAGE may be used to estimate the degree of hydrolysis by comparison of the hydrolysate to a molecular weight standard. Size exclusion chromatography may be used to separate various species within a hydrolysate and to estimate a molecular weight distribution profile.

In a preferred hydrolytic method, bovine lactoferrin was dissolved to 20 mg/mL in 50 mM Tris pH 8.0,5 mM CaCl2. Trypsin (Sigma T8642, TPCK treated, Type XII from bovine pancreas, 11700U/mg protein) was added at an enzyme substrate ratio of 1:50 w/w and the mixture incubated at 25° C for 3h. The reaction was stopped by the addition of PMSF to 1mM final concentration and extent of digestion monitored by SDS-PAGE. The tryptic digest (4mL) was applied to gel filtration on Sephacryl S300 (Amersham GE) (90cm x 2.6cm column) in 50 mM Tris, 0.15M NaCl pH 8.0. Suitable fractions containing the major fragments of bovine lactoferrin (Legrand et al., 1984) were then subjected to cation exchange chromatography on S Sepharose fast Flow (Amersham GE) (15cm x 1.6 cm column) using sodium phosphate buffer pH 6.5 and a salt gradient to 1 M NaCl. Final separation of the C lobe and N+C lobes was achieved by further gel filtration on Sephacryl S300 as above but using 10% v/v acetic acid as eluent (Mata et al., 1994). The identity of the dialysed (versus milli-Q water) and freeze-dried fragments was confirmed by SDS-PAGE and Edman N-terminal sequencing.

In another method, a tryptic digest as above was separated by RP-HPLC on a Vydac C18 column as in Superti et al., (2001) and the high mass fragments corresponding to C-lobe and N-lobe fragments recovered. Identity was confirmed by MALDI MS.

In one embodiment hydrolysates useful herein contain one or more functional fragments.

### 8. Anti-tumour Food Factors

Anti-tumour food components are reviewed in Park, et al., 2002 and Kris-Etherton, 2002.

In one embodiment the anti-tumour food factor is selected from vitamin D (including vitamin D1 [lumisterol], vitamin D2 [calciferol or ergocalciferol], vitamin D3 [cholecalciferol], vitamin D4 [22-dihydroerogocalciferol] and vitamin D5 [sitocalciferol] and vitamin D5 [7-dehydrositosterol]), vitamin D analogues (including but not limited to those referenced below), soy protein, one or more soybean components (including those selected from the group comprising but not limited to omega-3 fatty acids from soy, isoflavones from soy (e.g. genistein and/or daidzein), and lunasin peptides (such as those described in US patents US 6,107,287 and US 6,544,956 that are incorporated herein by reference, and those having accession numbers AAE49016, AAE49017, AAP62458 and AAP62459)), polyphenols (from green or black tea for example), lycopene (or tomato juice for example), wheat bran, flavonoids (or apple juice for example), inositol, resveratrol (or grape juice for example), propolis, mushroom extract, anthocyanins (or berry juice for example), almonds, ginseng, casein hydrolysate, and combinations thereof.

Examples of vitamin D compounds useful herein include but are not limited to calcitriol (1-alpha,25-dihydroxy [1,25(OH)2D3]; 1,25-dihydroxycholecalciferol), 1,25-dihydroxyergocalciferol, calcifediol (25-hydroxycholecalciferol), 25-hydroxyergocalciferol, ergocalciferol (and its precursor ergosterol), cholecalciferol (and its precursor 7-dehydrocholesterol), doxercalciferol, dihydrotachysterol, paracalcitol, seocalcitol [EB 1089; 1 (S),3(R)-Dihydroxy-20(R)-(5'-ethyl-5'-hydroxyhepta-1'(E),3'(E)-dien-1'-yl)-9,10-secopregna-5(Z),7(E),10(19)-triene)] as well as derivatives, analogs, homologs, precursors and metabolites thereof.

In one embodiment the anti-tumour food factor is selected from the group comprising anti-tumour foods and anti-tumour food components.

In one embodiment the anti-tumour food may be a functional food or derivative thereof that has anti-cancerous properties including fruits, vegetables, legumes, nuts, seeds, grains, spices, herbs, fungi, probiotics, apples, apricots, beans (eg green bean, black bean), chick peas, berries (eg blueberries, raspberries), cruciferous vegetables (eg broccoli, brussel sprouts, cabbage, cauliflower, collards, kale, kohlrabi, bok choy, radish, mustards, and turnips), carrot, cheese, corn products, cranberries, egg plant, flaxseed, allium vegetables [eg garlic, onion, spring onion (scallions), chive, leek, shallot], ginger (including ginger components gingerol, paradol, and beta-elemene), ginseng, grapefruit, grapes, grape juice, green or black tea, horseradish, kiwifruit, kumara, leeks, lemons, limes, noni fruit, onions, oranges, peanuts, peppers, rye products, salmon, soy milk products, soy nuts, soybeans, squash, tangerines, tomatoes, wheat bran products, rice, papaya, pawpaw, peaches, persimmons, strawberries, taro leaves, green banana, mango, watercress, yams, almonds, and combinations thereof.

In one embodiment the anti-tumour food component may be selected from the group comprising soy protein, one or more soybean components (including those selected from the group comprising but not limited to omega-3 fatty acids from soy, isoflavones from soy (e.g. genistein and/or daidzein), and lunasin peptides (such as those described in US patents US 6,107,287 and US 6,544,956 that are incorporated herein by reference, and those having accession numbers AAE49016, AAE49017, AAP62458 and AAP62459), shark cartilage, garlic extracts, selenium supplementation, tea extracts (e.g. green or black tea polyphenols/catechins/epigallocatechin gallate), curcuminoids, caffeine, carnosic acid, capsaicin, sesquiterpene lactones (eg parthenolide, costumolide, yomogin), cotylenin A, humulone, arginine, glutamine, retinoids from green leaf vegetables, cocoa powder, lycopene, glucosinolates from cruciferous vegetables, organosulphur compounds (allicin, diallyl sulfide, diallyl disulfide, allyl mercaptan), N-acetyl cysteine, allium compounds, carotenoids (including but not limited to β-carotene), coumarins, dietary fibres, dithiolthiones, flavonoids (eg myricetin, quercetin, rutin), indoles, inositol, inositol hexaphosphate, isoflavones (genistein, daidzein), isothiocyanates, monoterpenes (eg limonene, perillic acid, methol, carveol), wheat bran, diterpene esters, polyphenols, riboflavin 5' phosphate, cinnamaldehyde, vanillin, umbelliferone, phenols (eg cinnamic acid), polyphenols, plant sterols (eg sitostanol, stigmasterol, campesterol), acylglycosylsterols, phytosteroids, protease inhibitors, saponins, isoprenoids, terprenoids, tocotrienols, retinoids, ellagic acid, polyamines, resveratrol, hydroxycinnamic acids [eg (E)-ferulic acid and (E)-p-coumaric acid], chlorophyllin, propolis and some of its components (eg caffeic acid, phenyl esters, artellipin C), red wine, tannic acid, mushroom extracts, anthocyanins (eg cyanidins), mushroom β-glucans (eg lentinan), spinach leaf extracts, natural antioxidant mixture from spinach leaf, noni juice, vitamins A, B6, C, and E, extract of Siamese cassia, extract of Beta vulgaris, extracts of lemon grass and bamboo grass, carnosic acid, capsaicin, sesquiterpene lactones (eg parthenolide, costunolide, yomogin), cotylenin A, humulone, and omega-3 fatty acids (including eicosapentaenoic acid (EPA) and docasahexaenoic acid (DHA)), and combinations thereof.

In one embodiment the anti-tumour food component is selected from the group comprising vitamin D, vitamin B6, taurine, arginine, glutamine, α-lactalbumin, colostrum whey, full or partial casein hydrolysates, casein peptide(s) known to be immunostimulatory (eg immunocasokinins, caseinophosphopeptides, casomorphins, casokinins), colostrinin peptide, colostrum, calcium and calcium phosphate, folate, cysteine-rich milk proteins, lactoperoxidase, HAMLET (α-lactalbumin-oleic acid complex), fragments of plasminogen, prosaposin, saposins, catalase, lactoperoxidase, fatty acid binding protein, ribonuclease, beta-glucuronidase inhibitor, BRCA1, BRCA2, CD36, interferon, tumour necrosis factor, interleukin 2 (IL-2), kininogen and fragments, kininostatin, cystatin, fetuin, neutrophil defensins, interleukin 12 (IL-12), chitinase-like proteins, dystroglycan, prostasin, SPARC-like proteins, and thrombospondin, or a combination thereof.

### Soy Protein

Soy has been promoted as an agent that aids heart health and healthy bones, prevents cancer and alleviates menopausal symptoms (Kerwin, 2004). The anti-cancer effects of soy have been attributed to soy protein itself which is lower in sulphur amino acid content than animal protein and has been shown to inhibit the development of carcinogen-induced tumors in animals. Other soy components with anti-cancer activity include protease inhibitors, isoflavones such as genistein which can have anti-cancer or pro-cancer effects, and saponins.

### Vitamin D and Vitamin D Receptor Ligands

Vitamin D is widely reported as having anti-cancer properties (Harris, et al., 2004). In addition, vitamin D is believed to decrease the risk of developing many common and serious diseases apart from cancer, including type 1 diabetes, multiple sclerosis, cardiovascular disease, and osteoporosis (Holick, 2004). Dairy products are a major dietary source of vitamin D in countries such as New Zealand where milk and other dairy products are fortified with vitamin D. Vitamin D3 is 25-hydroxylated in the liver and converted in the kidney and peripheral organs into the active hormonal form 1-alpha,25-dihydroxy [1,25(OH)2D3] (calcitriol), which affects multiple processes related to cell growth and development. It exerts antiproliferative effects on tumour cells by causing arrest in the G0/G1 phase of the cell cycle, which leads to the down-regulation of growth promoting factors such as IGF-1, and the upregulation of negative growth regulators such as transforming growth factor beta. It causes tumour apoptosis, and is an inhibitor of tumour angiogenesis and metastasis (Nakagawa, et al., 2005). Administration of the active 1,25(OH)2D3 metabolite at doses necessary to inhibit tumor growth are associated with hypercalcemic toxicity. Synthetic structural analogs of 1,25(OH)2D3 that do not induce hypercalcemia have been reported to inhibit tumour growth and induce regression of tumors in animal models (Colston, et al., 2003; Nolan, et al., 1998; van Weelden, et al., 1998). The anti-cancer activity of vitamin D receptor (VDR) ligands has been demonstrated in models of carcinoma of the bladder, breast, colon, endometrium, kidney, lung, pancreas, prostate, sarcomas of the soft tissues and bone, neuroblastoma, glioma, melanoma, squamous cell carcinoma (SCC), and others (Beer, et al., 2004).

Phase I trials have demonstrated that intermittent weekly dosing allows substantial dose escalation and has produced potentially therapeutic peak calcitriol concentrations (Beer, et al., 2001). A phase II study reported encouraging levels of anti-tumour activity for the combination of high-dose 1α,25-dihydroxyvitamin D and docetaxel administered on a weekly schedule in patients with androgen-independent prostate cancer (Beer, et al., 2003).

Numerous studies have shown that daily intakes of 10,000 IU in humans are safe (in the absence of sunshine). Most cases of vitamin D toxicity have been reported to occur after the ingestion of greater than 50,000 IU daily for several years. Some argue that vitamin D is not particularly toxic (Saul, 2003).

Compounds that act as vitamin D receptor (VDR) ligands and that may be useful herein are generally grouped into three classes: (1) "deltanoids" that have a secosteroid scaffold, (2) "pseudo-secosteroids" that have the A-ring of vitamin D but one of the C or D rings is broken, and (3) "non-secosteroids" that are structurally distinct from secosteroids. For reviews of useful vitamin D analogs see Guyton, et al., 2003; Guyton, et al., 2001; Peleg, et al., 2003; and Yee, et al., 2005.

Useful vitamin D analogs are described in international application PCT/NZ2007/000389 (and references cited therein) incorporated herein in its entirety.

Assessment of these analogs in a method of the invention may be carried out by following the protocols described in the examples below.

### 9. Immune enhancement

The present inventors have found that milk fat, optionally with at least one additional therapeutic agent, is able to stimulate and enhance the immune system. In particular, as shown in the examples below, milk fat, optionally with at least one additional factor, is able to stimulate the generation of antigen-specific cytolytic activity (the activity of immune cells, particularly cytotoxic T-lymphocytes) and/or NK cell activity, improve the cellular immune response to antigens (through the activity of at least cytotoxic T-lymphocytes), improve immune protection (by at least restoring the activity of cytotoxic T-lymphocytes and/or NK cells and enhancing cytokine production), restore immune protection (by at least restoring or stimulating the activity of cytotoxic T-lymphocytes and/or NK cell activity and enhancing cytokine production) and generate pro-inflammatory and immunoregulatory mediators (Th1 and Th2 cytokines). It is believed that any functional variant of milk fat, including milk fat fractions and the like, whether or not in combination with at least one additional therapeutic agent, will exhibit similar activity as milk fat. Similarly, it is believed that when lactoferrin is to be administered, any functional variant or functional fragment of lactoferrin will exhibit similar activity as lactoferrin.

As shown in the Examples below, milk fat is effective in improving the generation of antigen-specific cytolytic activity and/or NK cell activity, improving the cellular immune response to antigens, improving immune protection and restoring immune protection.

Accordingly, the present invention relates to a method of stimulating the immune system of a subject comprising administration of milk fat or a milk fat analogue, optionally with at least one additional therapeutic agent, such as one or more anti-tumour agents, to the subject. The present invention also relates to methods of increasing the production of Th1 and Th2 cytokines within a tumor of a subject, of increasing the production of Th1 and Th2 cytokines within the intestine of a subject, of increasing the level of Th1 and Th2 cytokines in the systemic circulation of a subject, and of increasing an anti-tumour immune response in a subject.

In one embodiment of these methods of the invention, the subject is undergoing or will undergo a cancer therapy as described above.

In one embodiment the subject has undergone therapy, but is in relapse or is susceptible to relapse. In one embodiment the subject has a tumour refractory to therapy with a chemotherapeutic, anti-angiogenic or immunotherapeutic agent. In one embodiment the subject has previously undergone unsuccessful therapy with a chemotherapeutic, anti-angiogenic or immunotherapeutic agent.

In one embodiment the Th1 cytokine is selected from IL-18, TNF-α and IFN-γ. In one embodiment the Th2 cytokine is selected from IL-4, IL-5, IL-6 and IL-10. In one embodiment the level of Th1 or Th2 cytokine or cytokines is increased by at least about 5,10, 20, 30, 40, 50, 60, 70, 80, 90, 100, 150, 200, 250, 300, 350, 400, 450, 500, 550, 600, 650, 700, 750 or 800%

Where appropriate, these methods may be combined with treatments employing any one or more of the anti-tumour agents (including chemotherapeutic agents or immunotherapeutic agents) or anti-tumour therapies described below.

### 10. Cancer prevention

The present inventors have found that milk fat, whether alone or in combination with at least one additional therapeutic agent, is able to inhibit tumour formation and inhibit tumour growth and inhibiting tumour metastasis. Milk fat, alone or in combination with lactoferrin and particularly metal ion lactoferrin, releases or stimulates the release of anti-tumour factors such as T-cells and/or NK (natural killer) cells and apoptosis-inducing factors into systemic circulation, stimulates anti-tumour immunological activity and displays immune enhancing activity, anti-angiogenic activity and direct tumour cytotoxicity, and is able to induce apoptosis of tumour cells as shown in the examples below. It is believed that any functional variant of milk fat, or a milk fat fraction, or a milk fat analogue will exhibit similar activity as milk fat.

The present invention has utility in preventing cancer, particularly in preventing relapse (tumour growth) after surgery such as often results from growth and proliferation of secondary tumours, preventing tumour spread after diagnosis and preparing subjects for administration of an anti-tumour agent or anti-tumour therapy.

Solid tumours must form new blood vessels before they are able to grow beyond a certain size. Therefore, inhibiting angiogenesis, particularly tumour angiogenesis (blood vessel formation to supply tumours) has clear applications in treating cancer (Dass, 2004). As shown in the Examples below, orally administered milk fat, whether alone or in combination with lactoferrin and particularly metal ion lactoferrin, is able to significantly reduce the number of vessels in tumours and significantly reduce blood flow.

Inhibiting angiogenesis also has applications in other disorders including but not limited to cardiovascular diseases (atherosclerosis and restenosis for example), chronic inflammation (rheumatoid arthritis, osteoarthritis, and Crohn's disease for example), diabetes (diabetic retinopathy), psoriasis, eridometriosis, macular degeneration and adiposity. Therefore, milk fat or a milk fat analogue, preferably in combination with at least one additional therapeutic agent, more preferably lactoferrin or a functional variant or functional fragment thereof and particularly metal ion lactoferrin, have applications outside of cancer treatment and prevention.

Similarly, orally administered milk fat or a milk fat analogue, whether alone or in combination with at least one additional therapeutic agent, such as lactoferrin and particularly metal ion lactoferrin, is able to induce apoptosis of tumour cells, as shown in the Examples below. The Examples also show that apoptotic factors are present in blood serum of mice fed metal ion lactoferrin.

Therefore, the present invention also relates to methods of inhibiting tumour formation in a subject, inducing apoptosis in a subject, inducing apoptosis of tumour cells in a subject, inhibiting angiogenesis in a subject and inhibiting tumour angiogenesis in a subject comprising administration of lactoferrin or a metal ion functional variant or functional fragment thereof to the subject.

The present invention also relates to a method of maintaining or increasing anti-tumour factors in systemic circulation.

In one embodiment the subject is susceptible to cancer. In one embodiment the subject has a tumour refractory to therapy with a chemotherapeutic, anti-angiogenic or immunotherapeutic agent. In one embodiment the subject has previously undergone unsuccessful therapy with a chemotherapeutic, anti-angiogenic or immunotherapeutic agent.

Where appropriate, these methods may be combined with treatments employing any one or more of the anti-tumour agents (including chemotherapeutic agents or immunotherapeutic agents) or anti-tumour therapies described below.

### 11. Cancer treatment and prevention with combination therapies

The present inventors have found that milk fat is able to inhibit tumour growth and inhibiting tumour metastasis. Milk fat synergizes with immunotherapy (including that mediated by intratumoral gene transfer of B7-1), with chemotherapy (including with paclitaxel, doxorubicin, epirubicin or fluorouracil) or with dendritic cell therapy to substantially eradicate tumours. Milk fat is able to synergize with chemotherapy (including with paclitaxel, doxorubicin, epirubicin, fluorouracil, cyclophosphamide or methotrexate) to inhibit tumour growth and tumour metastasis. It is believed that any functional variant of milk fat, including milk fat fractions and the like, or a milk fat analogue, whether or not in combination with at least one additional therapeutic agent, will exhibit similar activity as milk fat. Similarly, it is believed that when lactoferrin is to be administered, any functional variant or functional fragment of lactoferrin will exhibit similar activity as lactoferrin. Milk fat alone or in combination with other therapies was able to suppress the outgrowth of 4T1 breast cancer tumours that disseminate to the lung and liver, and so is able to inhibit tumour metastasis.

As described above, milk fat was found to release or stimulate anti-tumour factors such as T-cells and/or NK (natural killer) cells and apoptosis-inducing factors into or in the systemic circulation, display immune enhancing activity, anti-angiogenic activity and direct tumour cytotoxicity, and the ability to induce apoptosis of tumour cells as shown in the examples below.

In one embodiment the chemotherapeutic agent is paclitaxel, doxorubicin, epirubicin, fluorouracil, cyclophosphamide or methotrexate.

In addition to the methods described above, the present invention relates to methods of inhibiting tumour growth or inhibiting tumour metastasis in a subject and methods of treating or preventing cancer in a subject comprising
(a) administration of milk fat, optionally with at least one additional therapeutic agent, and
(b) separate, simultaneous or sequential administration of at least one anti-tumour agent or anti-tumour therapy.

In one embodiment the subject is suffering from or is susceptible to cancer. In one embodiment the subject has a tumour refractory to therapy with a chemotherapeutic, anti-angiogenic or immunotherapeutic agent. In one embodiment the subject has previously undergone unsuccessful therapy with a chemotherapeutic, anti-angiogenic or immunotherapeutic agent.

In one embodiment the at least one anti-tumour agent is administered orally or parenterally although the preferred route depends on the anti-tumor agent selected. Preferably the at least one anti-tumour agent is administered orally or by intravenous, intraperitoneal or intratumoural injection. Preferably paclitaxel, doxorubicin, epirubicin, fluorouracil, cyclophosphamide and methotrexate are administered by intravenous or intraperitoneal injection. Preferably the expression plasmid encoding B7-1 is administered by intratumoural injection. Alternatively, tumour cells can be harvested from a patient, transfected ex vivo with B7-1 expression plasmid, then transfected cells injected into a patient. Alternatively, soluble B7-Ig fusion protein can be parenterally delivered. Preferably the dendritic cell therapy is administered by intravenous, intraperitoneal, or intratumoural injection.

In one embodiment the milk fat or a milk fat analogue is administered orally or parenterally.

In one embodiment the milk fat or a milk fat analogue, optionally with at least one additional therapeutic agent, is administered daily for at least about 1, 2, 3, 4, 5, 6, 7, 8, 9 or 10 weeks before administration of the anti-tumour agent or anti-tumour therapy.

In one embodiment the milk fat or a milk fat analogue, optionally with at least one additional therapeutic agent, is administered for at least about 1, 2, 3, 4, 5, 6, 7, 8, 9, 10, 11, 12, 13, 14, 15, 16, 17, 18, 19, 20 or 21 days or for at least about 1, 2, 3, 4, 5, 6, 7 or 8 weeks or for at least about 1, 2, 3, 4, 5 or 6 months before administration of the anti-tumour agent or the anti-tumour therapy

In one embodiment the milk fat or a milk fat analogue, optionally with at least one additional therapeutic agent, is administered for at least about 1, 2, 3, 4, 5, 6, 7, 8, 9, 10, 11, 12, 1.3, 14, 15, 16, 17, 18, 19, 20 or 21 days or for at least about 1, 2, 3, 4, 5, 6, 7 or 8 weeks or for at least about 1, 2, 3, 4, 5 or 6 months after administration of the anti-tumour agent or the anti-tumour therapy has begun.

Preferably the milk fat or a milk fat analogue, optionally with at least one additional therapeutic agent, is administered at least once daily including continuously over a day orally, by parenteral drip or by a combination of administrative routes (oral and parenteral, for example).

In one embodiment of a method of the invention the tumour is a large tumour, as described above.

In one embodiment of a method of the invention one or more of the white blood cell count, the red blood cell count, or the myeloid cell count of the subject is maintained or improved.

In one embodiment the milk fat or a milk fat analogue, and when administered the at least one additional therapeutic agent are administered in a dosage form comprising digestible fat, more preferably digestible fat and digestible protein, preferably lactoferrin, casein or other protein such as other edible proteins.

In one embodiment the milk fat or a milk fat analogue and at least one additional therapeutic agent provide a synergistic therapeutic effect that is better than the effects or either one alone, preferably better than the additive effects of either one alone. For example, preferably there is a greater effect on inhibition of tumour formation or growth, tumour regression, cytolytic effects, immune enhancement, generation of Th1 and Th2 cytokines, maintainenance or improvement in white blood cell count, red blood cell count, or myeloid cell count, treatment or prevention of anemia, cachexia, mucositis, or the responsiveness of a subject or a tumour to the treatment method.

These methods may be combined with treatments employing any one or more of the anti-tumour agents (including chemotherapeutic agents, immunotherapeutic agents, anticachectic agents, antimucositic agents, or hematopoietic agents) or anti-tumour therapies described below.

In one embodiment the anti-tumour therapy is selected from therapies such as, but not limited to, surgery, chemotherapies, radiation therapies, hormonal therapies, biological therapies/immunotherapies, anti-angiogenic therapies, cytotoxic therapies, vaccines, nucleic acid-based vaccines (eg nucleic acids expressing a cancer antigen such as DNA vaccines including p185 vaccines), viral-based therapies (eg adeno-associated virus, lentivirus), gene therapies, small molecule inhibitor therapies, nucleotide-based therapies (eg RNAi, antisense, ribozymes etc), antibody-based therapies, oxygen and ozone treatments, embolization, and/or chemoembolization therapies.

In one embodiment the anti-tumour therapy or anti-tumour agent is selected from chemotherapeutic agents including but not limited to those listed in published international patent application WO 2006/054908, incorporated by reference herein in its entirety.

In one preferred embodiment the chemotherapeutic agent is selected from paclitaxel, doxorubicin, epirubicin, fluorouracil, cyclophosphamide and methotrexate.

In one embodiment the anti-tumour agent is an immunotherapeutic agent. Preferably the immunotherapeutic agent is an expression plasmid encoding the T cell co-stimulator B7-1, a T cell co-stimulator, or a functionally related molecule, for example a B7-Ig chimera.

In one embodiment the anti-tumour agent or therapy comprises dendritic cell therapy.

In one embodiment the radiation therapy includes external beam radiation therapy (including gamma-ray and x-ray therapy) and internal radiation therapy using radioisotopes. Radioisotopes may also be used as anti-tumour agents according to the invention.

### 12. Methods of increasing tumour responsiveness to therapy

The inventors have shown in the Examples below that orally administered milk fat, whether alone or in combination with at least one additional therapeutic agent such as lactoferrin and particularly metal ion lactoferrin, is able to increase the responsiveness of a subject and increase the sensitivity of a tumour to anti-tumour agents. When lactoferrin is administered in combination with milk fat, it is believed that any functional variant, functional fragment, metal ion functional variant or metal ion functional fragment of lactoferrin will exhibit similar activity as a metal ion lactoferrin in combination with milk fat.

Therefore, the present invention also relates to a method of increasing the responsiveness of a subject to a therapy, such as an anti-cancer therapy selected from the group comprising surgery, chemotherapies, radiation therapies, hormonal therapies (eg tamoxifen, aromatase inhibitors), biological therapies/immunotherapies, anti-angiogenic therapies, cytotoxic therapies, vaccines, nucleic acid-based vaccines (eg nucleic acids expressing a cancer antigen such as DNA vaccines including p185 vaccines), viral-based therapies (eg adeno-associated virus, lentivirus), gene therapies, small molecule inhibitor therapies, nucleotide-based therapies (eg RNAi, antisense, ribozymes etc), antibody-based therapies, oxygen and ozone treatments, embolization, and/or chemoembolization therapy and combinations thereof comprising administration of milk fat or a milk fat analogue, optionally with at least one additional therapeutic agent, preferably lactoferrin, to a subject in need thereof separately, simultaneously or sequentially with the therapy.

The present invention also relates to a method of increasing the sensitivity of a tumour in a subject to a cancer therapy comprising oral or parenteral administration of milk fat or a milk fat analogue, optionally with at least one additional therapeutic agent, preferably lactoferrin, to a subject in need thereof separately, simultaneously or sequentially with administration of the therapy. Preferably the milk fat or milk fat analogue is as described above. Preferably the at least one additional therapeutic agent is as described above. Preferably the lactoferrin is as described above. Preferably the therapy is one described above.

Equally, the present invention also relates to a method of speeding the recovery of a subject undergoing cancer therapy comprising administration of milk fat or a milk fat analogue, optionally with at least one additional therapeutic agent, preferably lactoferrin, to a subject in need thereof separately, simultaneously or sequentially with administration of the therapy. In this embodiment of the invention, the subject recovers from the effects of the cancer or the cancer therapy more quickly than a subject not treated according to the invention. Preferably the subject is able to reduce the dose of or time spent receiving a cancer therapy.

These methods may be combined with treatments employing any one or more of the anti-tumour agents (including chemotherapeutic agents or immunotherapeutic agents) or anti-tumour therapies described above:

In one embodiment the milk fat or a milk fat analogue, optionally with at least one additional therapeutic agent are administered daily for at least about 1, 2, 3, 4, 5, 6, 7, 8, 9 or 10 weeks before administration of the anti-tumour agent or anti-tumour therapy. In one embodiment the milk fat or a milk fat analogue, optionally with at least one additional therapeutic agent are administered for at least about 1, 2, 3, 4, 5, 6, 7, 8, 9, 10, 11, 12, 13, 14, 15, 16, 17, 18, 19, 20 or 21 days or for at least about 1, 2, 3, 4, 5, 6, 7 or 8 weeks or for at least about 1, 2, 3, 4, 5 or 6 months before administration of the anti-tumour agent or the anti-tumour therapy. In one embodiment the milk fat or a milk fat analogue, optionally with at least one additional therapeutic agent are administered for at least about 1, 2, 3, 4, 5, 6, 7, 8, 9, 10, 11, 12, 13, 14, 15, 16, 17, 18, 19, 20 or 21 days or for at least about 1,2, 3, 4, 5, 6, 7 or 8 weeks or for at least about 1, 2, 3, 4, 5 or 6 months after administration of the anti-tumour agent or the anti-tumour therapy has begun. with at least one additional therapeutic agent, preferably lactoferrin, to a subject in need thereof separately, simultaneously or sequentially with the therapy.

The present invention also relates to a method of increasing the sensitivity of a tumour in a subject to a cancer therapy comprising oral or parenteral administration of milk fat or a milk fat analogue, optionally with at least one additional therapeutic agent, preferably lactoferrin, to a subject in need thereof separately, simultaneously or sequentially with administration of the therapy. Preferably the milk fat or milk fat analogue is as described above. Preferably the at least one additional therapeutic agent is as described above. Preferably the lactoferrin is as described above. Preferably the therapy is one described above.

Equally, the present invention also relates to a method of speeding the recovery of a subject undergoing cancer therapy comprising administration of milk fat or a milk fat analogue, optionally with at least one additional therapeutic agent, preferably lactoferrin, to a subject in need thereof separately, simultaneously or sequentially with administration of the therapy. In this embodiment of the invention, the subject recovers from the effects of the cancer or the cancer therapy more quickly than a subject not treated according to the invention. Preferably the subject is able to reduce the dose of or time spent receiving a cancer therapy.

These methods may be combined with treatments employing any one or more of the anti-tumour agents (including chemotherapeutic agents or immunotherapeutic agents) or anti-tumour therapies described above:

In one embodiment the milk fat or a milk fat analogue, optionally with at least one additional therapeutic agent are administered daily for at least about 1, 2, 3, 4, 5, 6, 7, 8, 9 or 10 weeks before administration of the anti-tumour agent or anti-tumour therapy. In one embodiment the milk fat or a milk fat analogue, optionally with at least one additional therapeutic agent are administered for at least about 1,2,3, 4, 5, 6,7, 8, 9,10,11, 12, 13, 14, 15, 16, 17, 18, 19, 20 or 21 days or for at least about 1, 2, 3, 4, 5, 6, 7 or 8 weeks or for at least about 1, 2, 3, 4, 5 or 6 months before administration of the anti-tumour agent or the anti-tumour therapy. In one embodiment the milk fat or a milk fat analogue, optionally with at least one additional therapeutic agent are administered for at least about 1, 2, 3, 4, 5, 6, 7, 8, 9, 10, 11, 12, 13, 14, 15, 16, 17, 18, 19, 20 or 21 days or for at least about 1, 2, 3, 4, 5, 6, 7 or 8 weeks or for at least about 1, 2, 3, 4, 5 or 6 months after administration of the anti-tumour agent or the anti-tumour therapy has begun.

### 13. Tumour types

In one embodiment the tumour is, the tumour cells are or the cancer is a leukemia, colon carcinoma, breast cancer, melanoma, skin or lung cancer. Tumour types to which the present invention relates are listed in published international patent application WO 2006/054908 that is incorporated by reference herein.

In one embodiment the tumour is, the tumour cells are or the cancer is a leukemia such as but not limited to, acute leukemia, acute lymphocytic leukemia, acute granulocytic leukemia, acute myelocytic leukemia such as myeloblastic, promyelocytic, myelomonocytic, monocytic, erythroleukemia leukemia and myelodysplastic syndrome, chronic leukemia such as but not limited to, chronic myelocytic leukemia, chronic granulocytic leukemia, chronic lymphocytic leukemia, and hairy cell leukemia.

In one embodiment the tumour is, the tumour cells are or the cancer is a lymphoma such as but not limited to Hodgkin's disease and non-Hodgkin's disease. In one embodiment the tumour is, the tumour cells are from or the cancer comprises a hematopoietic tumor of myeloid lineage such as but not limited to acute and chronic myelogenous leukemia, smoldering multiple myeloma, nonsecretory myeloma and osteosclerotic myeloma. In one embodiment the tumour is, the tumour cells are from or the cancer comprises a hematopoietic tumor of lymphoid lineage, including leukemia, acute and chronic lymphocytic leukemia, acute and chronic lymphoblastic leukemia, B-cell lymphoma, T-cell lymphoma, Burkitts lymphoma. In one embodiment the tumour is, the tumour cells are from or the cancer comprises a hematopoietic tumor of B lymphoid lineage. In one embodiment the tumour is, the tumour cells are from or the cancer comprises a hematopoietic tumor of T lymphoid lineage.

Additional cancers and related disorders that may be treated or prevented by methods and compositions of the present invention include but are not limited to the following: leukemias; lymphomas; multiple myelomas; Waldenström's macroglobulinemia; monoclonal gammopathy of undetermined significance; benign monoclonal gammopathy; heavy chain disease; bone and connective tissue sarcomas; brain tumors; breast cancer; adrenal cancer; thyroid cancer; pancreatic cancer; pituitary cancers; eye cancers; vaginal cancers; vulvar cancer; cervical cancers; uterine cancers; ovarian cancers; esophageal cancers; stomach cancers; colon cancers; rectal cancers; liver cancers; gallbladder cancers; cholangiocarcinomas; lung cancers; testicular cancers; prostate cancers; penal cancers; oral cancers; basal cancers; salivary gland; pharynx cancers; skin cancers; kidney cancers; Wilms' tumor; and bladder cancers. For a review of such disorders, see Fishman et al., 1985, Medicine, 2d Ed., J.B. Lippincott Co., Philadelphia and Murphy et al., 1997, Informed Decisions: The Complete Book of Cancer Diagnosis, Treatment, and Recovery, Viking Penguin, Penguin Books U.S.A.,Inc., United States of America.

Additional cancers or other abnormal proliferative diseases may be treated or prevented according to the invention include but are not limited to the following: carcinoma, including that of the liver, spleen heart, lung, small intestine, large intestine, rectum, kidney, brain, bladder, breast, colon, kidney, liver, lung, ovary, pancreas, stomach, cervix, thyroid and skin; including squamous cell carcinoma; hematopoietic tumors of lymphoid lineage, including leukemia, acute lymphocytic leukemia, acute lymphoblastic leukemia, B-cell lymphoma, T-cell lymphoma, Burkitts lymphoma; hematopoietic tumors of myeloid lineage, including acute and chronic myelogenous leukemias and promyelocytic leukemia; tumors of mesenchymal orignin, including fibrosarcoma and rhabdomyoscarcoma; other tumors, including melanoma, seminoma, tetratocarcinoma, neuroblastoma and glioma; tumors of the central and peripheral nervous system, including astrocytoma, neuroblastoma, glioma, and schwannomas; tumors of mesenchymal origin, including fibrosacoma, rhabdomyoscarama, and osteosarcoma; and other tumors, including melanoma, xenoderma pegmentosum, keratoactanthoma, seminoma, thyroid follicular cancer and teratocarcinoma.

In specific embodiments, malignancy or dysproliferative changes (such as metaplasias and dysplasias), or hyperproliferative disorders, are treated or prevented in the ovary, bladder, breast, colon, liver, lung, skin, pancreas, or uterus. In other specific embodiments, sarcoma, melanoma, or leukemia is treated or prevented.

### 14. Skin cancer treatment or prevention

A further embodiment of the present invention is a method of treating or preventing skin cancer comprising the step of applying milk fat or a milk fat analogue, optionally with at least one additional therapeutic agent, preferably lactoferrin, in or on the skin, and/or in the vicinity of the tumor.

In a preferred embodiment the skin is predisposed to skin cancer due to sun exposure.

In a preferred embodiment the cancer is a basal cell carcinoma, a squamous cell carcinoma, or a melanoma.

Preferably, the milk fat or a milk fat analogue is administered topically, either alone or in combination with standard anti-cancer regimens. Administration in the vicinity of the tumor includes administration near or adjacent to the margins of the tumor or directly in the margin area of the tumor. It is envisioned that milk fat inhibits carcinogenesis, stimulates anti-tumour immunity in the local tissue, inhibits tumour angiogenesis, and/or is directly tumouricidal (able to inhibit tumour growth). Briefly, milk fat, optionally with at least one additional therapeutic agent in a suitable carrier at strengths of 0.1%, 1%, 5 %, or 10% is applied twice a day to at-risk skin or cancerous skin lesion. Size progression of the tumour is monitored through CT scans and tumor markers where available.

Doses and treatment regimes can be informed by undertaking preclinical trials in a suitable animal model of skin cancer. A region of the skin of mice is shaved and treated with topical application of a carcinogen (for example, 7,12-dimethylbenz(a)-anthracene (DMBA)) that may be followed by irradiation with UV-B (Bestak, et al., 1996). Lactoferrin may be applied two days after carcinogen treatment or once a cancerous lesion has formed, preferably in the presence of a dermal penetration enhancer (such as 70% laureth sulphate and 30% phenylpiperazine) that could increase skin permeability. Lactoferrin is applied twice a day, or as otherwise required, to the skin or cancerous lesion and tumour growth monitored over a period of weeks to months.

Where appropriate, these methods may be combined with treatments employing any one or more of the anti-tumour agents (including chemotherapeutic agents or immunotherapeutic agents) or anti-tumour therapies described above.

### 15. Anemia

As shown in the Examples below, oral administration of milk fat is effective in reducing hematological suppression in a subject, and in treating or preventing anemia in a subject.

Anemia (sometimes anaemia) refers to a deficiency of red blood cells (RBCs) and/or hemoglobin. This results in a reduced ability of blood to transfer oxygen to the tissues, causing hypoxia. Varying degrees of anemia can therefore have a wide range of clinical consequences.

Using a morphological approach, anemia can be classified by the size of red blood cells. The size is reflected in the mean corpuscular volume (MCV), typically measured in femtolitres (fl). If the cells are smaller than normal (under 80 fl), the anemia is said to be microcytic; if they are normal size (80-100 fl), normocytic; and if they are larger than normal (over 100 fl), the anemia is classified as macrocytic.

Microcytic anemia is primarily a result of failures or insufficiencies in hemoglobin synthesis, and can be caused by several etiologies, including defects in heme synthesis, iron deficiency, anemia of chronic disorders, defects in globin synthesis, alpha- and beta-thalassemia, HbE syndrome, HbC syndrome, and other unstable hemoglobin diseases, sideroblastic defects including hereditary sideroblastic anemia, acquired sideroblastic anemia including lead toxicity, and reversible sideroblastic anemia. Iron deficiency anemia is the most common type of anemia overall and it has many causes. RBCs on often appear hypochromic (paler than usual) and microcytic (smaller than usual) when viewed with a microscope. Iron deficiency anemia is commonly caused by insufficient dietary intake or absorption of iron. Iron deficiency anemia can also be due to bleeding lesions of the gastrointestinal tract. Hemoglobinopathies, such as Sickle-cell disease and Thalassemias are usually classified as microcytic anemias.

Normocytic anaemia is when the overall Hb levels are decreased, but the red blood cell size (MCV) remains normal. Causes include acute blood loss, anemia of chronic disease, aplastic anemia (bone marrow failure), and hemolytic anemia. Hemolytic anemia causes a separate constellation of symptoms (also featuring jaundice and elevated LDH levels) with numerous potential causes. It can be autoimmune, immune, hereditary or mechanical (e.g. heart surgery). While frequently normocytic, it can result, because of cell fragmentation, in a microcytic anemia, or, because of premature release of immature red blood cells from the bone marrow, a macrocytic anemia.

Macrocytic anemia can be further divided into "megaloblastic anemia" or "non-megaloblastic macrocytic anemia". The cause of megaloblastic anemia is primarily a failure of DNA synthesis with preserved RNA synthesis, which result in restricted cell division of the progenitor cells. The megaloblastic anemias often present with neutrophil hypersegmentation (6-10 lobes). Megaloblastic anemia is the most common cause of macrocytic anemia. Megaloblastic anemia is commonly due to a deficiency of either vitamin B12 or folic acid (or both), in turn usually due either to inadequate intake or insufficient absorption. Folate deficiency normally does not produce neurological symptoms, while B12 deficiency does. Pernicious anemia is an autoimmune condition directed against the parietal cells of the stomach. Parietal cells produce intrinsic factor, required to absorb vitamin B12 from food. Therefore, the destruction of the parietal cells causes a lack of intrinsic factor, leading to poor absorption of vitamin B12. Megaloblastic anemia may also result from exposure to Methotrexate, zidovudine, and other drugs that inhibit DNA replication. The non-megaloblastic macrocytic anemias have different etiologies (i.e. there is unimpaired DNA synthesis,) which occur, for example in alcoholism.

Various specific anemias exist, including anemia of prematurity, which occurs in premature infants at 2 to 6 weeks of age and results from diminished erythropoietin response to declining hematocrit levels; Fanconi anemia, an hereditary disorder or defect featuring aplastic anemia and various other abnormalities; hereditary spherocytosis, a hereditary defect that results in defects in the red blood cell (RBC) membrane, causing the erythrocytes to be sequestered and destroyed by the spleen. This leads to a decrease in the number of circulating RBCs and, hence, anemia; sickle-cell anemia, a hereditary disorder, is due to the presence of the mutant hemoglobin S gene; warm autoimmune hemolytic anemia, an anemia caused by autoimmune attack against red blood cells, primarily by IgG; and cold agglutinin hemolytic anemia, primarily mediated by IgM.

Accordingly, the present invention relates to a method of treating or preventing anemia in a subject comprising administration of milk fat or a milk fat analogue, optionally with at least one additional therapeutic agent, to the subject. The present invention also relates to methods of reducing or inhibiting hematological suppression in a subject.

Reducing or inhibiting hematological suppression has application not only in treating subjects undergoing or who have undergone cancer therapy, but in other disorders including but not limited to the anemias described above. Therefore, milk fat or a milk fat analogue, preferably in combination with at least one additional therapeutic agent, more preferably lactoferrin or a functional variant or functional fragment thereof and particularly metal ion lactoferrin, have applications outside of cancer treatment and prevention.

### 16. Cachexia

As shown in the Examples below, oral administration of milk fat is effective in improving cachectic status of a subject and in treating or preventing cachexia in a subject.

Cachexia is one or more of loss of weight, muscle atrophy, fatigue, weakness and anorexia (a significant loss of appetite) in someone who is not actively trying to lose weight. It is usually associated with an underlying disorder, such as cancer, certain infectious diseases (e.g. tuberculosis, AIDS) and some autoimmune disorders. Cachexia physically weakens patients to a state of immobility stemming from anorexia, asthenia, and anemia, and response to standard treatment is usually poor.

Cancer patients with cachexia, whether from decreased physical activity, concomitant infection, or toxicities to the alimentary tract from chemotherapy and radiotherapy, are generally managed symptomatically for maintenance of nutritional status and quality of life.'Such management includes the use of mouthwash for stomatitis, frequent small volume feedings, antiemetics, antibiotics, transfusions of blood components, and oral and parenteral nutritional supplement. Food supplements can be effective in providing additional calories, protein, fat, vitamins, and minerals. In specific instances, such as the malabsorption syndrome secondary to pancreas carcinoma, exogenous pancreas extract has been used to improve fat and protein absorption (Perez MM, et al. Assessment of weight loss, food intake, fat metabolism, malabsorption, and treatment of pancreatic insufficiency in pancreatic cancer. Cancer 1983;52:346-52).

Various pharmacological agents have been administered in attempts to reverse, retard or halt progressive cachexia in cancer patients. These include corticosteroids, such as prednisolone, methylprednisolone, and dexamethasone, progestational agents, such as megestrol acetate, medroxyprogesterone acetate, cannabinoids, such as dronabinol, serotonin antagonists, such as cyproheptadine, prokinetic agents, such as metoclopramide and cisapride, anabolic steroids, such as nandrolone decanoate and fluoxymesterone, inhibitors of phosphoenolpyruvate carboxykinase, such as hydrazine sulfate, methylxanthine analogs, such as pentoxifylline and lisofylline, thalidomide, cytokines and anticytokines, such as Anti-IL-6 antibody, IL-12, branched-chain amino acids, eicosapentaenoic acid, inhibitors of prostaglandin synthesis, such as indomethacin and ibuprofen, hormones, such as melatonin, and β2-adrenoceptor agonists, such as clenbuterol.

Accordingly, the present invention relates to a method of treating or preventing cachexia in a subject comprising administration of milk fat or a milk fat analogue, optionally with at least one additional therapeutic agent, to the subject. The present invention also relates to methods of improving the cachectic status of a subject.

Treating or preventing cachexia has application not only in treating subjects undergoing or who have undergone cancer therapy, but in other disorders associated with weight loss or fatigue, including but not limited to the disorders described above. Therefore, milk fat or a milk fat analogue, preferably in combination with at least one additional therapeutic agent, more preferably lactoferrin or a functional variant or functional fragment thereof and particularly metal ion lactoferrin, has applications outside of cancer treatment and prevention.

### 17. Mucositis

As shown in the Examples below, oral administration of milk fat is effective in ameliorating damage to the gut such as ulceration and in treating or preventing mucositis.

Mucositis is a condition characterized by damage to the epithelium of the oral-pharyngeal cavity and gastrointestinal (GI) tract, resulting in inflammation and ulceration of these mucous membranes. In the oral cavity and esophagus, mucositis is characterized by painful ulceration. Further down the digestive tract, mucositis causes diarrhea, which is often severe and debilitating. Mucositis usually results from radiation and/or chemotherapy, occurring to some degree in approximately 40% of patients who receive cancer chemotherapy. Epithelial cells are more susceptible to the cytotoxic effects of radiation and chemotherapy because of their relatively high rate of turnover compared to cells in other organs. In most instances, epithelial cells of the mucous membranes have a more rapid turnover than the cancer being treated and are vulnerable to damage by cytotoxic agents and radiation. Diagnosis and monitoring of mucositis is achieved through patient interview (such as a pain questionnaire), oral exam and endoscopy. Some assays are also suggested to be determinative for diagnosis including sucrose breath test, citrullene and transglutaminase assays.

Treatment of mucositis is mainly supportive. Oral hygiene is currently the mainstay of treatment, typically achieved by frequent washing of the mouth. Water-soluble jellies can be used to lubricate the mouth. A variety of topical palliative agents exist to manage the pain and sensitivity that are associated with mucositis. Salt mouthwash can soothe the pain and keep food particles clear so as to avoid infection. Medicinal agents include Chlorhexidine gluconate, lidocaine (Xylocaine), dyclonine HCl (Dyclone), and benzocaine in Orabase. Additionally, diphenhydramine HCl (Benadryl), which has topical anesthetic activity, may be mixed as a suspension with equal parts of either Kaopectate or milk of magnesia. Benzydamine hydrochloride (HCl) is a nonsteroidal rinse with antiinflammatory, analgesic, and anesthetic properties. The use of sucralfate, widely administered in the treatment of gastric ulcers, as a rinse in the treatment of radiation- and chemotherapy-induced mucositis has been reported by a number of investigators.

Microbicides, such as chlorhexidine gluconate, polymyxin E, tobramycin, and amphotericin have been reported to be of some clinical value. Antiinflammatory agents such as betamethasone and indomethacin have been reported to be of potential value in preventing or reducing the severity of radiation-induced mucositis. Palifermin (KEPIVANCE), a human keratinocyte growth factor (KGF), has been reported to enhance epithelial cell proliferation, differentiation, and migration. Other reported therapies rely on the use of cytokines and other modifiers of inflammation (such as IL-1, IL-11, TGF-beta3), amino acid supplementation (for example, with glutamine), vitamins, colony-stimulating factors such as granulocyte-macrophage colony-stimulating factor (GM-CSF), cryotherapy, and laser therapy.

Accordingly, the present invention relates to a method of treating or preventing cachexia in a subject comprising administration of milk fat or a milk fat analogue, optionally with at least one additional therapeutic agent, to the subject. The present invention also relates to methods of improving the cachectic status of a subject.

Treating or preventing mucositis and reducing or ameliorating damage to the gut has application not only in treating subjects undergoing or who have undergone cancer therapy, but in other disorders associated with damage to gut epithelia, including but not limited to the disorders described above. Therefore, milk fat or a milk fat analogue, preferably in combination with at least one additional therapeutic agent, more preferably lactoferrin or a functional variant or functional fragment thereof and particularly metal ion lactoferrin, has applications outside of cancer treatment and prevention.

### 18. Leukocytopenia

As shown in the examples below, oral administration of milk fat is effective in reducing haematological suppression in a subject, and in treating and preventing leukocytopenia in a subject.

Leukocytopenia (also know as leukopenia, and including the conditions sometimes referred to as lymphopenia or lymphocytopenia) refers to a reduction of white blood cells (WBCs) in an individual. This results in a reduced ability of the body to fight infections causing the body to be very vulnerable. Varying degree of leukocytopenia can therefore equate to a wide range of clinical consequences.

The average adult has a WBC count of 4500-10000 cells/cubic millimetre (varies between sex and individuals). Leukopenia is generally regarded as having a WBC count of less than 4000 cells/cubic millilitre (again varies from sex and individual). The two main forms of leukocytopenia are neutropenia and granulocytopenia.

Neutropenia is the main type of leukocytopenia and is characterised by the reduction of neutrophils. Other than cancer, many syndromes are associated with neutropenia the majority of which are genetic in nature. Kostmann's neutropenia, Shwachmann's syndrome, agammaglobulinemia, dysgammaglobulinemia, myelokathexis, cartilage-hair hypoplasia syndrome and dyskeratosis congenita are just a few examples of disorders associated with neutropenia. Neutropenia is also associated with nutritional deficiencies such as lack of Vitamin B12, folic acid or copper deficiency.

Granulocytopenia is sometimes used interchangeably with neutropenia, but more accurately it is the depression of eosinophils and basophils as well as neutrophils.

Leukocytopenia can also be caused when individuals undergo chemotherapy or radiotherapy. It is a very common side effect and patients are usually given time to recovery and produce more WBCs before therapy is continued. In general, leukocytopenia is normally treated with steroids or vitamins to stimulate the bone marrow into producing more WBCs.

Accordingly, the present invention relates to the method of treating or preventing leukocytopenia in a subject comprising administering of milk fat or a milk fat analogue, optionally with at least one additional therapeutic agent, to the subject. The present invention also relates to method of reducing or inhibiting hematological suppression in a subject.

Reducing or inhibiting hematological suppression has application not only in treating subjects undergoing or who have undergone cancer therapy, but in other disorders including but not limited to the leukocytopenias mentioned above. Therefore milk fat or a milk fat analogue; preferably in combination with at least one additional therapeutic agent, more preferably lactoferrin or a functional variant or functional fragment thereof and particularly metal ion lactoferrin, have applications outside of cancer treatment and prevention.

Various aspects of the invention will now be illustrated in non-limiting ways by reference to the following examples.

### EXAMPLES

### Mice and Reagents

Six to nine week old female C57BL/6 and Balb/c mice (University of Auckland, New Zealand) were used, where each diet group contained 5 or 6 mice unless otherwise indicated. Mice were kept in an air-conditioned room with controlled humidity, temperature, and 12 h light:dark cycle. The mouse EL-4 T cell thymic lymphoma of C57BL/6 origin, and the 4T1 breast cancer cell line of BALB/c (H-2b) origin were purchased from the American Type Culture Collection (Rockville, MD, USA). EL-4 cells were cultured at 37°C in DMEM medium (Gibco BRL, Grand Island, NY, USA), whereas 4T1 cells were maintained as monolayer cultures at 37°C in RPMI 1640 medium (Gibco BRL, Grand Island, NY, USA). The media were supplemented with 10% foetal calf serum, 50 U/ml penicillin/streptomycin, 2 mM L-glutamine, and 1 mM pyruvate. Paclitaxel was obtained from Bristol-Meyers Squibb, WA, USA. All experiments were conducted under a protocol approved by the Animal Ethics Committee, University of Auckland.

### Milk Fat Preparations

Milk fat enriched in conjugated linoleic and vaccenic acids was prepared by supplementary free fatty acid feeding of pasture fed cows according to the method of Harfoot et al (58. Normal anhydrous milk fat was obtained from Fonterra Co-operative Group Limited, NZ. The compositions of the milk fat and enriched milk fat used in the treatment diets are summarised in Tables 6a and 6b. Data in Tables 6a and 6b was obtained using FAMES, extended FAMES, CLA and milk fat analyses known in the art.

**TABLE 6a. Content of milk fat diets**

| **CLA component** | **Normal (% w/w)** | **Enriched (%w/w)** | **Fold increase** |
|---|---|---|---|
| CLA (c-9, t-11) | 1.17 | 5.04 | 3.3 |
| CLA (t-10, c-12) | - | - | |
| CLA (minor c18:2 isomers) | 0.55 | 1.30 | 1.4 |
| TOTAL CLA (all forms) | 1.72 | 6.34 | 2.7 |
| Ratio c-9, t-11 CLA to total CLAs | 68.0% | 79.5% | |

**TABLE 6b. Content of milk fat diets**

| **Fatty acid component** | **Normal (% w/w)** | **Enriched (% w/w)** | **Percentage decrease** |
|---|---|---|---|
| c4:0 (butyric acid) | 3.2 | 3.2 | |
| c6:0 (caproic acid) | 2.3 | 1.7 | 26% |
| c8:0 (caprylic acid) | 1.3 | 1 | 23% |
| c10:0 (capric acid) | 2.8 | 2.2 | 21% |
| c10:1 (2-decenoate) | 0.3 | 0.2 | 33% |
| c12:0 (lauric acid) | 3.2 | 2.5 | 22% |
| c12:1 (11-dodecenoic acid) | 0.2 | 0.1 | 50% |
| c13:0 br (tridecanoic acid br) | 0.1 | 0 | 100% |
| c13:0 (tridecanoic acid) | 0.1 | 0.1 | |
| c14:0 b(myristic acid br) | 0.2 | 0.1 | 50% |
| c14:0 (myristic acid) | 10.9 | 9.1 | 17% |
| c14:1 (myristoleic acid) | 0.9 | 0.8 | 11% |
| c15:0 iso br | 0.4 | 0.3 | 25% |
| c 15:0 ante-iso br | 0.6 | 0.6 | |
| c15:0 (pentadecanoic acid) | 1.3 | 1.2 | 8% |
| c16:0 br | 0.2 | 0.2 | |
| c16:0 (palmitic acid) | 30.6 | 19.7 | 36% |
| c16:1 (palmitoleic acid) | 1.8 | 3 | |
| c17:0 iso br | 0.6 | 0.6 | |
| c 17:0 ante-iso br | 0.4 | 0.5 | |
| c17:0 (margaric acid) | 0.8 | 0.5 | 38% |
| c17:1 | 0.3 | 0.3 | |
| c18:0 (stearic acid) | 10.5 | 4.6 | 56% |
| c18:1 n-9 (oleic acid) | 16.6 | 11.9 | 28% |
| c18:1 n-7 (vaccenic acid) | 4.7 | 22.9 | |
| c18:2 n-6 (linoleic acid) | 1.4 | 2.1 | |
| c18:3 n-3 | 0.8 | 0.4 | 50% |
| c18:2 (CLA) | 1.2 | 5.3 | |
| c18:4 + CLA isomers | 0 | 0 | |
| c20:0 (arachidic acid) | 0.2 | 0.1 | 50% |
| c20:1 n-11 | 0.1 | 0.1 | |
| c20:1 n-9 (eicosenoic acid) | 0 | 0.1 | |
| c20:2 n-6 | 0 | 0 | |
| c20:3 n-3 (eicosatrienoic acid) | 0.1 | 0 | 100% |
| c20:4 n-6 (arachidonic acid) | 0.1 | 0 | 100% |
| c20:3 n-3 | 0 | 0.1 | |
| c20:4 n-3 (eicosatetraenoic acid) | 0.1 | 0.2 | |
| c20:5 n-3 (eicosapentaenoic acid, EPA) | 0.1 | 0.2 | |
| c22:0 (behenic acid) | 0.1 | 0.1 | |
| c22:1 n-13, n-11 (docosenoic acid) | 0 | 0.1 | |
| c22:2 n-9 | 0 | 0 | |
| c22:4 n-6 | 0 | 0 | |
| c22:5 n-3 (docosapentaenoic acid) | 0.1 | 0 | 100% |
| c24:0 (lignoceric acid) | 0 | 0 | |
| c22:6 n-3 (DHA) | 0 | 0 | |
| c24:1 | 0 | 0 | |

Fatty acids as determined by FAME analysis. Fold increase refers to increase in lipid levels in enriched milk fat versus milk fat. c18:1 n-7 provides an estimate of the c18:1 trans fatty acid content.

### Lactoferrin Preparation

Bovine lactoferrin was prepared from skim milk (Fonterra Co-Operative Group Limited, New Zealand) using the method of Norris et al (Norris, G E *et al.,* 1989). A SP Big Beads ion exchanger was loaded with skim milk and washed with water. The column was eluted with 0-0.5M NaCl solution and the eluate discarded. The column was then eluted with 0.5-1.0M NaCl and the eluate recovered. The recovered eluate was subjected to UF/DF using a 30kDa membrane to reduce salts and low molecular weight components. Filtration was continued until the retentate was between 90 and 93% bovine lactoferrin. The lactoferrin extract obtained had natural levels of iron-saturation of approximately 15% and is referred to as bLf in the following Examples. Iron-saturated bovine lactoferrin extract (100% saturated) was prepared from natural bLf by the method of Law et al., (Law and Reiter, 1977), and is referred to as Lf+ in the following Examples.

### Diets

The experimental diets were prepared by Crop & Food Research, Palmerston North, New Zealand using as a base the powdered AIN93G formulation. Casein was used as the protein source, and soybean oil as the lipid source, in the AIN93G diet, and contained no lactoferrin. The casein was substituted in the experimental diets with natural bLf or Lf+ prepared as described above, such that the total protein content of the diet was unchanged. The diet contained 28 g of iron-saturated bLf or 28 g of natural bLf extract per 2.4 Kg of diet. The soybean oil was substituted in the experimental diets with either enriched milk fat or normal anhydrous milk fat prepared as described above, such that the total fat content of the diet was unchanged. Fresh diet was provided biweekly, and mice had free access to food and water throughout the study. The Phospholipid Concentrate Phospholac 600™ (PC600™) phospholipid fraction was sourced from Fonterra Co-operative Group Limited, New Zealand.

### Experimental Tumour Models and Therapies

Tumours were established by s.c. injection of 2 x 10⁵ EL-4 or 2 x 10⁴ 4Tl cells into the left flank of mice, and tumour growth determined by measuring two perpendicular diameters. Animals were euthanased when tumours reached more than 1.0 cm in diameter, in accord with Animal Ethics Approval (University of Auckland). All experiments included 5 or 6 mice per treatment group, unless otherwise indicated. Paclitaxel (30 mg dissolved in 5 ml of Cremophor® EL and dehydrated alcohol) was diluted in 0.9% NaCl and administered i.p. at 30 mg/Kg. In the case of the 4T1 metastatic model of breast cancer, after the mice were euthanased at the end of experiments, the organs including lung, liver and spleen were removed and weighed. The numbers of lung surface metastases were counted. The livers were fixed with 4% paraformaldehyde and transverse 10 µm sections were made at 5 different levels to cover the entire liver. The sections were stained with haematoxylin and eosin. Metastatic nodules containing more than 6 cancer cells were counted, and the mean was taken to represent the number of metastases. Blood was collected by cardiac puncture at the time of autopsy, and blood cells were counted with a hemocytometer.

### Measurement of the Generation of Antitumour Cytotoxic T-lymphocytes (CTLs)

Splenocytes were harvested following tumour cell injection on days specified. They were incubated at 37°C with EL-4 target cells in graded E:T ratios in 96-well roundbottom plates. After a 4-hour incubation, 50 µl of supernatant was collected, and lysis was measured using the Cyto Tox 96 Assay Kit (Promega, Madison, WI, USA). Background controls for non-specific target and effector cell lysis were included. After background subtraction, percentage of cell lysis was calculated using the formula: 100 x (experimental spontaneous effector-spontaneous target/maximum target-spontaneous target).

### Measurement of Apoptosis

Tumours were excised and immediately frozen in dry ice, and stored at -70°C for later *in situ* detection of apoptotic cells in tumours. Frozen serial sections of 6-µm thickness were fixed with paraformaldehyde solution (4% in PBS, pH 7.4), and permeabilized with a solution containing 0.1% Triton X-100 and 0.1 % sodium citrate. They were incubated with 20 µl TUNEL reagent (In Situ apoptosis detection kit from Boehringer Mannheim, Germany) for 60 min at 37°C, and examined by fluorescence microscopy. Adjacent sections were counterstained with haematoxylin to count the total number of cells, or the number of apoptotic cells in ten randomly selected fields (magnification of x40). The apototic index (AI) was calculated as the number of apoptotic cells x 100/total number of nucleated cells. For detection of apoptotic cells *in vitro,* the numbers of apoptotic and necrotic tumour cells were measured by staining with annexin-V-fluos, TUNEL, and trypan blue. For measurement of gut apoptosis, jejunal sections were stained by the TUNEL method using a DeadEndTM Fluorometric TUNEL System (Promega, Madison, WI, USA). Apoptotic cells in 10 randomly selected cypts were counted, and the data expressed as apoptotic bodies per crypt.

### Assessment of Vascularity

To determine tumour vascularity, 10-µm frozen tumour sections were fixed with acetone, rinsed with PBS, blocked with 2% BSA for 2 hr, and incubated overnight with the anti-CD31 antibody MEC 13.3, or an anti-CD105 mAb. They were subsequently incubated for 30 min with secondary antibodies using the VECTASTAIN Universal Quick kit (Vector Laboratories, Burlingame, CA, USA); and developed with Sigma FAST DAB (3,3'-diaminobenzidine tetrahydrochloride) and CoC12 enhancer tablets (Sigma), and counterstained with hematoxylin.' Stained blood vessels were counted in five or six blindly chosen random fields (0.155 mm²) at x40 magnification, and the mean of the highest three counts was calculated. To visualize blood vessels open to flow, DiO7 (Molecular Probes, Eugene, OR) was injected into the tail vein at a concentration of 1.0 mg/Kg one minute prior to collecting tissues, as described previously(Ding *et al.,* 2001). The concentric circles method was also used to assess vascularity.

### Analysis of Cachexia

The mice were weighed at the start and end of the experiment. After the mice were euthanased, the tumours were excised and weighed. To determine the extent of adipose tissue and muscle wasting, epididymal adipose tissue and the left gastrocnemius muscle were dissected and weighed. The carcass weight was calculated as the difference in weight between the whole body and the tumour.

### Measurement of Gut Damage

The jejuni from mice were fixed in 4% paraformaldehyde, embedded in paraffin, sectioned at 4 µm, and sections stained with hematoxylin-eosin. In each specimen, 20 well preserved villi were randomly selected and their lengths were measured by microscopy using an objective micrometer.

The activity of jejunal γ-glutamyl transpeptidase (γ-GGT) was recorded as a separate measure of gut damage, as described previously Ziotnik *et al*., 2005). A segment of the jejunum (approximately 5 cm) was excised and flushed with 10 ml of PBS, and cut into two 2 cm² segments which were placed into 1.0 ml of 1.0% Triton X-100, 0.15 M NaCl, 100 mM Tris, pH 8.0 (Tris buffer). Twenty microlitres of a solution containing 0.3 ml of 100 mM glycyl-glycine, pH 8.0, 0.08 ml Tris buffer, 0.5 ml of 5 mM γ-glutamyl-p-nitroanilide was added to final volume of 0.9 ml. The gut slices were incubated for 10 min in a shaking water bath at 37°C, and γ-GGT activity read at 405 nm. Results were expressed as units of γ-GGT activity per cm of jejunum, where one unit is defined as the γ-GGT activity that releases 1.0 mol of p-nitroaniline in 1 h.

### Statistical Analysis

Results were expressed as mean values ± SEM or ± 95% confidence interval, and statistical significance was evaluated using a Student's t test (Examples 1 to 17) or analysis of variance or covariance followed by Tukey's multiple comparison procedure or 1-sided Dunnett's test (Examples 18 to 21). A value of P < 0.05 denotes statistical significance, whereas P < 0.001 denotes results that are highly significant.

### EXAMPLE 1

Bovine lactoferrin of greater than 90% purity was sourced from the Fonterra Co-operative Group. For the preparation of apo-Lf, a solution of Lf at approximately 80 mg/mL in milliQ water (pH ~ 5.7) was adjusted to pH 2.08 by careful addition of 6 M HCl. The solution was stirred at RT for 1 h then dialysed against 10 volumes of 0.1 M citric acid overnight at 4°C using SpectraPor tubing with a nominal molecular weight cut-off of 3.5 kDa (Spectrum Companies, Ranco Dominguez, CA, USA). The dialysis fluid was changed twice over a 24 h period, and the Lf solution freeze-dried to a white semi-crystalline powder. For preparation of 50% Fe-saturated lactoferrin, an 8% solution of lactoferrin in 0.1 M sodium bicarbonate was adjusted to pH 8.2 with careful addition of 6 M NaOH. An appropriate volume of 50 mM ferric nitrilo-triacetate (Fe-NTA) (Bates et al., 1967; Brock & Arzabe, 1976) was added to give ~ 50% saturation of the lactoferrin (allowing for the purity of the Lf and its native Fe saturation of ~ 12%). After stirring for 1 h at RT, the solution (pH 8.01) was dialysed against 10 volumes of milli-Q water overnight at 4° C using SpectraPor tubing as above. The dialysis fluid was changed twice over a 24h period and the Lf solution freeze-dried to a salmon red semi-crystalline powder. Lactoferrin of ~ 100% Fe saturation was prepared essentially as for the 50% Fe-saturated material except that the amount of Fe-NTA was adjusted accordingly, and following addition of Fe-NTA, the pH was re-adjusted to 8.0 with careful addition of 6 M NaOH. The final product was a deep salmon red semi-crystalline powder. Fe saturation levels of the final products were verified by spectrophotometric titration (Bates et al., 1967; Brock & Arzabe, 1976). The apo-lactoferrin was approximately 5% Fe-saturated.

### EXAMPLE 2

This example shows that milk fat suppresses the growth of EL-4 tumours, whereas milk fat enriched in conjugated linoleic and vaccenic acids is ineffective.

Groups of six C57BL/6 mice were fed the base AIN-93 diet, or the same diet substituted with 120 g of either milk fat or enriched milk fat per 2.4 Kg of diet, representing ~71% of the fat component of the diet. Mice were challenged subcutaneously with 2 x 10⁵ EL-4 tumour cells two weeks after being placed on the diets. Tumour size as measured by two perpendicular diameters (in centimetres) was monitored until day 91, or until tumours reached 1 cm in diameter. Each point represents the mean tumour size with 95% confidence intervals for either 6 mice, or the number of mice indicated.

Enriched milk fat slowed the growth of tumours by 25% at day 49 compared to the control diet, but the effect was not significant (Figure 1). In marked contrast, milk fat completely prevented the development of tumours in 2 of 6 mice. The growth of tumours in the other 4 mice fed the milk fat diet was similar to that for mice fed enriched milk fat.

### EXAMPLE 3

This example shows that milk fat synergizes with immunotherapy to eradicate EL-4 tumours.

Tumours were established in groups of five mice fed either enriched milk fat, milk fat, or the control diet, as described in Example 2 above. Tumour size as measured by two perpendicular diameters (in centimetres) was monitored. The tumours were injected with DNA-liposome complexes containing 60 µg of a B7-1 expression plasmid when tumours reached ~0.4 cm in diameter. The timing of administration of the plasmid is indicated by the arrow. Tumour size as measured by two perpendicular diameters (in centimetres) was monitored until day 91, or until tumours reached 1 cm in diameter.Tumours of this particular size remain partially susceptible to B7-1 immunogene therapy, as evidenced by the fact that the control tumours of four mice slowly regressed for one week, but then regrew. However, the tumour of one mouse regressed and disappeared altogether over a period of 4 weeks following injection of B7-1 plasmid (Figure 2).

The tumours of mice fed milk fat regressed more quickly than those of controls, taking only 2 weeks to disappear after injection of the B7-1 plasmid. In contrast, the tumours of mice fed enriched milk fat resisted B7-1 immunogene therapy, and grew at a rate similar to the tumours of mice fed the control diet. Thus, milk fat augments cancer immunotherapy, whereas enriched milk fat is less effective.

### EXAMPLE 4

This example shows that milk fat synergizes with iron-saturated lactoferrin to completely inhibit tumorigenesis.

The applicant's previous studies have shown that iron-saturated Lf (Lf+) is able to inhibit tumour growth (WO/2006/054908). This experiment investigates the effect of coadministration of milk fat and Lf+. We sought to determine whether milk fat might synergize with Lf+ in combating lymphoma.

Groups of six mice were fed a control AIN-93G diet, diets containing either 28 g Lf+ or 120 g of milk fat (AMF) per 2.4 Kg of diet, or a diet containing a combination of both Lf and milk fat. Day 0 refers to the day the mice were placed on their diets. After 2 weeks on the diets, 2 x 10⁵ EL-4 cells were injected into the flanks of mice. Tumour size as measured by two perpendicular diameters (in centimetres) was monitored until day 56. Each point represents the mean tumour size with 95% confidence intervals for either 6 mice, or the number of mice indicated.

The appearance of tumours was delayed by one week in 5 of 6 mice fed Lf+, and in all 6 mice fed the milk fat diet (Figure 3A). One of 6 mice fed Lf+ completely rejected the tumour challenge. In contrast, all six mice fed the combination of Lf+ and milk fat completely rejected the tumour challenge, indicating that milk fat synergizes with Lf+ to eradicate lymphomas.

### EXAMPLE 5

This example shows that milk fat augments the ability of Lf+ to enhance anti-tumour leukocyte cytotoxicity and tumour apoptosis.

Splenocytes were harvested from the mice described in Example 4 at day 56 and tested for their cytolytic activity against EL-4 target cells. The anti-tumour cytolytic activity of splenocytes obtained from mice fed either Lf+ or milk fat was significantly increased by 66% (P < 0.001) and 61% (P < 0.01), respectively, compared with that of mice fed the control diet (Figure 3B). The anti-tumour cytolytic activity was further increased by 86% (P < 0.001) for mice fed the combination of Lf+ and milk fat compared to controls.

Sections were prepared from tumours from mice described in Example 4 at day 56, and were stained by the terminal deoxynucleotidyltransferase-mediated deoxyuridine triphosphate-digoxigenin nick end labeling (TUNEL) method, and also by the annexin-V-fluos method. The number of apoptotic cells detected by TUNEL or annexin-V-fluos staining of tumour sections was determined for 10 randomly selected fields viewed at x40 magnification. The apoptotic index (A/I) is the number of apoptotic (TUNEL or annexin-V-fluos positive) cells x (100/total number of cells). Milk fat and Lf+ stimulated tumour apoptosis by 73% and 68%, respectively, compared to the control diet (Figure 3C). In accord with its increased anti-tumour cytolytic activity, the combination of milk fat and Lf+ increased tumour apoptosis by 84%.

### EXAMPLE 6

This example shows that milk fat inhibits tumour angiogenesis.

The effects of Lf+ and milk fat on tumour blood flow and vascularity were analyzed by staining of tumour sections prepared as described in Example 5 with anti-CD31 and anti-CD105 mAbs, and by perfusion of DiO7, and respectively. Tumour sections were prepared at day 56 from mice treated as described in Example 4, and stained with either the anti-CD31 mAb MEC13.3 or an anti-CD105 mAb to visualize blood vessels, or alternatively DiO7 was injected into the tail vein one minute prior to collecting tissues in order to visualize blood flow. Stained blood vessels were counted from six mice in six blindly chosen random fields.

As shown in Figure 3D, the number of CD31+ vessels in the tumours of mice fed Lf+, milk fat, or the combination of milk fat and Lf+ was significantly reduced by 84% (P < 0.001), 72% (P < 0.001), and 84% (P < 0.01) respectively, and the blood flow was reduced by 84% (P < 0.001), 68% (P < 0.001), and 84% (P < 0.001) respectively, compared to that of mice maintained on the control diet. Similar results were obtained from staining for CD105+ vessels, and perfusion of DiO7 as other markers of tumour vascularity. These results indicate that milk fat inhibits angiogenesis.

### EXAMPLE 7

This example shows that alpha lipid powder (Phospholac 600^{™}), sphingomyelin, and 9,11 CLA isomer inhibit the growth of tumours, but do not synergize effectively with Lf+ to eradicate tumours.

Groups of five C57BL/6 mice were fed the base AIN-93 diet (Figure 4A), or the same diet substituted with 28 g of Lf+ (Figure 4B),120 g of phospholac 600^{™} (Figure 4C), or 1.2 g of sphingomyelin (Figure 4D) (representing ~71 and 0.71% of the fat component of the diet, respectively) per 2.4 Kg of diet. Mice were challenged subcutaneously with EL-4 tumour cells two weeks after being placed on the diets. Tumour size was again measured by two perpendicular diameters (in centimetres).

Lf+ delayed tumour growth, with the growth of two tumours being delayed by two weeks, and one by at least one week. Phospholac 600^{™} completely inhibited tumorigenesis in one mouse and delayed tumorigenesis in another mouse by 24 days, whereas it had no apparent affect on the growth of tumours in the remaining three mice, compared to mice fed the control diet. Sphingomyelin was as effective as Lf+ in that it delayed tumour growth by ~1-wee in two mice, and by ~2-weeks in two other mice, whereas it had no appreciable affect on tumour growth of the remaining mouse. Additional groups of five mice were fed combinations of Lf+ (28 g per 2.4 Kg of diet) and either Phospholac 600^{™} (120 g per 2.4 Kg of diet) (Figure 4E) or sphingomyelin (1.2 g per 2.4 Kg of diet) (Figure 4F). Lf+ in combination with phospholac 600^{™} did not lead to complete tumour eradication (as seen herein with the combination of Lf+ and milk fat). Rather, the overall effect was similar to that observed with phospholac 600^{™} monotherapy.

The combination of Lf+ with sphingomyelin was less effective than the respective monotherapies, such that tumour growth was similar to control mice, suggesting that the two bioactives may antagonize each other (Figure 4F).

Inclusion of the c-9, t-11 CLA isomer in the mouse diet at 5 g per 2.4 Kg of diet, representing 3% of the fat component of the diet, only weakly inhibited the growth of 4T1 tumours, and showed no synergy with Lf+.

### EXAMPLE 8

This example shows that milk fat delays the growth of 4T1 breast cancer tumours, either alone or in combination with chemotherapy.

Groups of 6 Balb/c mice were fed the base AIN-93 diet, or the same diet substituted with 5% milk fat, representing ~71% of the fat component of the diet. Mice were challenged subcutaneously with 2 x 10⁴ 4T1 tumour cells two weeks after being placed on the diets. The mice were monitored for tumour growth, and tumour size was measured every three days.

Tumours were palpable 3 days later in mice fed the milk fat diet compared with mice fed the control diet, and on the 35^{th} day after challenge when the mice were euthanased the tumours formed were on average 21% smaller (P < 0.05) (Figure 5). The chemotherapeutic drug paclitaxel was injected intraperitoneally (30 mg/kg body weight) once tumours reached ~0.5 cm in diameter, causing a 31% (P <0.05) reduction (at day 35) in the size of tumours fed the control diet. At day 35, paclitaxel treatment had reduced the sizes of the tumours of mice fed milk fat by 35% (P < 0.05) and 49% (P < 0.01), respectively, compared with those of paclitaxel-treated mice fed the control diet, and untreated mice fed the control diets.

### EXAMPLE 9

This example shows that milk fat suppresses the outgrowth of 4T1 breast cancer tumours that disseminate to the lung and liver.

*Suppression of lung metastases:* The 4T1 breast cancer cell line is highly metastatic and disseminates to the lung and liver. The lungs of the mice in Example 8 (day 35) were inspected for tumours and micrometastases. The mean numbers of tumours on the lung surface of untreated mice fed the control diet, paclitaxel-treated mice fed the control diet, untreated mice fed milk fat, and paclitaxel-treated mice fed milk fat were 32, 18, 22, and 10, respectively (Figure 6).

Paclitaxel therapy and the feeding of high doses of milk fat significantly (P < 0.01) reduced the numbers of tumours on the lung surface by 44% and 31 %, respectively, compared with untreated mice fed the control diet.

The milk fat diet in combination with paclitaxel therapy resulted in even greater reductions in tumour numbers of 63% (P < 0.001) and 44% (P < 0.05), compared with untreated mice fed the control diet, and paclitaxel treated mice fed the control diet, respectively. In accord, the lung weights of paclitaxel-treated mice fed the control diet, untreated mice fed milk fat, and paclitaxel-treated mice fed milk fat were significantly reduced by 30, 22, and 40%, compared to untreated mice fed the control diet (Table 7).

**Table 7. Body, tumour, organ and tissue weights, and blood cell counts¹**

| | **Tumour-bearing mice** | | | | **Non-tumour-bearing mice** | |
|---|---|---|---|---|---|---|
| | Control diet (n=6) | Control diet + paclitaxel (n=6) | Milk fat diet (n=6) | Milk fat diet + paclitaxel (n=6) | Control diet (n=6) | Milk fat diet (n=6) |
| Body weight (g) | 18.7 ± 2.7² | 17.7 ± 1.5 | 20.3 ± 2.0 | 20.8 ± 1.4⁵ | 20.8 ± 0.98 | 21.3 ± 2.3 |
| Carcass body weight (g) | 18.4 ± 2.8² | 17.6 ± 1.4 | 20.1 ± 1.9⁴ | 20.7 ± 1.3⁵ | -- | -- |
| Tumor (mg) | 263.5 ± 31.7 | 103.4 ± 24.1³ | 143 ± 20.9⁴ | 48.3 ± 11.2⁴ | -- | -- |
| Spleen (mg) | 79.2 ± 9.4 | 72.35 ± 8.2³ | 82.34 ± 7.5⁴ | 80.28 ± 4.9⁵ | 82.6 ± 12.7 | 82.3 ± 9.5 |
| Liver (mg) | 1268.9 ± 86.3 | 1210.1 ± 72.4 | 1230.8 ± 63.7 | 1201.3 ± 82.9⁵ | 1204.5 ± 102.5 | 1221.2 ± 94.3 |
| ung (mg) | 389.8 ± 24.6² | 274.5 ± 30.6³ | 302.1 ± 9.7⁴ | 234.5 ± 25.6'⁵ | 183.4 ± 13.9 | 190.1 ± 17.8 |
| Gastrocnemius muscle (mg) | 96.3 ± 8.6² | 101.2 ± 9.3 | 128.5 ± 7.9⁴ | 129.2 ± 10.8⁵ | 158.7 ± 25.2 | 171.7 ± 12.52 |
| Epididymal adipose tissue(mg) | 26.7 ± 8.2² | 24.3 ± 6.7 | 55.3 ± 7.1⁴ | 58.6 ± 6.0⁵ | 154.4 ± 23.9 | 172.5 ± 31.8² |
| WBC (x 10³/µl) | 4.21 ± 0.57² | 2.37 ± 0.39³ | 5.34 ± 0.76⁴ | 5.48 ± 0.56⁵ | 5.89 ± 0.74 | 6.12 ± 0.93 |
| RBC (x 10⁶/µl) | 6.35 ± 0.62² | 3.21 ± 0.74³ | 8.65 ± 0.93⁴ | 7.36 ± 0.87⁵ | 8.23 ± 0.58 | 8.67 ± 0.62 |

| | | | | | | |
|---|---|---|---|---|---|---|
| ¹Data are expressed as means ± SEM. Statistical significance was determined by the student's *t* test. ²P < 0.05 compared to body weight of non-tumor bearing mice fed the control diet; ^{3,4}P < 0.05 compared to tumour-bearing mice fed the control diet; ⁵P < 0.05 compared to tumour-bearing mice fed the control diet and treated with paclitaxel. | | | | | | |

*Suppression of liver metastases:* Similarly, the livers of the above mice were inspected for tumours and micrometastases. The livers were removed, section, and stained with hematoxylin/eosin, and the numbers of metastatic nodules inside the livers were counted. The mean numbers of metastases for untreated mice fed the control diet, paclitaxel-treated mice fed the control diet, untreated mice fed milk fat, and paclitaxel-treated mice fed milk fat were 108, 59, 74, and 36, respectively (Figure 7). Thus, paclitaxel therapy and the feeding of high doses of milk fat significantly (P < 0.01) reduced the numbers of tumours in the liver by 45% and 32%, respectively, compared with untreated mice fed the control diet.

The milk fat diet in combination with paclitaxel therapy resulted in even greater reductions in tumour numbers of 67% (P < 0.001) and 39% (P < 0.05), compared with untreated mice fed the control diet, and paclitaxel-treated mice fed the control diet, respectively.

### EXAMPLE 10

This example shows that milk fat inhibits tumour angiogenesis.

The above 4T1 primary tumours (Example 8; day 35) of untreated mice fed the control diet, paclitaxel-treated mice fed the control diet, untreated mice fed milk fat, and paclitaxel-treated mice fed milk fat were excised, sectioned, and stained with an anti-CD31 antibody to identify vascular endothelial cells so as to measure tumour angiogenesis. Blood vessels stained with the anti-CD31 mAb were counted in blindly chosen random fields to record mean vessel density (8A), or the median distance to the nearest CD31 mAb-labeled blood vessel from an array point (8B).

Paclitaxel therapy and the feeding of high doses of milk fat significantly (P < 0.05) reduced microvessel density (CD31+ vessels) by 37% and 31%, respectively, compared with untreated mice fed the control diet (Figure 8A). In accord they significantly (P < 0.05) increased the median distance to the nearest CD31+ vessels by 71 and 59%, respectively (Figure 8B).

The milk fat diet in combination with paclitaxel therapy resulted in an even greater reduction in microvessel density by 52% (P < 0.001) and 22% (P < 0.05), compared with untreated mice fed the control diet, and palcitaxel-treated mice fed the control diet, respectively. In accord, the milk fat diet in combination with paclitaxel therapy resulted in even greater increase in the median distance to the nearest CD31+ vessels of 125% (P < 0.001) and 31 % (P < 0.05), compared with untreated mice fed the control diet, and paclitaxel-treated mice fed the control diet, respectively.

### EXAMPLE 11

Cachexia is a serious problem for cancer patients as it physically weakens patients and reduces their response to treatment. Improved nutrition is one avenue for combating cachexia. This experiment investigated whether feeding of milk fat would improve the cachetic status of mice in Applicant's model of breast cancer, and whether long-term feeding of high-dose milk fat which contains hypercholesterolemic saturated fatty acids (eg stearate) would be detrimental to certain organs such as the liver and spleen.This example shows that a high dose milk fat diet displays no apparent organ toxicity, and attenuates cachexia caused by advanced cancer.

*Lack of organ toxicity.* The spleens and livers of mice fed high doses of milk fat showed no obvious signs of toxicity, and there was no significant change in their weight (P > 0.05), compared with those of mice fed the control diet (Table 7).

*Reduction of cachexia* The 4T1 model of metastatic breast cancer represents an ideal model of cancer cachexia. Establishment of tumours resulted in a significant (P < 0.05) 12% reduction in carcass body weight, as reflected by losses in the weights of gastrocnemius muscle and epididymal adipose tissues of 40% (P < 0.05) and 83% (P < 0.01), respectively, compared with non-tumour bearing mice fed the same control diet (Table 7). Reduced cachexia was observed in the mice fed the milk fat diet, as evidenced by the significantly (P < 0.05) increased carcass body weight (9.2 and 17.6%), and weight of gastrocnemius muscle (33.4 and 27.6%) and epididymal adipose tissues (107 and 141%) in untreated mice fed milk fat, and paclitaxel-treated mice fed milk fat, compared with those of untreated mice fed the control diet, and paclitaxel-treated mice fed the control diet, respectively. Increases in the weight of gastrocnemius muscle and epididymal adipose tissues was not confined to tumour-bearing mice, as feeding of milk fat to healthy non-tumour-bearing mice increased the respective weights of the latter tissues by 8.2 and 12% (P < 0.05), compared with those of healthy non-tumour-bearing mice fed the control diet.

### EXAMPLE 12

One of the most severe side-effects of cancer and cancer treatment such as chemotherapy is hematologic suppression, which can lead to immunologic inadequacy, and in turn cause severe infections and even death. This example shows that a milk fat diet attenuates hematologic suppression caused by cancer and chemotherapy.

Dyshematopoiesis was found in the cachectic mice of Example 11 above, as the red blood cell (RBC) and white blood cell (WBC) counts were significantly (P <0.05) reduced by 23 and 29%, respectively; compared with those of healthy non-tumour bearing mice fed with the same control diets (Table 7). Paclitaxel chemotherapy caused a further 49 and 44% reduction in RBC and WBC counts, and a corresponding 8.5% reduction in spleen weights, compared to tumour-bearing mice fed the control diet.

In contrast, RBC and WBC counts of paclitaxel-treated mice fed milk fat were reduced by only 11% and 7%, and spleen weights by 3%, respectively, compared with those of healthy non-tumour bearing mice fed the control diet (Table 7). The WBC counts of untreated tumour-bearing mice fed milk fat were reduced by only 5%, whereas the RBC counts were slightly increased by 9% (P < 0.05), and spleen weights were completely restored. The RBC and WBC counts of paclitaxel-treated mice fed milk fat were significantly (P < 0.01) increased by 56 and 57%, and spleen weights by 11% (P < 0.05), respectively, compared with those of paclitaxel-treated mice fed the control diet (Table 7). The RBC and WBC counts of untreated tumour-bearing mice fed milk fat were increased by 34 and 27% (P < 0.05), compared with those of untreated tumour-bearing mice fed the control diet.

Thus, a milk fat diet attenuates hematologic suppression by increasing or restoring red and white blood cell numbers that are diminished by cancer and by chemotherapy.

### EXAMPLE 13

Cytotoxic drugs damage the intestinal villi, causing them to become flattened, and thereby altering the absorption properties of the gut (Melichar *et al.,* 2005). This example shows that a milk fat diet ameliorates gut damage induced by chemotherapy.

To determine whether milk fat protected the gut against paclitaxel-induced damage, groups of 6 Balb/c mice were fed the base AIN-93 diet, or the same diet substituted with 5% milk fat, representing ~71% of the,fat component of the diet. Two weeks later each mouse received an intraperitoneal injection of paclitaxel (30 mg/kg body weight). One week later they were killed and the jejunum was removed. Paraffin-embedded sections of the jejunum of mice treated with control diet, milk fat diet, control diet plus paclitaxel, and milk fat diet plus paclitaxel were stained with hematoxylin and eosin.

Untreated healthy mice fed the control diet and the milk fat diet had intact intestinal villi as expected. In contrast, the sections of mice treated with paclitaxel revealed that the jejuni of these mice contained broken and flattened villi. The villi of paclitaxel-treated mice fed milk fat were largely intact, suggesting the latter diet exerts a protective effect on the lining of the intestine.

The average length of the villi was measured in order to quantify the degree of gut damage. Paclitaxel significantly reduced the average length of the villi by 61 % (P < 0.01), compared with that of control mice (Figure 9A). The milk fat diet protected against intestinal injury caused by paclitaxel, as the average length of the villi of paclitaxel-treated mice fed the milk fat diet was reduced by 29% (P < 0.05), compared with control mice. The villi of paclitaxel-treated mice fed the milk fat diet were significantly longer (40%, P < 0.05) than those of paclitaxel-treated mice fed the control diet.

The activity of jejunal γ-glutamyl transpeptidase (γ-GGT), a marker of the brush border epithelium of the small intestine (Tate and Meister, 1981 and Ferraris, *et al.,* 1992), was recorded as a separate measure of gut damage. Paclitaxel treatment markedly reduced the level of γ-GGT in the mucosa of the jejunum by 56% (P < 0.01), compared to untreated control mice (Figure 9B), whereas γ-GGT activity in paclitaxel-treated mice fed the milk fat diet was reduced by only 29% (P < 0.05). Jejunal γ-GGT activity was significantly increased (78%, p < 0.05) in paclitaxel-treated mice fed the milk fat diet, compared to that of paclitaxel-treated mice fed the control diet.

Mucositis of the small intestine caused by chemotherapy is associated with cellular apoptosis in the crypts that precedes villous atrophy *(Keefe, et al.,* 2000). There were very few apoptotic cells in the crypts of untreated mice fed the control and milk fat diets. In contrast, the intestinal sections of mice fed the control diet and treated with paclitaxel contained large numbers of apoptotic cells. The milk fat diet markedly reduced the number of apoptotic bodies. Apoptotic cells in 10 randomly selected crypts were counted and expressed as apoptotic bodies per crypt (Figure 10). Paclitaxel treatment significantly increased the apoptotic body count by 6-fold, compared with untreated mice. The milk fat diet significantly (P < 0.05) reduced the apoptotic body count of paclitaxel-treated mice by 39%, compared with the control diet.

### DISCUSSION OF EXAMPLES 1-13

The above experiments show that normal anhydrous milk fat has anti-tumour activity when orally administered. The administration of milk fat augmented immunotherapy of established lymphoma, and augmented chemotherapeutic treatment of established breast cancer tumours.

Milk fat in combination with Lf+ completely inhibited tumourigenesis in all subjects challenged with lymphoma, causing increased anti-tumour cytolytic activity, tumour apoptosis and reductions in tumour vascularity.

The administration of milk fat in combination with chemotherapeutic treatment delayed the growth of breast cancer tumours, while milk fat, either alone or in combination with chemotherapeutic treatment significantly suppressed metastasis of breast cancer tumours to the lung and liver and outgrowth in these organs.

Anti-tumour activity was also observed with the administration of milk fat fractions. Here, both Phospholac 600^{™} and sphingomyelin caused delayed tumourigenesis.

Moreover, the administration of milk fat attenuated hematologic suppression, cachexia and gut damage resulting from breast cancer tumours and/or chemotherapy.

### EXAMPLE 14

This example shows that milk fat significantly inhibits weight loss due to chemotherapy, and can facilitate weight gain.

### Diets

The experimental diets were prepared by Crop & Food Research, Palmerston North, New Zealand using as a base the powdered AIN93G formulation. Casein was used as the protein source, and soybean oil as the lipid source, in the AIN93G diet. The soybean oil was substituted in the experimental diets with anhydrous milk fat (AMF), such that the total lipid content of the diet was unchanged. The diets contained either 100, 35.7, or 7.1 g of AMF per 2 Kg of diet, representing 70, 25, and 5% of the total lipid content (140 g lipid/2Kg) of the diet. Fresh diet was provided biweekly, and mice had free access to food and water throughout the study.

### Experimental model to analyze the side-effects of chemotherapy

Cyclophosphamide was administered in accord with Animal Ethics Approval (University of Auckland). All experiments included 6 mice per treatment group, unless otherwise indicated. Cyclophosphamide, diluted in PBS, was administered i.p. at 300 mg/Kg. Blood was collected by cardiac puncture at the time of autopsy.

Groups of six C57BL/6 mice were fed an AIN93G diet or the same diet substituted with either 5%, 25% or 70% milk fat, and after 4 weeks of feeding were injected intraperitoneally with Cyclophosphamide (300 mg/Kg of body weight) at day 0. The mice were killed 4, 8, and 12 days later, and tissues analyzed for the side-effects of drug treatment, as described below.

There was no significant difference in the body weights of mice at day 0 (i.e., 4 weeks after feeding the four different diets prior to chemotherapy), as shown in Figure 11A. In contrast, just 4 days after chemotherapy, mice fed the control diet had lost 7% of their body weight (Figure 11B). The two highest doses of milk fat significantly reduced the extent of the weight loss at day 4 by 54% (P < 0.001) and 39% (P = 0.001·7) compared with control diet, respectively. The lowest dose of milk fat non-significantly reduced the weight loss by 19% (P = 0.068), compared with control. Mice on the control diet showed little improvement in body weight gain at day 12 after chemotherapy, whereas in contrast the body weights of mice fed the lowest dose of milk fat had retuned almost to normal (P < 0.001), and mice on the two highest doses of milk fat had actually gained weight (Figure 11C).

### EXAMPLE 15

This example shows that milk fat facilitates the recovery of circulating and splenic white blood cells (WBC) after chemotherapy, stimulates the development of colony forming units in the spleen, and promotes reconstitution of the spleen.

Feeding of the different milk fat diets for 4 weeks had no significant effect on the peripheral WBC count prior to chemotherapy. Cyclophosphamide severely reduced the peripheral WBC count at day 4 in the mice in Example 14, by more than 90% irrespective of the diet fed (Figure 12). The WBC count had begun to recover by day 8 in all four groups of mice. Recovery of the WBC count by day12 was significantly (P = 0.0028) increased in the group of mice fed the highest dose of milk fat. For these mice, the WBC count had returned to normal. In contrast, lower doses of milk fat did not significantly facilitate recovery of the WBC count.

Feeding of the different milk fat diets for 4 weeks had no discernible effect on the cellularity of the spleen prior to chemotherapy (Figure 13A). Cyclophosphamide severely reduced the cellularity of the spleen at day 4 in the mice fed the control diet. The 70%, 25%, and 5% milk fat diets attenuated the loss of spleen cellularity by 52%, 41 %, and 25%, respectively, at day 4 compared with control diet. The 70% and 25% milk fat diets attenuated the loss of spleen cellularity by 32%, and 27%, respectively, at day 8, whereas only the 70% milk fat diet influenced spleen cellularity at day 12 (Figure 13A).

Replenishment of cells within the spleen began 4 days after chemotherapy with the development of splenic colony forming units. The 70%, 25%, and 5% milk fat diets each significantly stimulated the generation of splenic colony forming units by 207%, 130%, and 85%, respectively, at day 8 (Figure 13B). By day 12 the cellularity of the spleen had almost returned to normal in mice fed the 70% and 25% milk fat diets, hence not surprisingly the numbers of colony forming units were significantly reduced in the milk fat diets, whereas the spleens of mice fed the control diet were still undergoing repair (Figure 13B).

### EXAMPLE 16

This example shows that milk fat increases the size and haemoglobin content of red blood cells (RBC) following chemotherapy.

Cyclophosphamide reduced the peripheral RBC count at day 8 in the mice in Example 14, irrespective of the diet fed (Figure 14A). The loss of RBC was 17% to 22% at day 4, which was less than the extent of the loss of WBC in Example 15. RBC numbers continued to diminish until day 8, and had not completely recovered at day 12. The milk fat diets had no significant affect on cyclophosphamide-induced reductions in RBC counts. While the higher doses of milk fat appeared to enhance recovery of RBC numbers, the results were not significant.

Surprisingly, the two highest doses of milk fat either prevented the loss of and/or facilitated the recovery of hematocrit (HCT) levels (Figure 14B). Hematocrit is the percent of whole blood that is composed of red blood cells, and is a measure of both the number of RBC and their size. Given that the milk fat diets did not significantly affect RBC numbers, it can be inferred that the increase in HCT levels is a result of an increase in the mean corpuscular volume of the RBC. The 70% milk fat diet significantly (P = 0.023) attenuated the reduction in HCT level at day 8 (18% reduction compared with 31% reduction for the control diet), and increased the HCT level to almost normal levels by day 12 (P < 0.001). The 25% milk fat diet significantly facilitated the recovery of HCT levels at days 8 (P = 0.017) and 12 (P = 0.006), when an 18% reduction at day 8 was observed compared with 31 % reduction for the control diet, and a 9% reduction at day 12 was observed compared with 24% reduction for the control diet. The effects of the lowest dose of milk fat did not reach significance.

A key question was whether an increase in HCT level would also reflect an increase in the overall level of haemoglobin. The 70% milk fat diet attenuated the reduction in hemoglobin at day 8. when a 22% reduction compared with 31% reduction for the control diet was observed, but the change did not reach significance (Figure 14C). However, it significantly increased hemoglobin levels by 18% at day 12 (P = 0.0048), compared to those of mice fed the control diet. The 25% milk fat diet significantly facilitated the recovery of hemoglobin levels at days 8 (P = 0.030) and 12 (P = 0.015), when a 20% reduction at day 8 was observed compared with 31 % reduction for the control diet, and a 12% reduction at day 12 was observed compared with 26% reduction for the control diet. The effects of the lowest dose of milk fat did not reach significance.

In summary, the results indicate that while ingestion of milk fat does not significantly influence RBC numbers, it increases RBC size and haemoglobin content and may thereby have a potential beneficial effect on anemia. These results have important implications for the treatment of iron-deficiency anemia, an example of microcytic anemia typified by abnormally small RBC.

### EXAMPLE 17

As described above, mucositis occurs when cancer chemotherapy destroys the rapidly dividing epithelial cells lining the gastrointestinal tract, leaving the mucosal tissue open to ulceration and infection. This example shows that milk fat attenuates gut damage due to chemotherapy.

To determine whether milk fat protected the gut against cyclophosphamide-induced damage, the jejuni of mice in Example 14 were sectioned, paraffin-embedded, and stained with hematoxylin and eosin.

The feeding of the different milk fat diets for 4 weeks had no discernible effect on the villi of the jejunum prior to chemotherapy (Figure 15). The sections of mice treated with a single injection of cyclophosphamide revealed that the jejuni of these mice contained broken and flattened villi. The average length of the villi was used a measure of chemotherapy-induced damage. Cyclophosphamide significantly reduced the average length of the villi by 65% (P < 0.001), compared with that of healthy control mice (Figure 15). The 70%, 25%, and 5% milk fat diets protected against intestinal injury caused by cyclophosphamide, as the average length of the villi of cyclophosphamide-treated mice fed the milk fat diets was reduced by 36% (P < 0.01), 41% (P < 0.01), and 52% (P < 0.001), respectively, at day 4, and by 21% (P < 0.05), 24% (P < 0.01), and 31% (P < 0.01), respectively, at day 8 compared with mice fed the control diet. The villi of cyclophosphamide-treated mice fed the 70% and 25% milk fat diets were significantly longer at 4 days (71% and 58%, respectively, both P < 0.01), and at 8 days (44% and 36%, respectively, both P <0.05) after chemotherapy than those of cyclophosphamide-treated mice fed the control diet. By day 12 the intestine of mice fed the control diet had begun to recover, and only the highest dose of milk fat was seen to retain a significant effect.

The results suggest that the milk fat diets exert a protective effect on the lining of the intestine, particularly during the first few days following chemotherapy when the effects of treatment are most severe. Without wishing to be bound by any theory, protection of the intestine by the milk fat diets is believed to at least in part contribute to the reduced weight loss due to chemotherapy and to the increased weight gain observed in mice fed these diets. A milk fat diet appears to help maintain appetite, and the absorptive properties of the gut. The increased weight gain was not simply due to the higher calorific value of milk fat, as the diets were balanced for energy content and the milk fat-fed mice did not put on weight during the four week feeding period prior to chemotherapy.

### DISCUSSION OF EXAMPLES 14-17

Examples 1-13 above examined the affects of milk fat on the side-effects of chemotherapy in tumour-bearing mice. In examples 14-17, the direct effects of milk fat on the side-effects of cyclophosphamide-mediated chemotherapy in otherwise normal healthy mice were examined. Cyclophosphamide is a drug that strongly inhibits cell metabolism and reproduction, hence as a side-effect it inhibits the growth and multiplication of the cells of the immune and blood system causing myelosuppression and anemia. Studies report that chemotherapy-induced anemia, including mild-to-moderate anemia, has an adverse impact on quality of life of cancer patients (Groopman JE, Itri LM. Chemotherapy-induced anemia in adults: incidence and treatment. J Natl Cancer Inst. 1999 Oct 6;91(19):1616-34). Chemotherapy can also reduce the quality of life of cancer patients by causing weight loss as a result of poor appetite, mucositis, nausea, gut damage, and dehydration.

The results obtained herein indicate that milk fat is effective in preventing or attenuating the side-effects of chemotherapy.

### EXAMPLE 18

This example shows that milk fat synergizes with Lf+ to reduce the multiple side-effects of chemotherapy in a subject including hematological suppression, anemia, and damage to the gut.

### Diets

The experimental diets were prepared by Crop & Food Research, Palmerston North, New Zealand using as a base the powdered AIN93G formulation. Casein was used as the protein source, and soybean oil as the lipid source, in the AIN93G diet. The soybean oil was substituted in the experimental diets with anhydrous milk fat (AMF), such that the total lipid content of the diet was unchanged. The casein was substituted in the experimental diets with Lf+, such that the total protein content of the diet was unchanged. The diets substituted with AMF contained 35 g of AMF per 2 Kg of diet, representing 25% of the total lipid content (140 g lipid/2 Kg) of the diet. The diets substituted with Lf+ contained either 0.1, 1, or 10 g of Lf+ per 2 Kg of diet, representing 0.025, 0.25, and 2.5% of the total protein content (400 g protein/2 Kg) of the diet. Some diets contained a combination of 35 g of AMF per 2 Kg of diet and one of the above amounts of Lf+. Fresh diet was provided biweekly, and mice had free access to food and water throughout the study.

### Experimental model to analyze the side-effects of chemotherapy

Cyclophosphamide was administered in accord with Animal Ethics Approval (University of Auckland). All experiments included 24 mice per treatment group. Cyclophosphamide, diluted in PBS, was administered i.p. at 300 mg/Kg. For blood collection, mice were deeply anesthetized and exsanguinated by cardiac puncture.

Groups of 24 C57BL/6 mice containing an equal number of female and male mice were fed an AIN93G diet or the same diet substituted with milk fat, Lf+, or a combination of milk fat and Lf+, and after 4 weeks of feeding were injected intraperitoneally with cyclophosphamide (300 mg/Kg of body weight) at day 0. The mice were killed in groups of six at day 0, and 4, 8, and 12 days later, and blood and gut tissues analyzed for the side-effects of drug treatment, as described below.

Average villi length on day 8 was analyzed using two-way analysis of variance (ANOVA) for the effects of milk fat supplementation (0 vs. 25%), Lf+ supplementation (0 vs. 0.025%) and their interaction. Individual groups were compared using the Tukey's multiple comparison procedure..

Milk fat synergizes with Lf+ to inhibit chemotherapy-induced gut damage. The feeding of the different milk fat diets for 4 weeks had no discernible effect on the length of the villi of the jejunum prior to chemotherapy (Figure 16). The average length of the villi was used as a measure of chemotherapy-induced damage. Cyclophosphamide significantly reduced the average length of the villi in al 11 groups by day 4 (Figure 16). The 25% milk fat facilitated recovery from intestinal injury caused by cyclophosphamide. On day 8, the average villi length in the milk fat group was significantly greater (P<0.001) than in the control group

The combination of milk fat and Lf+ resulted in significantly greater villi length compared to milk fat only and Lf+ only (P<0.01).

### EXAMPLE 19

This example shows that milk fat synergizes with Lf+ to facilitate the recovery of circulating white blood cells (WBC) after chemotherapy.

Difference in WBC counts between day 4 and day 8 was analyzed using two-way analysis of covariance (ANCOVA) for the effects of milk fat supplementation (0 vs. 25%), Lf+ supplementation (0 vs. 0.025%) and their interaction. Body weight at the time of chemotherapy was included as a covariate. The combination of milk fat and Lf+ was compared to milk fat only and Lf+ only using a one-sided Dunnett's test.

WBC counts in cardiac puncture samples were recorded on the day of chemotherapy and 4, 8, and 12 days later, as described in Example 18. Feeding of the milk fat diet and the 0.025% Lf+ diet for 4 weeks had no significant effect on the peripheral WBC count prior to chemotherapy (Figure 17). Cyclophosphamide severely reduced the peripheral WBC count at day 4 in all the mice in Example 19. The WBC count had begun to recover by day 8 in all groups of mice. Hence a comparison was made of numbers of WBC at the nadir (Day 4) versus Day 8 when there had been substantial recovery in WBC numbers. The increase in WBC count was significantly greater in mice fed the combination of 0.025% Lf+ and milk fat, compared to the 0.025% Lf+ diet (P = 0.049), and the milk fat diet (P = 0.012).

### EXAMPLE 20

This example shows that milk fat synergizes with Lf+ to increase the number and size of red blood cells (RBC) following chemotherapy, with a trend to increase haemoglobin content.

RBC counts, HCT, and hemoglobin levels were analyzed using two-way ANCOVA for the effects of milk fat supplementation (0 vs. 25%), Lf+ supplementation (0 vs. 0.25%) and their interaction. Body weight at the time of chemotherapy was included as a covariate. The combination of milk fat and Lf+ was compared to milk fat only and Lf+ only using a one-sided Dunnett's test.

RBC counts, HCT, and hemoglobin in cardiac puncture samples were recorded on the day of chemotherapy and 4, 8, and 12 days later, as described in Example 18.

Cyclophosphamide reduced the RBC count at days 4 and 8 in the mice in Example 20, irrespective of the diet fed (Figure 18). RBC numbers continued to diminish until day 8, and then began to recover, but full recovery was not reached at day 12. Milk fat appeared to reduce the nadir at days 4 and 8, and synergized with 0.25% Lf to facilitate recovery of RBC numbers at Day 12. RBC counts on day 12 were significantly greater in mice fed the combination of 0.25% Lf+ and milk fat, compared to the 0.25% Lf+ diet (P = 0.018), and the milk fat diet (P = 0.024).

Cyclophosphamide reduced the HCT at days 4 and 8 in the mice in Example 20, irrespective of the diet fed (Figure 19). HCT continued to diminish until day 8, and then began to recover, but full recovery was not reached at day 12. Milk fat appeared to reduce the nadir at days 4 and 8, and synergized with 0.25% Lf to facilitate recovery of HCT levels at Day 12. HCT on day 12 were significantly greater in mice fed the combination of 0.25% Lf+ and milk fat, compared to the 0.25% Lf+ diet (P = 0.046), and the milk fat diet (P = 0.047).

Cyclophosphamide reduced the hemoglobin levels at days 4 and 8 in the mice in Example 20, irrespective of the diet fed (Figure 20). Hemoglobin continued to diminish until day 8, and then began to recover, but full recovery was not reached at day 12. Milk fat appeared to reduce the nadir at days 4 and 8, and synergized with 0.25% Lf to facilitate recovery of hemoglobin at Day 12. Hemoglobin on day 12 was significantly higher in mice fed the combination of 0.25% Lf+ and milk fat, compared to the 0.25% Lf+ diet (P = 0.038). It was higher but not significantly so compared to the milk fat diet (P = 0.094).

In summary, the results indicate that ingestion of milk fat in combination with 0.25% Lf+ significantly increases RBC numbers, and RBC size, and there is a trend to increase overall haemoglobin content. These results have important implications for the treatment of anemia, including chemotherapy-induced anemia.

### EXAMPLE 21

This example shows that milk fat significantly attenuates chemotherapy-induced anorexia/cachexia. Diets containing a combination of milk fat and Lf+ also attenuate chemotherapy-induced anorexia/cachexia.

Body weight was recorded on the day of chemotherapy and 4, 8, and 12 days later, as described in Example 18. Milk fat significantly (P = 0.004) increased the body weights of mice, independently of gender, Lf+ supplementation, and day - i.e. due to the lack of any significant interactions, animals of both genders, all days and all Lf groups were pooled for analysis (Figure 21). The effects of milk fat were specific to chemotherapy, as neither the milk fat diet, nor the other diets, had any significant effect on the body weights of the mice prior to chemotherapy.

### INDUSTRIAL APPLICATION

The methods, medicinal uses and compositions of the present invention have utility in inhibiting tumour growth, maintaining or improving one or more of the white blood cell count, the red blood cell count, or the myeloid cell count, stimulating the immune system and in treating or preventing cancer. The methods and medicinal uses may be carried out by employing dietary (as foods or food supplements), nutraceutical or pharmaceutical compositions. ,

Those persons skilled in the art will understand that the above description is provided by way of illustration only and that the invention is not limited thereto.

### REFERENCES

Ainscough, EW, Brodie A, M.; Plowman J E. The chromium, manganese, cobalt and copper complexes of human lactoferrin. Inorganica Chimica Acta 1979,33 (2) 149-53.
Andrade LN, de Lima TM, Curi R, Castrucci AM. Toxicity of fatty acids on murine and human melanoma cell lines. Toxicol In Vitro. 2005; 19: 553-60.
Baker EN, Baker HM, Kidd RD., Lactoferrin and transferrin: functional variations on a common structural framework. Biochem Cell Biol. 2002;80(1):27-34.
Banni S, Angioni E, Murru E, Carta G, Melis MP, Bauman D, Dong Y, Ip C. Vaccenic acid feeding increases tissue levels of conjugated linoleic acid and suppresses development of premalignant lesions in rat mammary gland. Nutr Cancer. 2001;41:91-7.
Bates GW and Schlabach MR (1973). The reaction of Ferric salts with Transferrin. J Biol. Chem 248, 3228-3232.
Bates GW, Billups C, Saltman P, (1967). The kinetics and mechanism of iron exchange between chelates and transferrin. 1. The complexes of citrate and nitrilotriacetic acid. J. Biol. Chem 242, 2810-2815.
Beer TM, Eilers KM, Garzotto M, Egorin MJ, Lowe BA, Henner WD. Weekly high-dose calcitriol and docetaxel in metastatic androgen independent prostate cancer. J Clin Oncol. 2003;21:123-8.
Beer TM, Munar M, Henner WD. A Phase I trial of pulse calcitriol in patients with refractory malignancies: pulse dosing permits substantial dose escalation. Cancer. 2001;91:2431-9.
Beer TM, Myrthue A. Calcitriol in cancer treatment: from the lab to the clinic. Mol Cancer Ther. 2004;3:373-81.
Belobrajdic DP, McIntosh GH. Dietary butyrate inhibits NMU-induced mammary cancer in rats. Nutr Cancer. 2000;36:217-23.
Bestak R, Halliday GM. Sunscreens protect from UV-promoted squamous cell carcinoma in mice chronically irradiated with doses of UV radiation insufficient to cause edema. Photochem Photobiol. 64:188-93, 1996.
Bougnoux P, Chajes V, Lanson M, Hacene K, Body G, Couet C, Le Floch O. Prognostic significance of tumor phosphatidylcholine stearic acid level in breast carcinoma. Breast Cancer Res Treat. 1992; 20: 185-94.
Bowie JU, Reidhaar-Olson JF, Lim WA, Sauer RT. Deciphering the message in protein sequences: tolerance to amino acid substitutions. Science. (1990) 247(4948): 1306-10.
Bradlow HL, Sepkovic DW. Diet and breast cancer. Ann N Y Acad Sci. 2002; 963: 247-67.
Brock JH; Arzabe FR (1976), Cleavage of diferric bovine transferrin into two monoferric fragments. FEBS Lett 69, 63-66.
Brock JH. The physiology of lactoferrin. Biochem Cell Biol 2002;80:1-6.
Buckman DK, Chapkin RS, Erickson KL. Modulation of mouse mammary tumor growth and linoleate enhanced metastasis by oleate. J Nutr. 1990; 120: 148-57.
Bylund, G. (Ed.) Dairy processing handbook: 1995 Tetra Pak Processing Systems AB, S-221 86 Lund, Sweden.
Caclwell, R.C. and G.F. Joyce. Randomization of genes by PCR mutagenesis. PCR Methods Appl. 1992;2: 28-33.
Calonghi N, Pagnotta E, Parolin C, Tognoli C, Boga C, Masotti L. 9-Hydroxystearic acid interferes with EGF signalling in a human colon adenocarcinoma. Biochem Biophys Res Commun. 2006; 342:585-8. '
Chen BQ, Xue YB, Liu JR, Yang YM, Zheng YM, Wang XL, Liu RH. Inhibition of conjugated linoleic acid on mouse forestomach neoplasia induced by benzo (a) pyrene and chemopreventive mechanisms. World J Gastroenterol. 2003; 9: 44-9.
Cohen LA, Zhao Z, Pittman B, Scimeca J. Effect of soy protein isolate and conjugated linoleic acid on the growth of Dunning R-3327-AT-1 rat prostate tumours. Prostate. 2003; 54: 169-80.
Colomer R, Menendez JA. Mediterranean diet, olive oil and cancer. Clin Transl Oncol. 2006; 8: 15-21.
Colston KW, Pirianov G, Bramm E, Hamberg KJ, Binderup L. Effects of Seocalcitol (EB1089) on nitrosomethyl urea-induced rat mammary tumors. Breast Cancer Res Treat. 2003;80:303-11.
Conneely OM. Antiinflammatory activities of lactoferrin. J Am College Nutr 2001;20:389S-395S.
Corl BA, Barbano DM, Bauman DE, Ip C. cis-9, trans-11 CLA derived endogenously from trans-11 18:1 reduces cancer risk in rats. J Nutr. 2003;133:2893-900.
Dass CR Tumour angiogenesis, vascular biology and enhanced drug delivery. J Drug Target. 2004 Jun;12(5):245-55
Ding I, Sun JZ, Fenton B et al. Intratumoral administration of endostatin plasmid inhibits vascular growth and perfusion in MCa-4 murine mammary carcinomas. Cancer Res. 2001; 61: 526-531.
Ealey KN, el-Sohemy A, Archer MC. Conjugated linoleic acid does not inhibit development of aberrant crypt foci in colons of male Sprague-Dawley rats. Nutr Cancer. 2001; 41: 104-6.
Facon MJ, Skura BJ. Antibacterial activity of lactoferricin, lysozyme and EDTA against Salmonella enteritidis. Int.Dairy J. 1996, 6 (3) 303-13.
Fermor BF, Masters JR, Wood CB, Miller J, Apostolov K, Habib NA. Fatty acid composition of normal and malignant cells and cytotoxicity of stearic, oleic and sterculic acids in vitro. Eur J Cancer. 1992; 28A: 1143-7.
Ferraris RP, Villenas SA, Diamond J.Regulation of. brush-border enzyme activities and enterocyte migration rates in mouse small intestine. Am J Physiol. 1992; 262: G1047-G1059.
Foley AA, Bates GW. The purification of lactoferrin from human whey to batch extraction Analytica 1Biochemistry 1987, 162 (1) 296-300.
Freireich EJ, Gehan EA, Rall DP, Schmidt LH, Skipper HE., Quantitative comparison of toxicity of anticancer agents in mouse, rat, hamster, dog, monkey, and man. Cancer Chemother Rep. (1966) 50(4):219-44.
Gaard M, Tretli S, Loken EB. Dietary fat and the risk of breast cancer: a prospective study of 25,892 Norwegian women. Int J Cancer. 1995; 63: 13-7.
Galdiero F, Carratelli CR, Nuzzo I, Bentivoglio C, De Martino L, Gorga F, Folgore A, Galdiero M. Beneficial effects of myristic, stearic or oleic acid as part of liposomes on experimental infection and antitumor effect in a murine model. Life Sci. 1994; 55: 499-509.
Goldbohm RA. Intake of conjugated linoleic acid, fat, and other fatty acids in
Goodman MT, Wu AH, Tung KH, McDuffie K, Kolonel LN, Nomura AM, Terada K, Wilkens LR, Murphy S, Hankin JH. Association of dairy products, lactose, and calcium with the risk of ovarian cancer. Am J Epidemiol. 2002; 156: 148-57.
Gulaia NM, Smirnov IM, Shmal'ko IuP, Mel'nik AA, Mel'nik SN. Effect of N-palmitoyl- and N-stearoylethanolamines on lipid peroxidation in mouse tissues in metastatic Lewis carcinoma. Ukr Biokhim Zh. 1993; 65:96-101.
Guyton KZ, Kensler TW, Posner GH. Cancer chemoprevention using natural vitamin D and synthetic analogs. Annu Rev Pharmacol Toxicol. 2001;41:421-42.
Guyton KZ, Kensler TW, Posner GH. Vitamin D and vitamin D analogs as cancer chemopreventive agents. Nutr Rev. 2003; 61:227-38.
Ha YL, Grimm NK, Pariza MW. Anticarcinogens from fried ground beef: heat-altered derivatives of linoleic acid. Carcinogenesis. 1987; 8:1881-7.
Harada H, Yamashita U, Kurihara H, Fukushi E, Kawabata J, Kamei Y. Antitumor activity of palmitic acid found as a selective cytotoxic substance in a marine red alga. Anticancer Res. 2002; 22: 2587-90.
Harfoot CG and Hazlewood GP. "Lipid metabolism in the rumen" in P.N. Hobson (Ed.) "The Rumen Microbial Ecosystem" at pages 285 to 322, Elsevier Applied Science Publishers, London (1988).
Harris DM, Go VL. Vitamin D and colon carcinogenesis. J Nutr. 2004;134:3463S-3471S.
Hjartaker A, Laake P, Lund E. Childhood and adult milk consumption and risk of premenopausal breast cancer in a cohort of 48,844 women - the Norwegian women and cancer study. Int J Cancer. 2001; 93: 888-93.
Holick MF. Vitamin D: importance in the prevention of cancers, type 1 diabetes, heart disease, and osteoporosis. Am J Clin Nutr. 2004:79:362-71.
Hooper CW, Robscheit FS, Whipple GH. Am J Physiol. Blood regeneration following simple anemia: III. Influence of bread and milk, crackermeal, rice and potato, casein and gliadin in varying amounts and combinations. 1920; 53,206-35.
Inaba K, Inaba M, Romani N, Aya H, Deguchi M, Ikehara S, Muramatsu S, Steinman RM. Generation of large numbers of dendritic cells from mouse bone marrow cultures supplemented with granulocyte/macrophage colony-stimulating factor. J Exp Med. 1992; 176:1693-1702.
Jung KC, Park CH, Hwang YH, Rhee HS, Lee JH, Kim HK, Yang CH. Fatty acids, inhibitors for the DNA binding of c-Myc/Max dimer, suppress proliferation and induce apoptosis of differentiated HL-60 human leukemia cell. Leukemia. 2006; 20: 122-7.
Kanwar J, Berg R, Lehnert K, and Krissansen GW. Taking lessons from dendritic cells: Multiple xenogeneic ligands for leukocyte integrins have the potential to stimulate anti-tumour immunity. Gene Therapy 1999;6:1835-44.
Kanwar JR, Kanwar R, Pandey S, Ching L-M, and Krissansen G.W. Vascular attack by 5,6-dimethylxanthenone-4-acetic acid combined with B7.1-mediated immunotherapy overcomes immune-resistance and leads to the eradication of large tumors. Cancer Res 2000;61:1948-56.
Kanwar JR, Shen WP, Kanwar RK, Berg RW, Krissansen GW. Effects of survivin antagonists on growth of established tumors and B7-1 immunogene therapy. J Natl Cancer Inst. 2001;93:1541-52.
Karthikeyan S, Sujata Sharma, Ashwani K. Sharma, M. Paramasivam, Savita Yadav, A. Srinivasan and Tej P. Singh, Structural variability and functional convergence in lactoferrins, Current Science (1999) 77(2):241
Kawakami H et al. Effect of lactoferrin on iron solubility under neutral conditions. BiosScience Biotech Biochem 1993; 57(8) 1376-1377.
Keefe DMK, Brealey J, Goland GJ, Cummins AG. Chemotherapy for cancer causes apoptosis that precedes hypoplasia in crypts of the small intestine in humans. Gut 2000; 47: 632-637.
Kerwin SM. Soy saponins and the anticancer effects of soybeans and soy-based foods. Curr Med Chem Anti-Canc Agents. 2004;4:263-72.
Kim KH, Park HS. Dietary supplementation of conjugated linoleic acid reduces colon tumour incidence in DMH-treated rats by increasing apoptosis with modulation of biomarkers. Nutrition. 2003; 19: 772-7.
Kimmerlin T, Seebach D. 100 years of peptide synthesis: ligation methods for peptide and protein synthesis with applications to beta-peptide assemblies. J Pept Res. 2005 Feb;65(2):229-60.
Kimura Y. Carp oil or oleic acid, but not linoleic acid or linolenic acid, inhibits tumor growth and metastasis in Lewis lung carcinoma-bearing mice. J Nutr. 2002; 132: 2069-75.
Knekt P, Jarvinen R, Seppanen R, Pukkala E, Aromaa A. Intake of dairy products and the risk of breast cancer. Br J Cancer. 1996; 73: 687-91.
Kris-Etherton P. Bioactive compounds in foods: Their role in prevention of cardiovascular disease and cancer. Americal Journal of Medicine 113: 71 S-88S, 2002.
Kristiansen E, Madsen C, Meyer O, Roswall K, Thorup I. Effects of high-fat diet on incidence of spontaneous tumors in Wistar rats. Nutr Cancer. 1993; 19: 99-110.
Larsson SC, Bergkvist L, Wolk A. High-fat dairy food and conjugated linoleic acid intakes in relation to colorectal cancer incidence in the Swedish Mammography Cohort. Am J Clin Nutr. 2005; 82: 894-900.
Larsson SC, Orsini N, Wolk A. Milk, milk products and lactose intake and ovarian cancer risk: a meta-analysis of epidemiological studies. Int J Cancer. 2006; 118: 431-41.
Law, B. A. and Reiter, B., The isolation and bacteriostatic properties of lactoferrin from bovine milk whey. J. Dairy Res. (1977) 44:595-599.
Legrand D, Mazurier J, Metz-Boutigue M-H, Jolles J, Jo;;es P, Montreuil J & Spik G (1984). Characterization and localization of an iron-binding 18-kDa glycopeptide isolated from the N-terminal half of human lactotransferrin. Biochimica et Biophysica acta 787, 90-96.
Leung, D.W., Chen, E.Y., Goeddel, D.V. A Method for Random Mutagenesis of a Defined DNA Segment Using a Modified Polymerase Chain Reaction. Technique 1989; 1:11-15.
Ma J, Giovannucci E, Pollak M, Chan JM, Gaziano JM, Willett W, Stampfer MJ. Milk intake, circulating levels of insulin-like growth factor-I, and risk of colorectal cancer in men. J Natl Cancer Inst. 2001; 93: 1330-6.
Masson PL, Heremans JF. Studies on lactoferrin, the iron-binding protein of secretions. Protides of the Biological Fluids 1966,14,115-24.
Masso-Welch PA, Zangani D, Ip C, Vaughan MM, Shoemaker S, Ramirez RA, Ip MM. Inhibition of angiogenesis by the cancer chemopreventive agent conjugated linoleic acid. Cancer Res. 2002;62:4383-9.
Mata L, Castillo H, Sanchez L, Puyol P, Calvo M. Effect of trypsin on bovine lactoferrin and interaction between the fragments under different conditions. J Dairy Res. (1994) 61(3):427-32.
Matsubara K, Saito A, Tanaka A, Nakajima N, Akagi R, Mori M, Mizushina Y. Catechin conjugated with fatty acid inhibits DNA polymerase and angiogenesis. DNA Cell Biol. 2006; 25: 95-103.
Melichar B, Dvorak J, Hyspler R, Zadak Z. Intestinal permeability in the assessment of intestinal toxicity of cytotoxic agents. Chemotherapy. 2005; 51: 336-8.
Menendez JA, Vellon L, Colomer R, Lupu R. Oleic acid, the main monounsaturated fatty acid of olive oil, suppresses Her-2/neu (erbB-2) expression and synergistically enhances the growth inhibitory effects of trastuzumab (Herceptin) in breast cancer cells with Her-2/neu oncogene amplification. Ann Oncol. 2005; 16: 359-71.
Metz-Boutigue MH, Jolles J, Mazurier J, Schoentgen F, Legrand D, Spik G, Montreuil J, Jolles P. Human lactotransferrin: amino acid sequence and structural comparisons with other transferrins. EurJBiochem. 1984;145(3):659-76.
Miller A, Stanton C, Murphy J, Devery R. Conjugated linoleic acid (CLA)-enriched milk fat inhibits growth and modulates CLA-responsive biomarkers in MCF-7 and SW480 human cancer cell lines. Br J Nutr. 2003; 90: 877-85.
Moore SA, Anderson BF, Groom CR, Haridas M, Baker EN. Three-dimensional structure of diferric bovine lactoferrin at 2.8 A resolution. J Mol Biol. 1997 Nov 28;274(2):222-36.
Moorman PG, Terry PD. Consumption of dairy products and the risk of breast cancer: a review of the literature. Am J Clin Nutr. 2004; 80: 5-14.
Nakagawa K, Sasaki Y, Kato S, Kubodera N, Okano T. 22-Oxa-1 alpha,25-dihydroxyvitamin D3 inhibits metastasis and angiogenesis in lung cancer. Carcinogenesis. 2005 ;26:1044-54.
Nelson RL, Samelson SL. Inability of the mutagen-blocking agent oleic acid to protect against colon carcinogenesis in the rat. Mutat Res. 1984; 140(2-3): 155-7.
Nguyen LT, Schibli DJ, Vogel J. Structural studies and model membrane interactions of two peptides derived from bovine lactoferricin. Journal ofPeptide Science 2005, 11 (7) 379-89.
Nolan E, Donepudi M, Van Weelden K, Flanagan L, Welsh JE. Dissociation of vitamin D3 and anti-estrogen mediated growth regulation in MCF-7 breast cancer cells. Mol Cell Biochem 1998;188:13-20.
Norris GE, Baker HM, Baker EN. Preliminary crystallographic studies on human apo-lactoferrin in its native and deglycosylated forms. J Mol Biol. 1989; 209(2):329-31.
O'Shea M, Devery R, Lawless F, Murphy J, Stanton C. Milk fat conjugated linoleic acid (CLA) inhibits growth of human mammary MCF-7 cancer cells. Anticancer Res. 2000; 20: 3591-601.
Park EJ and Pezzuto JM. Botanicals in cancer prevention. Cancer and Metastasis Reviews 21: 231-255, 2002.
Parodi PW. Conjugated linoleic acid and other anticarcinogenic agents of bovine milk fat. J Dairy Sci. 1999; 82: 1339-49.
Parodi PW. Cows' milk fat components as potential anticarcinogenic agents. J Nutr. 1997; 127: 1055-60.
Parodi PW. Dairy product consumption and the risk of breast cancer. J Am Coll Nutr. 2005; 24(6 Suppl): 556S-68S.
Peleg S, Posner GH. Vitamin D analogs as modulators of vitamin D receptor action. Curr Top Med Chem. 2003;3:1555-72.
Pierce A, Colavizza D, Benaissa M, Maes P, Tartar A, Montreuil J, Spik G. Molecular cloning and sequence analysis of bovine lactotransferrin. Eur J Biochem. 1991; 196(1):177-84.
Qin LQ, Xu JY, Wang PY, Ganmaa D, Li J, Wang J, Kaneko T, Hoshi K, Shirai T, Sato A. Low-fat milk promotes the development of 7,12 dimethylbenz(A)anthracene (DMBA)-induced mammary tumours in rats. Int J Cancer. 2004; 110: 491-6.
Sambrook, J.; Fritsch, E.F.; Maniatis, T. (1989). Molecular Cloning: A Laboratory Manual. Cold Spring Harbour Lab Press, Cold Spring Harbour, New York.
Saul AW. Vitamin D: Deficiency, diversity and dosage. J Orthomolecular Med. 2003;18:194-204.
Schulz M, Lahmann PH, Riboli E, Boeing H. Dietary determinants of epithelial ovarian cancer: a review of the epidemiologic literature. Nutr Cancer. 2004; 50: 120-40.
Shin MH, Holmes MD, Hankinson SE, Wu K, Colditz GA, Willett WC. Intake of dairy products, calcium, and vitamin d and risk of breast cancer. J Natl Cancer Inst. 2002; 94: 1301-11.
Siegel I, Liu TL, Yaghoubzadeh E, Keskey TS, Gleicher N. Cytotoxic effects of free fatty acids on ascites tumor cells. J Natl Cancer Inst. 1987; 78:271-7.
Steinman RM, Turley S, Mellman I, Inaba K. The induction of tolerance by dendritic cells that have captured apoptotic cells. J Exp Med. 2000 7;191:411-416.
Stemmer WP. DNA shuffling by random fragmentation and reassembly: in vitro recombination for molecular evolution. Proc Natl Acad Sci USA 1994;91:10747-10751.
Sun, X., Kanwar, J.R., Leung, E., Lehnert, K., Wang, D., and Krissansen, G.W. Gene transfer of antisense hypoxia inducible factor-1α enhances the therapeutic efficacy of cancer immunotherapy. Gene Therapy 8: 638-645, 2001.
Superti F, Siciliano R, Rega B, Giansanti F, Valenti P, Antonini G (2001) Involvement of bovine laetoferrin metal saturation, sialic acid and protein fragments in the inhibition of rotavirus infection. Biochim Biophya Acta 1528 (2-3) 107-15.
Suzuki I, Iigo M, Ishikawa C, Kuhara T, Asamoto M, Kunimoto T, Moore MA, Yazawa K, Araki E, Tsuda H. Inhibitory effects of oleic and docosahexaenoic acids on lung metastasis by colon-carcinoma-26 cells are associated with reduced matrix metalloproteinase-2 and -9 activities. Int J Cancer. 1997; 73: 607-12.
Tate SS, Meister A. Gamma-glutamyl transpeptidase: catalytic, structural and functional aspects. Mol Cell Biochem. 1981; 39: 357-368.
Tomita M, Bellamy W, Takase M, Yamauchi K, Wakabayashi H,Kawasi K. Potent antibacterial peptides generated by pepsin digestion of bovine lactoferrin. J. Dairy Science 1991, 74 (12) 4137-4
Tomita M, Takase M, Bellamy W, Shimamura S. A review: the active peptide of lactoferrin. Acta Paediatr. Jpn. 1994, 36, 585-91.
Tomita M, Takase, M, Wakabayashi H & Bellamy W (1994) Antimicrobial Peptides of Lactoferrin in Lactoferrin Structure and Function, pp209-218. Eds TW Hutchens, SV Rumball, B Lonnerdal, Plenum Press, New York.
Tomita M, Wakabayashi H, Yamauchi K, Teraguchi S, Hayasawa H; Bovine lactoferrin and lactoferricin derived from milk: production and applications. Biochemistry and Cell Biology 2002, 80(1) 109-12.
Tseng M, Breslow RA, Graubard BI, Ziegler RG. Dairy, calcium, and vitamin D intakes and prostate cancer risk in the National Health and Nutrition Examination Epidemiologic Follow-up Study cohort. Am J Clin Nutr. 2005; 81: 1147-54.
Tsuda H, Sekine K, Fujita K, Iigo M. Cancer prevention by bovine lactoferrin and underlying mechanisms-a review of experimental and clinical studies. Biochem Cell Biol 2002;131-36.
Tsuji S, Hirata Y, Matsuoka K. Two apparent molecular forms of bovine lactoferrin. J Dairy Sci. 1989;72(5):1130-6.
van der Kraan MIA, Groenink J, Nazmi K, Veerman ECI,. Bolscher JGM, Nieuw Amerongen AV. Lactoferrampin: a novel antimicrobial peptide in the N1-domain of bovine lactoferrin. Peptides 2004, 25 (2) 177-83.
Van Duuren BL, Goldschmidt BM. Co-carcinogenic and tumor-promoting agents in tobacco carcinogenesis. J Natl Cancer Inst. 1976; 56: 1237-42.
van Veen HA, Geerts ME, van Berkel PH, Nuijens JH. The role of N-linked glycosylation in the protection of human and bovine lactoferrin against tryptic proteolysis. Eur. J. Biochem. (2004). 271 (4): 678-684:
van Weelden K, Flanagan L, Binderup L, Tenniswood M, Welsh JE. Apoptotic regression of MCF-7 xenografts in nude mice treated with the vitamin D analog EB1089. Endocrinology 1998;13 9:2102-10.
Veierod MB, Laake P, Thelle DS. Dietary fat intake and risk of prostate cancer: a prospective study of 25,708 Norwegian men. Int J Cancer. 1997 27;73(5):634-8.
Viejo-Diaz M, Andrés MT, Pérez-Gil J, Sánchez M, Fierro J F. Potassium Efflux Induced by a New Lactoferrin-Derived Peptide Mimicking the Effect of Native Human Lactoferrin on the Bacterial Cytoplasmic Membrane. Biochemistry (Moscow) 2003, 68 (2) 217 - 27.
Voorrips LE, Brants HA, Kardinaal AF, Hiddink GJ, van den Brandt PA, relation to postmenopausal breast cancer: the Netherlands Cohort Study on Diet and Cancer. Am J Clin Nutr. 2002; 76: 873-82.
Ward PP, Uribe-Luna S, Conneely OM. Lactoferrin and host defense. Biochem Cell Biol 2002;80:95-102.
Watkins SM, Carter LC, Mak J, Tsau J, Yamamoto S, German JB. Butyric acid and tributyrin induce apoptosis in human hepatic tumour cells. J Dairy Res. 1999;66:559-67.
Weinberg ED. Human lactoferrin: a novel therapeutic with broad spectrum potential. Pharm Pharmacol 2001;53:1303-10.
Whipple GH and Robscheit FS. Blood regeneration in severe anemia. Am J Physiol. 1926; 79: 280-88.
Yamaki T, Yano T, Satoh H, Endo T, Matsuyama C, Kumagai H, Miyahara M, Sakurai H, Pokorny J, Shin SJ, Hagiwara K. High oleic acid oil suppresses lung tumorigenesis in mice through the modulation of extracellular signal-regulated kinase cascade. Lipids. 2002; 37: 783-8.
Yamasaki M, Ikeda A, Hirao A, Tanaka Y, Miyazaki Y, Rikimaru T, Shimada M, Sugimachi K, Tachibana H, Yamada K. Effect of dietary conjugated linoleic acid on the in vivo growth of rat hepatoma dRLh-84. Nutr Cancer. 2001; 40: 140-8.
Yee YK, Chintalacharuvu SR, Lu J, Nagpal S. Vitamin D receptor modulators for inflammation and cancer. Mini Rev Med Chem. 2005;5:761-78.
Yoshida, S. and Xiuyn, Ye. Isolation of Lactoperoxidase and Lactoferrins from Bovine Milk Acid Whey by Carboxymethyl Cation Exchange Chromatography. J Dairy Sci. 1991; 74:1439-1444.
Ziotnik Y, Patya M, Vanichkin A, Novogrodsky A. Tyrphostins reduce chemotherapy-induced intestinal injury in mice: assessment by a biochemical assay. Br J Cancer. 2005; 92: 294-297.
Zusman I, Gurevich P, Madar Z, Nyska A, Korol D, Timar B, Zuckerman A. Tumor-promoting and tumor-protective effects of high-fat diets on chemically induced mammary cancer in rats. Anticancer Res. 1997; 17(1A): 349-56.
The invention will now be defined with reference to the following clauses:
1. A method of inhibiting tumour formation, inhibiting tumour growth, inhibiting tumour metastasis or treating or preventing cancer in a subject, the method comprising separate, simultaneous or sequential administration of an effective amount of milk fat or a milk fat analogue and one or more anti-tumour agents to a subject in need thereof.
2. A method of stimulating the immune system of a subject, the method comprising administration of an effective amount of milk fat or a milk fat analogue to a subject in need thereof.
3. The method of clause 2 wherein the administration increases an anti-tumour immune response in the subject.
4. A method of inducing apoptosis in a subject, the method comprising administration of an effective amount of milk fat or a milk fat analogue to a subject in need thereof..
5. The method of clause 4 wherein the apoptosis is of tumour cells.
6. A method of inhibiting angiogenesis in a subject, the method comprising administration of an effective amount of milk fat or a milk fat analogue to a subject in need thereof.
7. The method of clause 6 wherein the angiogenesis is tumour angiogenesis.
8. A method of treating or preventing anemia caused by low hemoglobin or red blood cell levels, cachexia, mucositis, or leukopaenia, or of maintaining or improving one or more of the white blood cell count, the red blood cell count, and the myeloid cell count of a subject, the method comprising administration of an effective amount of milk fat or a milk fat analogue to a subject in need thereof.
9. A method of increasing the responsiveness of a subject to a cancer therapy, of increasing the sensitivity of a tumour in a subject to a cancer therapy, or of speeding the recovery of a subject undergoing cancer therapy, the method comprising administration of an effective amount of milk fat or a milk fat analogue to a subject in need thereof, separately, simultaneously or sequentially with administration of the therapy.
10. The method of any one of clauses 2 to 9 further comprising the separate, simultaneous or sequential administration to the subject of one or more anti-tumour agents.
11. Use of milk fat or a milk fat analogue and optionally one or more anti-tumour agents for the treatment of a subject suffering from or being treated for cancer.
12. The use of clause 11 wherein the use
   (a) inhibits tumour formation or inhibits tumour growth in the subject; or
   (b) increases an anti-tumour immune response in the subject; or
   (c) induces apoptosis in the subject; or
   (d) inhibits angiogenesis in the subject; or
   (e) treats or prevents anemia caused by low hemoglobin or red blood cell levels, cachexia, mucositis, or leukopaema, or maintains or improves one or more of the white blood cell count, the red blood cell count, and the myeloid cell count of the subject; or
   (f) increases the responsiveness of the subject to a cancer therapy, increases the sensitivity of a tumour in the subject to a cancer therapy, or speeds the recovery of the subject.
13. The use of clause 11 or clause 12 wherein the milk fat or a milk fat analogue is administered separately, simultaneously or sequentially with the one or more anti-tumor agents.
14. A composition comprising about 2 grams to about 210 grams of milk fat or a milk fat analogue and from about 0.1 grams to about 210 grams of one or more anti-tumour agents selected from the group comprising lactoferrin, apo-lactoferrin, a lactoferrin polypeptide, a functional lactoferrin variant, a functional lactoferrin fragment, metal ion lactoferrin, naturally iron-saturated lactoferrin, substantially fully iron-saturated lactoferrin, a metal ion lactoferrin functional variant, a metal ion lactoferrin functional fragment, or a mixture thereof.
15. A composition of clause 14 comprising
   (a) about 2 grams to about 210 grams of milk fat or a milk fat analogue and from about 0.7 grams to about 70 grams of the one or more anti-tumour agents;
   (b) about 3 5 grams to about 210 grams of milk fat or a milk fat analogue and from about 0.35 grams to about 210 grams of the one or more anti-tumour agents;
   (c) about 10 grams to about 200 grams of milk fat or a milk fat analogue and from about 2.5 grams to about 70 grams of the one or more anti-tumour agents;
   (d) about 10 grams to about 200 grams of milk fat or a milk fat analogue and from about 0.25 grams to about 5 grams of the one or more anti-tumour agents;
   (e) about 15 grams to about 30 grams of milk fat or a milk fat analogue and from about 1 grams to about 6 grams of the one or more anti-tumour agents; or
   (f) about 3 grams to about 8 grams of milk fat or a milk fat analogue and from about 0.1 grams to about 1 grams of the one or more anti-tumour agents.
16. A product comprising milk fat or a milk fat analogue and one or more additional anti-tumour agents as a combined preparations for simultaneous, separate or sequential use, wherein the product is suitable for administration to a subject suffering from or being treated for cancer.
17. The product of clause 16 wherein the administration
   (a) inhibits tumour formation or inhibits tumour growth in the subject; or
   (b) increases an anti-tumour immune response in the subject; or
   (c) induces apoptosis in the subject; or
   (d) inhibits angiogenesis in the subject; or
   (e) treats or prevents anemia caused by low hemoglobin or red blood cell levels, cachexia, mucositis, or leukopaenia, or maintains or improves one or more of the white blood cell count, the red blood cell count, and the myeloid cell count of the subject; or
   (f) increases the responsiveness of the subject to a cancer therapy, increases the sensitivity of a tumour in the subject to a cancer therapy, or speeds the recovery of the subject.
18. A method as set out in any one of clauses 1 to 10, a use as set out in any one of clauses 11 to 13, a composition as set out in clauses 14 or 15, or a product as set out in clauses 16 or 17, wherein one or more anti-tumour agents is selected from the group consisting anti-tumour food factors, chemotherapeutic agents, immunotherapeutic agents, hematopoietic agents, anticachectic agents, or antimucositic agents.
19. A method as set out in clauses 1 or 10,-a use as set out in any one of clauses 11 to 13, a composition as set out in clauses 14 or 15, or the product as set out in clauses 16 or 17 wherein one or more anti-tumour agents is a lactoferrin, apo-lactoferrin, a lactoferrin polypeptide, a functional lactoferrin variant, a functional lactoferrin fragment, metal ion lactoferrin, naturally iron-saturated lactoferrin, substantially fully iron-saturated lactoferrin, a metal ion lactoferrin functional variant, a metal ion lactoferrin functional fragment, or a mixture of any two of more thereof.
20. The method, use, composition, or product as set out in clause 18 wherein the anti-tumour food factor is selected from vitamin D (including vitamin D1 [lumisterol], vitamin D2 [calciferol or ergocalciferolj, vitamin D3 [cholecalciferol], vitamin D4 [22- dihydroerogocalciferol] and vitamin D5 [sitocalciferol] and vitamin DS [7-dehydrositosterol]), vitamin D analogues, soy protein, one or more soybean components, one or more omega-3 fatty acids from soy, one or more isoflavones from
   soy, genistein, daidzein, one or more lunasin peptides, one or more polyphenols, . lycopene, wheat bran, flavonoids, inositol, resveratrol, propolis, mushroom extract, anthocyanins, almonds, ginseng, casein hydrolysate, and combinations or any two or more thereof.
21. A method as set out in clauses 1 or 10, a use as set out in any one of clauses 11 to 13, a composition as set out in clauses 14 or 15, or the product as set out in clauses 16 or 17 wherein the milk fat is selected from the group comprising dairy lipids, dairy lipid fractions, dairy lipid hydrolysates, dairy lipid fraction hydrolysates, or bovine milk fat.
22. The method, use, composition, or product as set out in clause 21 wherein the milk fat is selected from the group consisting of cream, butter, anhydrous milk fat, butter milk, butter serum, hard milk fat fractions, soft milk fat fractions, sphingolipid fractions, milk fat globular membrane fractions, phospholipid fractions, and complex lipid fractions, and combinations thereof, and hydrolysates thereof, and fractions of the hydrolysates, and combinations of hydrolysed and/or non-hydrolysed fractions.
23. The method, use, composition, or product as set out in clause 22 wherein the milk fat is anhydrous milk fat or cream.
24. The method, use, composition, or product as set out in any one of clauses 1 to 23 wherein the milk fat or milk fat analogue comprises
   (a) between about 23%(w/w) and about 32%(w/w) palmitic acid;
   (b) between about 15%(w/w) and about 22%(w/w) oleic acid;
   (c) between about 10%(w/w) and about 15%(w/w )stearic acid;
   (d) between about 9%(vi/w) and about 12%(w/w) myristic acid;
   (e) between about 3%(w/w) and about 5%(w/w) butyric acid;
   (f) any two of a), b), c), d), or e) above;
   (g) any three of a), b), c), d), or e) above;
   (h) any four of a), b), c), d), or e) above; or
   (i) each of a), b), c), d), and e) above.

## Claims

1. Milk fat or a milk fat analogue for use in the treatment of mucositis, including in a subject at risk of developing, suffering from, or being treated for mucositis.

2. The milk fat or a milk fat analogue for use according to claim 1 wherein the milk fat or a milk fat analogue is formulated for separate, simultaneous or sequential administration with one or more anti-tumour agents.

3. The milk fat or a milk fat analogue for use according to claim 1 or 2 wherein the milk fat or a milk fat analogue is present in a composition additionally comprising one or more anti-tumour agents.

4. The milk fat or a milk fat analogue for use according to any one of claims 1 to 3 wherein the one or more anti-tumour agents is selected from the group comprising lactoferrin, apo-lactoferrin, a lactoferrin polypeptide, a functional lactoferrin variant, a functional lactoferrin fragment, metal ion lactoferrin, naturally iron-saturated lactoferrin, substantially fully iron-saturated lactoferrin, a metal ion lactoferrin functional variant, a metal ion lactoferrin functional fragment, or a combination of any two of more thereof; anti-tumour food factors including vitamin D (including vitamin D1 [lumisterol], vitamin D2 [calciferol or ergocalciferol], vitamin D3 [cholecalciferol], vitamin D4 [22-dihydroerogocalciferol] and vitamin D5 [sitocalciferol] and vitamin D5 [7-dehydrositosterol]), vitamin D analogues, soy protein, one or more soybean components, one or more omega-3 fatty acids from soy, one or more isoflavones from soy, genistein, daidzein, one or more lunasin peptides, one or more polyphenols, lycopene, wheat bran, flavonoids, inositol, resveratrol, propolis, mushroom extract, anthocyanins, almonds, ginseng, casein hydrolysate, and combinations of any two or more thereof; chemotherapeutic agents; immunotherapeutic agents; hematopoietic agents; anticachectic agents; antimucositic agents; and combinations of any two or more thereof.

5. The milk fat or a milk fat analogue for use according to any one of claims 1 to 4 wherein the milk fat is selected from the group comprising dairy lipids, dairy lipid fractions, dairy lipid hydrolysates, dairy lipid fraction hydrolysates, bovine milk fat, cream, butter, anhydrous milk fat, butter milk, butter serum, hard milk fat fractions, soft milk fat fractions, sphingolipid fractions, milk fat globular membrane fractions, phospholipid fractions, and complex lipid fractions, and combinations thereof, and hydrolysates thereof, and fractions of the hydrolysates, and combinations of hydrolysed and/or non-hydrolysed fractions.

6. The milk fat or a milk fat analogue for use according to any one of claims 1 to 5 wherein the milk fat or milk fat analogue is anhydrous milk fat or cream, or the milk fat or milk fat analogue comprises
(a) between about 23%(w/w) and about 32%(w/w) palmitic acid;
(b) between about 15%(w/w) and about 22%(w/w) oleic acid;
(c) between about 10%(w/w) and about 15%(w/w)stearic acid;
(d) between about 9%(w/w) and about 12%(w/w) myristic acid;
(e) between about 3%(w/w) and about 5%(w/w) butyric acid;
(f) any two of a), b), c), d), or e) above;
(g) any three of a), b), c), d), or e) above;
(h) any four of a), b), c), d), or e) above; or
(i) each of a), b), c), d), and e) above.

7. A composition comprising about 2 grams to about 210 grams of milk fat or a milk fat analogue and from about 0.7 grams to about 210 grams of one or more anti-tumour agents, including a composition comprising
(a) about 2 grams to about 210 grams of milk fat or a milk fat analogue and from about 0.7 grams to about 70 grams of the one or more anti-tumour agents;
(b) about 35 grams to about 210 grams of milk fat or a milk fat analogue and from about 0.7 grams to about 210 grams of the one or more anti-tumour agents;
(c) about 10 grams to about 200 grams of milk fat or a milk fat analogue and from about 2.5 grams to about 70 grams of the one or more anti-tumour agents;
(d) about 10 grams to about 200 grams of milk fat or a milk fat analogue and from about 0.7 grams to about 5 grams of the one or more anti-tumour agents;
(e) about 15 grams to about 30 grams of milk fat or a milk fat analogue and from about 1 grams to about 6 grams of the one or more anti-tumour agents; or
(f) about 3 grams to about 8 grams of milk fat or a milk fat analogue and from about 0.1 grams to about 1 grams of the one or more anti-tumour agents.

8. A composition comprising about 2 grams to about 210 grams of milk fat or a milk fat analogue to which has been added from about 0.1 grams to about 210 grams of one or more anti-tumour agents, including a composition comprising
(a) about 0.1 gram to about 50g of milk fat or a milk fat analogue and from about 1 microgram to about 1 gram of the one or more anti-tumour agents;
(b) about 0.1 gram to about 50g of milk fat or a milk fat analogue and from about 1 microgram to about 1 milligram of the one or more anti-tumour agents;
(c) about 2 grams to about 210 grams of milk fat or a milk fat analogue and from about 0.7 grams to about 70 grams of the one or more anti-tumour agents;
(d) about 35 grams to about 210 grams of milk fat or a milk fat analogue and from about 0.35 grams to about 210 grams of the one or more anti-tumour agents;
(e) about 10 grams to about 200 grams of milk fat or a milk fat analogue and from about 2.5 grams to about 70 grams of the one or more anti-tumour agents;
(f) about 10 grams to about 200 grams of milk fat or a milk fat analogue and from about 0.25 grams to about 5 grams of the one or more anti-tumour agents;
(g) about 15 grams to about 30 grams of milk fat or a milk fat analogue and from about 1 grams to about 6 grams of the one or more anti-tumour agents; or
(h) about 3 grams to about 8 grams of milk fat or a milk fat analogue and from about 0.1 grams to about 1 gram of the one or more anti-tumour agents.

9. The composition as claimed in claim 7 or 8 wherein the composition is or is present in an edible consumer product, including an edible consumer product selected from the group comprising a confectionery product, a dessert, a liquid, a drink, a food bar, a muesli bar, a spread, a sauce, a dip, an ice cream, a yoghurt, a cheese, a milk shake, a yoghurt shake, a milk powder, an infant or growing up formula, or an enteric liquid.

10. A product comprising milk fat or a milk fat analogue to which has been added one or more additional anti-tumour agents as a combined preparation for simultaneous, separate or sequential use, wherein the product is for use in the treatment of mucositis or is suitable for administration to a subject at risk of developing, suffering from, or being treated for mucositis.

11. A product for use according to claim 10 wherein the anti-tumour agent is suitable for parenteral administration.

12. The composition as claimed in any one of claims 7 to 9 or the product for use according to claim 10 or 11 wherein the one or more anti-tumour agents is selected from the group comprising lactoferrin, apo-lactoferrin, a lactoferrin polypeptide, a functional lactoferrin variant, a functional lactoferrin fragment, metal ion lactoferrin, naturally iron-saturated lactoferrin, substantially fully iron-saturated lactoferrin, a metal ion lactoferrin functional variant, a metal ion lactoferrin functional fragment, or a combination of any two of more thereof; anti-tumour food factors including vitamin D (including vitamin D1 [lumisterol], vitamin D2 [calciferol or ergocalciferol], vitamin D3 [cholecalciferol], vitamin D4 [22-dihydroerogocalciferol] and vitamin D5 [sitocalciferol] and vitamin D5 [7-dehydrositosterol]), vitamin D analogues, soy protein, one or more soybean components, one or more omega-3 fatty acids from soy, one or more isoflavones from soy, genistein, daidzein, one or more lunasin peptides, one or more polyphenols, lycopene, wheat bran, flavonoids, inositol, resveratrol, propolis, mushroom extract, anthocyanins, almonds, ginseng, casein hydrolysate, and combinations of any two or more thereof; chemotherapeutic agents; immunotherapeutic agents; hematopoietic agents; anticachectic agents; antimucositic agents; and combinations of any two or more thereof.

13. The composition as claimed in any one of claims 7 to 9 or 12 or the product for use according to any one of claims 10 to 12 wherein the milk fat is selected from the group comprising dairy lipids, dairy lipid fractions, dairy lipid hydrolysates, dairy lipid fraction hydrolysates, bovine milk fat, cream, butter, anhydrous milk fat, butter milk, butter serum, hard milk fat fractions, soft milk fat fractions, sphingolipid fractions, milk fat globular membrane fractions, phospholipid fractions, and complex lipid fractions, and combinations thereof, and hydrolysates thereof, and fractions of the hydrolysates, and combinations of hydrolysed and/or non-hydrolysed fractions.

14. The composition as claimed in any one of claims 7 to 9, 12, or 13 or the product as for use according to any one of claims 10 to 13 wherein the milk fat or milk fat analogue comprises
(a) between about 23%(w/w) and about 32%(w/w) palmitic acid;
(b) between about 15%(w/w) and about 22%(w/w) oleic acid;
(c) between about 10%(w/w) and about 15%(w/w )stearic acid;
(d) between about 9%(w/w) and about 12%(w/w) myristic acid;
(e) between about 3%(w/w) and about 5%(w/w) butyric acid;
(f) any two of a), b), c), d), or e) above;
(g) any three of a), b), c), d), or e) above;
(h) any four of a), b), c), d), or e) above; or
(i) each of a), b), c), d), and e) above.

15. The composition as claimed in any one of claims 7 to 9 or 12 to 14 or the product for use according to any one of claims 10 to 14 for treating mucositis.
